# EUROPEAN PATENT APPLICATION

(11) **EP 4 512 827 A2**
(43) Date of publication of application: **26.02.2025**
(21) Application number: 24202238.2
(22) Date of filing: 09.11.2016
(51) Int. Cl.: C07K 16/28

(54) **COMPOSITIONS AND METHODS FOR TREATING AUTOIMMUNE DISEASES AND CANCERS**

(30) Priority: 10.11.2015 US 201562253437 P
(62) Divisional of application: 16864893.9
(71) Applicant: Yale University, New Haven CT 06510 (US)
(72) Inventor: CHEN, Lieping, Hamden, CT 06514 (US); WANG, Jun, New Haven, CT 06511 (US); SUN, Jingwei, New Haven, CT 06511 (US)
(74) Representative: Richards, Michèle E.

(57) **Abstract**

The present invention provides methods and compositions for treating a cancer, methods for increasing an immune response against a tumor, methods for treating an autoimmune disease, and methods for decreasing an inflammation response in a subject in need thereof by modulating the expression and/or activity of Siglec 15 and/or its binding ligands.

## Description

### RELATED APPLICATIONS

This application claims priority to U.S. Provisional Application No. 62/253,437, filed November 10, 2015, which is incorporated by reference herein in its entirety.

### BACKGROUND OF THE INVENTION

Cancer has been known as one of the leading causes of death in industrialized nations. Cancers are caused by the progressive growth of the progeny of a single transformed cell. Treating cancer requires that all the malignant cells be removed or destroyed without killing the patient. An attractive way to achieve this would be to induce an immune response against the tumor that would discriminate between the cells of the tumor and their normal cell counterparts. Indeed, the immune system has a great potential for the specific destruction of tumors with no toxicity to normal tissue. The immune system's natural capacity to detect and destroy abnormal cells may prevent the development of many cancers. In addition, the long-term memory of the immune system may prevent cancer recurrence.

However, cancer cells are sometimes able to avoid detection and destruction by the immune system by reducing the expression of tumor antigens on their surface, making it harder for the immune system to detect them. Alternatively, cancer cells may express proteins on their surface that induce immune cell inactivation, or induce cells in the surrounding environment to release substances that suppress immune responses and promote tumor cell proliferation and survival. Thus, there remains an ongoing need to identify new molecules that modulate the immune responses against tumors for the development of new immunotherapeutic agents. (Sznol M and Chen L, Clin Cancer Res 19(5): 1021-1034, 2013)

The cell membranes of immune cells are covered with a dense coating of various glycans, such as sialic acid, which are recognized by various glycan-binding proteins. Sialic-acid-binding immunoglobulin-like lectins (Siglecs) are a family of type I membrane proteins that regulates the functions of cells in the innate and adaptive immune systems through glycan recognition. (Kameda Y. et al., J. Bone Miner. Res. 2013, 28(12): 24463-75). Siglecs proteins contain a sialic acid binding component and an Ig-like molecule. They are involved in cell-adhesion interactions, neuronal, brain functions, and neuron development. There are two smaller members of the Siglec family: Siglec 14 and Siglec 15. Both of these molecules have small intracellular domains and intra-membrane domains that can be phosphorylated. They are small enough to transmit signals across the cell membrane due to their size. These Siglec members are found in both mouse and human and are highly evolutionarily conserved between species.

Previous microarray data indicate that Siglec 15 is present in myeloid cells in the periphery, in macrophages and in monocytes. Siglec 15 is generally expressed on monocytes, macrophages, dendritic cells, B cells and osteoclasts. Siglec 15 expression in monocytes, myeloid cells and B cells can be induced and upregulated by RANK ligand and M-CSF (Stuible M, et al.. J Biol Chem. 2014, 289(10): 6498-512; Takamiya R, et al., Glycobiology. 2013; 23(2): 178-87). Siglec-15-deficient mice exhibit mild osteopetrosis resulting from impaired osteoclast development. (Kameda Y. et al., J. Bone Miner. Res. 28(12): 24463-75, 2013). Although the role of Siglec 15 in osteoclast differentiation and bone remodeling has been extensively studied, little is known about the immunological function of Siglec 15 (Stuible M, et al.. J Biol Chem. 2014, 289(10): 498-512). Recent studies have shown the expression and co-localization between Siglec 15 and CD68 in tumors of the lung, rectal adenocarcinoma and hepatocellular carcinoma, which are found expressed in macrophages, further supporting the expression of Siglec 15 in myeloid cells (Takamiya R, et al., Glycobiology. 2013; 23(2): 178-87).

Accordingly, there is a need in the art for the identification of additional molecules that modulate the immune responses against tumors in order to broaden the success rate and take full advantage of anti-tumor and autoimmune immunotherapies.

### SUMMARY OF THE INVENTION

The present invention is based, at least in part, on the discovery that Siglec 15 plays a critical role in suppression of the immune responses to cancer, thus, providing a rationale to manipulate the Siglec 15 pathway for future development of therapeutic cancer drugs. In particular, it has been discovered that a decrease in expression of Siglec 15 reduces tumor size and prolongs the survival of mice having brain tumors. In addition, inhibition of Siglec 15 activity by blocking the interaction between Siglec 15 and its ligands in a brain inflammation mouse model, as well as abolishing the expression of Siglec 15 in Siglec 15 knockout mice accelerated brain inflammation.

Accordingly, the present invention provides methods of treating a cancer or increasing an immune response to a cancer by inhibiting or suppressing the expression and/or activity of Siglec 15 and/or its binding ligands *e.g.* MAG, LRRC4C, and/or Sialyl-Tn. In another aspect, the present invention includes methods of treating an autoimmune disease, or decreasing an inflammation response in a subject by increasing the expression and/or activity of Siglec 15 and/or its binding ligands, *e.g.*, MAG, LRRC4C, and/or Sialyl-Tn.

In one aspect, the present invention provides a method of treating a cancer in a subject in need thereof, comprising administering to the subject an effective amount of a modulator of Siglec 15, wherein the modulator decreases the expression and/or activity of Siglec 15 in the subject, thereby treating a cancer in the subject.

In some embodiments, the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15. In other embodiments, the Siglec 15 fusion protein is a Siglec 15-Fc fusion protein.

In some embodiments, the modulator blocks the interaction between Siglec 15 and a binding ligand. In some embodiments, the binding ligand is selected from the group consisting of MAG and LRRC4C. In some embodiments, the modulator is a mutant Siglec 15 protein that has a single substitution mutation at residue 143. In other embodiments, the modulator is a mutant Siglec 15 protein that has a deletion of the IgV domain. In other embodiments, the binding ligand is Sialyl-Tn. In some embodiments, the modulator is a soluble Sialyl-Tn molecule. In other embodiments, the modulator is an antibody, or antigen-binding fragment thereof, that specifically binds Sialyl-Tn.

In some embodiments, the modulator decreases the expression and/or activity levels of MAG and/or LRRC4C. In other embodiments, the modulator decreases the expression and/or activity level of proteins containing Sialyl-Tn. In some embodiments, the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.

In some embodiments, the cancer is selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma. In other embodiments, the brain cancer is glioblastoma. In some embodiments, the cancer is colon cancer, endometriod cancer, kidney cancer (*e*.*g*., kidney papillary cell carcinoma, kidney clear cell carcinoma), liver cancer, thyroid cancer, lung cancer (*e*.*g*., lung adenocarcinoma, lung squamous cell carcinoma), head and neck cancer, breast cancer, cervical cancer, prostate cancer, bladder cancer, glioblastoma, rectal cancer, or bile duct cancer. In one embodiment, the cancer is brain cancer. In one embodiment, the cancer is glioblastoma. In one embodiment, the cancer is not a blood-derived cancer. In one embodiment, the cancer is not leukemia. In one embodiment, the cancer is not acute myeloid leukemia (AML).

In some embodiments, the subject does not suffer from an ongoing autoimmune disease.

In other embodiments, the subject is human.

In one aspect, the present invention provides a method of reducing a tumor size in a subject in need thereof, comprising administering to the subject an effective amount of a modulator of Siglec 15, wherein the modulator decreases the expression and/or activity of Siglec 15, thereby reducing the tumor size in the subject.

In some embodiments, the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15. In some embodiments, the Siglec 15 fusion protein is a Siglec 15-Fc fusion protein.

In some embodiments, the modulator blocks the interaction between Siglec 15 and a binding ligand. In some embodiments, the binding ligand is selected from the group consisting of MAG and LRRC4C. In other embodiments, the binding ligand is Sialyl-Tn. In some embodiments, the modulator is a mutant Siglec 15 protein that has a single substitution mutation at residue 143. In other embodiments, the modulator is a mutant Siglec 15 protein that has a deletion of the IgV domain.

In some embodiments, the modulator decreases the expression and/or activity of MAG and LRRC4C. In some embodiments, the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.

In some embodiments, the tumor is associated with a cancer selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma. In some embodiments, the brain cancer is glioblastoma. In some embodiments, the cancer is colon cancer, endometriod cancer, kidney cancer (e.g., kidney papillary cell carcinoma, kidney clear cell carcinoma), liver cancer, thyroid cancer, lung cancer (*e*.*g*., lung adenocarcinoma, lung squamous cell carcinoma), head and neck cancer, breast cancer, cervical cancer, prostate cancer, bladder cancer, glioblastoma, rectal cancer, or bile duct cancer. In one embodiment, the cancer is brain cancer. In one embodiment, the cancer is glioblastoma. In one embodiment, the cancer is not a blood-derived cancer. In one embodiment, the cancer is not leukemia. In one embodiment, the cancer is not acute myeloid leukemia (AML).

In some embodiments, the subject does not suffer from an ongoing autoimmune disease.

In other embodiments, the subject is human.

In one aspect, the present invention provides a method of prolonging the survival of a subject having a cancer, comprising administering to the subject an effective amount of a modulator of Siglec 15, wherein the modulator decreases the expression and/or activity of Siglec 15, thereby prolonging the survival of the subject.

In some embodiments, the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15. In some embodiments, the Siglec 15 fusion protein is a Siglec 15-Fc fusion protein.

In some embodiments, the modulator blocks the interaction between Siglec 15 and a binding ligand. In other embodiments, the binding ligand is selected from the group consisting of MAG and LRRC4C. In other embodiments, the binding ligand is Sialyl-Tn. In some embodiments, the modulator is a mutant Siglec 15 protein that has a single substitution mutation at residue 143. In other embodiments, the modulator is a mutant Siglec 15 protein that has a deletion of the IgV domain.

In some embodiments, the modulator decreases the expression and/or activity of MAG and LRRC4C. In some embodiments, the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.

In some embodiments, the cancer is selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma. In some embodiments, the brain cancer is glioblastoma. In some embodiments, the cancer is colon cancer, endometriod cancer, kidney cancer (*e*.*g*., kidney papillary cell carcinoma, kidney clear cell carcinoma), liver cancer, thyroid cancer, lung cancer (*e*.*g*., lung adenocarcinoma, lung squamous cell carcinoma), head and neck cancer, breast cancer, cervical cancer, prostate cancer, bladder cancer, glioblastoma, rectal cancer, or bile duct cancer. In one embodiment, the cancer is brain cancer. In one embodiment, the cancer is glioblastoma. In one embodiment, the cancer is not a blood-derived cancer. In one embodiment, the cancer is not leukemia. In one embodiment, the cancer is not acute myeloid leukemia (AML).

In some embodiments, the subject does not suffer from an ongoing autoimmune disease.

In other embodiments, the subject is human.

In one aspect, the present invention provides a method of increasing an immune response against a tumor in a subject in need thereof, comprising administering to the subject an effective amount of a modulator of Siglec 15, wherein the modulator decreases the expression and/or activity of Siglec 15, thereby increasing an immune response against the tumor in the subject.

In some embodiments, the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15. In some embodiments, the Siglec 15 fusion protein is a Siglec 15-Fc fusion protein.

In some embodiments, the modulator blocks the interaction between Siglec 15 and a binding ligand. In some embodiments, the binding ligand is selected from the group consisting of MAG and LRRC4C. In other embodiments, the binding ligand is Sialyl-Tn. In some embodiments, the modulator is a mutant Siglec 15 protein that has a single substitution mutation at residue 143. In other embodiments, the modulator is a mutant Siglec 15 protein that has a deletion of the IgV domain.

In some embodiments, the modulator decreases the expression and/or activity of MAG and LRRC4C. In some embodiments, the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.

In some embodiments, the tumor is associated with a cancer selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma. In some embodiments, the brain cancer is glioblastoma. In some embodiments, the cancer is colon cancer, endometriod cancer, kidney cancer (*e*.*g*., kidney papillary cell carcinoma, kidney clear cell carcinoma), liver cancer, thyroid cancer, lung cancer (*e*.*g*., lung adenocarcinoma, lung squamous cell carcinoma), head and neck cancer, breast cancer, cervical cancer, prostate cancer, bladder cancer, glioblastoma, rectal cancer, or bile duct cancer. In one embodiment, the cancer is brain cancer. In one embodiment, the cancer is glioblastoma. In one embodiment, the cancer is not a blood-derived cancer. In one embodiment, the cancer is not leukemia. In one embodiment, the cancer is not acute myeloid leukemia (AML).

In some embodiments, the subject does not suffer from an ongoing autoimmune disease.

In other embodiments, the subject is human.

In another aspect, the present invention provides a method of treating an autoimmune disease in a subject in need thereof, comprising administering to the subject an effective amount of a modulator of Siglec 15, wherein the modulator increases the expression and/or activity of Siglec 15 in the subject, thereby treating an autoimmune disease in the subject.

In some embodiments, the modulator is selected from the group consisting of a small molecule activator of Siglec 15, an agonist antibody of Siglec 15, or antigen-binding fragment thereof, or a protein and a nucleic acid that activates the transcription and/or translation of Siglec 15.

In some embodiments, the modulator promotes the interaction between Siglec 15 and a binding ligand. In some embodiments, the binding ligand is selected from the group consisting of MAG and LRRC4C. In other embodiments, the binding ligand is Sialyl-Tn. In some embodiments, the modulator increases the expression and/or activity of MAG and LRRC4C.

In some embodiments, the modulator is selected from the group consisting of a small molecule activator of MAG, an agonist antibody for MAG, or antigen-binding fragment thereof, a protein and a nucleic acid that activates the transcription and/or translation of MAG, a small molecule activator of LRRC4C, an agonist antibody for LRRC4C, or antigen-binding fragment thereof, or a protein and a nucleic acid that activates the transcription and/or translation of LRRC4C.

In other embodiments, the modulator is selected from the group consisting of a MAG protein, a nucleic acid encoding the MAG protein, an LRRC4C protein or a nucleic acid encoding the LRRC4C protein. In one embodiment, the modulator is a synthetic Sialyl-Tn, or a synthetic peptide bearing Sialyl-Tn.

In some embodiments, the autoimmune disease is an inflammatory brain disease. In other embodiments, the inflammatory brain disease is multiple sclerosis. In other embodiments, the inflammatory brain disease is experimental autoimmune encephalomyelitis (EAE). In some embodiments, the subject is human.

In one aspect, the present invention provides a method of treating an autoimmune disease in a subject in need thereof, comprising administering to the subject an effective amount of Siglec 15 protein, a nucleic acid encoding the Siglec 15 protein, thereby treating an autoimmune disease in the subject.

In some embodiments, the Siglec 15 protein is selected from the group consisting of a full-length Siglec 15 protein, a functional fragment of Siglec 15, or an IgV domain of Siglec 15.

In some embodiments, the autoimmune disease is an inflammatory brain disease. In other embodiments, the inflammatory brain disease is multiple sclerosis. In other embodiments, the inflammatory brain disease is experimental autoimmune encephalomyelitis (EAE). In some embodiments, the subject is human.

In another aspect, the present invention provides a method of decreasing a brain inflammatory response in a subject in need thereof, comprising administering to the subject an effective amount of a modulator of Siglec 15, wherein the modulator increases the expression and/or activity of Siglec 15, thereby decreasing a brain inflammatory response in the subject.

In some embodiments, the modulator is selected from the group consisting of a small molecule activator of Siglec 15, an agonist antibody of Siglec 15, or antigen-binding fragment thereof, or a protein and a nucleic acid that activates the transcription and/or translation of Siglec 15.

In some embodiments, the modulator promotes the interaction between Siglec 15 and a binding ligand. In some embodiments, the binding ligand is selected from the group consisting of MAG and LRRC4C. In other embodiments, the binding ligand is Sialyl-Tn. In other embodiments, the modulator increases the expression and/or activity of MAG and LRRC4C.

In some embodiments, the modulator is selected from the group consisting of a small molecule activator of MAG, an agonist antibody for MAG, or antigen-binding fragment thereof, a protein and a nucleic acid that activates the transcription and/or translation of MAG, a small molecule activator of LRRC4C, an agonist antibody for LRRC4C, or antigen-binding fragment thereof, or a protein and a nucleic acid that activates the transcription and/or translation of LRRC4C.

In other embodiments, the modulator is selected from the group consisting of a MAG protein, a nucleic acid encoding the MAG protein, an LRRC4C protein or a nucleic acid encoding the LRRC4C protein.

In some embodiments, the brain inflammatory response is associated with multiple sclerosis. In some embodiments, the brain inflammatory response is associated with experimental autoimmune encephalomyelitis (EAE). In some embodiments, the subject is human.

In one aspect, the present invention provides a method of decreasing a brain inflammatory response in a subject in need thereof, comprising administering to the subject an effective amount of Siglec 15 protein, or a nucleic acid encoding the Siglec 15 protein, thereby decreasing a brain inflammatory response in the subject.

In some embodiments, the Siglec 15 protein is selected from the group consisting of a full-length Siglec 15 protein, a functional fragment of Siglec 15, or an IgV domain of Siglec 15.

In some embodiments, the brain inflammatory response is associated with multiple sclerosis. In some embodiments, the brain inflammatory response is associated with experimental autoimmune encephalomyelitis (EAE). In some embodiments, the subject is human.

In another aspect, the present invention provides a method for identifying a compound useful for treating an autoimmune disease or a cancer in a subject, comprising providing a test compound; determining the effect of the test compound on the expression and/or activity of Siglec 15; and selecting a compound which modulates the expression and/or activity of Siglec 15, thereby identifying a compound useful for treating an autoimmune disease or a cancer in the subject.

In some embodiments, an increase in the expression and/or activity of Siglec 15 indicates that the compound is useful for treating an autoimmune disease. In other embodiments, a decrease in the expression and/or activity of Siglec 15 indicates that the compound is useful for treating a cancer.

In one aspect, the present invention provides a method of identifying a compound useful for increasing an immune response against a tumor in a subject in need thereof, comprising providing a test compound; determining the effect of the test compound on the expression and/or activity of Siglec 15; and selecting a compound which decreases the expression and/or activity of Siglec 15, thereby identifying a compound useful for increasing an immune response against the tumor in the subject.

In another aspect, the present invention provides a method of identifying a compound useful for decreasing a brain inflammatory response in a subject in need thereof, comprising providing a test compound; determining the effect of the test compound on the expression and/or activity of Siglec 15; and selecting a compound which increases the expression and/or activity of Siglec 15, thereby identifying a compound useful for decreasing a brain inflammatory response in the subject.

In one aspect, the present invention provides a Siglec 15 modulator, wherein the modulator modulates the interaction between Siglec 15 and MAG, LRRC4C, and/or Sialyl-Tn. In some embodiments, the modulator binds to the IgV domain of Siglec 15. In other embodiments, the modulator binds to an epitope comprising residue 143 of Siglec 15.

In some embodiments, the modulator is an inhibitor of Siglec 15. In other embodiments, inhibitor is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15.

In some embodiments, the modulator is an activator of Siglec 15. In other embodiments, the activator is selected from the group consisting of a small molecule activator of Siglec 15, an agonist antibody of Siglec 15, or antigen-binding fragment thereof, or a protein and a nucleic acid that activates the transcription and/or translation of Siglec 15.

The present invention is illustrated by the following drawings and detailed description, which do not limit the scope of the invention described in the claims.

### BRIEF DESCRIPTION OF THE DRAWINGS

**Figure 1A** depicts the RNA expression pattern of Siglec 15 in different mouse tissues using RT-PCR. **Figure 1B** depicts the RNA expression pattern of Siglec 15 in human tissues using microarray analysis. **Figure 1C** graphically depicts the expression pattern of Siglec 15 based on microarray analysis. Siglec 15 is generally expressed on monocytes, macrophages, dendritic cells, B cells and osteoclasts.
**Figure 2** depicts Siglec 15 expression levels in human cancer, relative to normal tissue.
**Figure 3A** depicts the expression of human Siglec 15 in human cancer cell lines, as determined using NCI60 microarray data (BioGPS; arbitrary units). **Figure 3B** depicts the expression of human Siglec 15 in human cancer cell lines, as determined using FACS.
**Figure 4A** depicts interaction between Siglec 15 and the binding ligands, myelin-associated glycoprotein (MAG) and leucine rich repeat containing 4C (LRRC4C). Interaction between Siglec 15 and MAG or LRRC4C is well conserved between mouse and human. **Figure 4B** depicts binding between Sialyl-Tn antigen and Siglec 15-mIg fusion protein, or control mIg, as determined using Octet streptavidin biosensors.
**Figure 5** depicts the mRNA expression of MAG in normal tissue and cancer. Expression of MAG is enriched in brain or brain related tumor.
**Figure 6** depicts the mRNA expression of LRRC4C in normal tissue and cancer. Expression of LRRC4C is up-regulated in cancers such as brain cancer, breast cancer, ovarian cancer, renal cell carcinoma and Ewing sarcoma.
**Figure 7** graphically depicts the binding domain for the Siglec 15-MAG or LRRC4C interaction. Specifically, the IgV domain of Siglec 15 is required for interaction with MAG and LRRC4C. A R143A mutation in the IgV domain prevents binding to either MAG or LRRC4C, suggesting that both ligands bind residue 143 of the IgV domain in Siglec 15.
**Figure 8A** depicts the level of ³H thymidine incorporation in PBMCs stimulated with the anti-CD3 antibody OKT3 and exposed to immobilized human Siglec 15 or control IgG. **Figure 8B** depicts the level of luciferase activity observed in Jurkat NF-AT reporter cells following incubation with 293T.m.OKT3 cells over-expressing mock plasmid (Mock), full-length FASLG (FASLG), full-length Siglec 15 (Siglec15 FL), a construct encoding the Siglec15 ectodomain and the B7-H6 transmembrane domain (Siglec15 ATM), or LRRC4C.

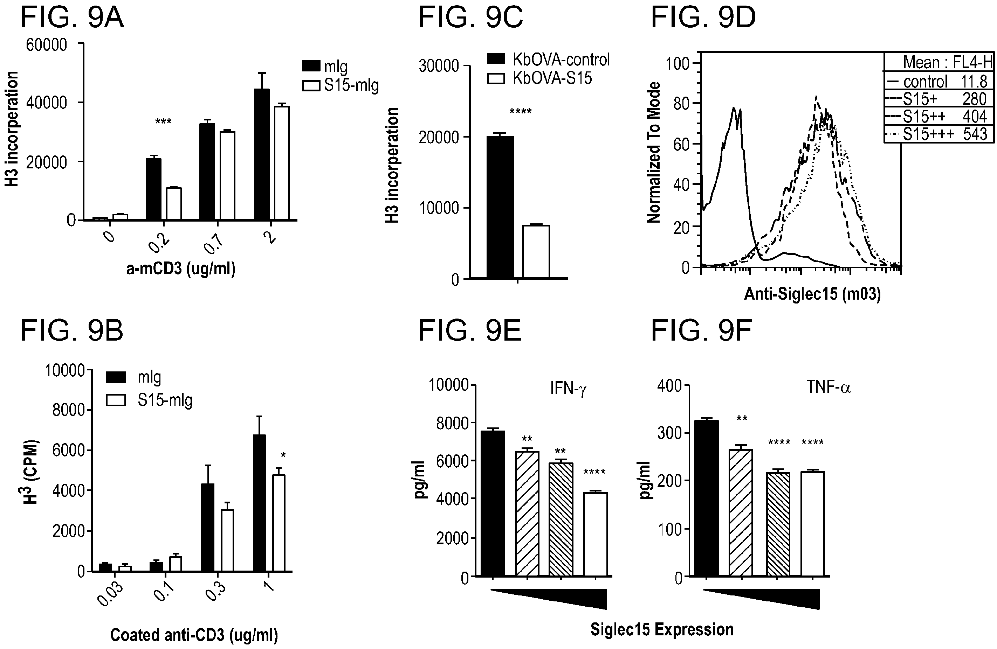

**Figure 9A** depicts the level of ³H thymidine incorporation in mouse splenocytes stimulated with immobilized anti-CD3, and either immobilized mouse Siglec-15-mIgG fusion protein (S15-mlg) or control IgG (mIg). **Figure 9B** depicts the level of³H thymidine incorporation in mouse splenocytes stimulated with immobilized anti-CD3, and either soluble mouse Siglec-15-mIgG fusion protein (S15-mlg) or control IgG (mIg). **Figure 9C** depicts the level of ³H thymidine incorporation in activated splenocytes from OT-1 transgenic mice co-cultured with 293T-KbOVA cells overexpressing full-length mouse Siglec 15 (KbOVA-S15) or mock control (KbOVA-control). **Figure 9D** depicts the level of Siglec 15 expression in three 293T-KbOVA cell lines over-expressing mouse Siglec 15. **Figure 9E** depicts the level of IFN-γ present in the supernatant of activated splenocytes from OT-1 transgenic mice co-cultured with 293T-KbOVA cells having increasing levels of Siglec 15 expression (determined in Figure 9D). **Figure 9F** depicts the level of TNF-α present in the supernatant of activated splenocytes from OT-1 transgenic mice co-cultured with 293T-KbOVA cells having increasing levels of Siglec 15 expression.
**Figure 10A** depicts the dose-response of OT-1 T cell killing of 293T-KbOVA target cells, as indicated by an adherence-based cytotoxicity assay (ACEA Biosciences). **Figure 10B** compares the adherence signal of 293T-KbOVA cells overexpressing Siglec15 (293T-KbOVA-S15) with the adherence signal of mock-transfected 293T-KbOVA cells (293T-KbOVA-control), in the presence of increasing amounts of OT-1 T cells. Ratios indicate the proportion of OT-1 T cells to 293T-KbOVA cells.

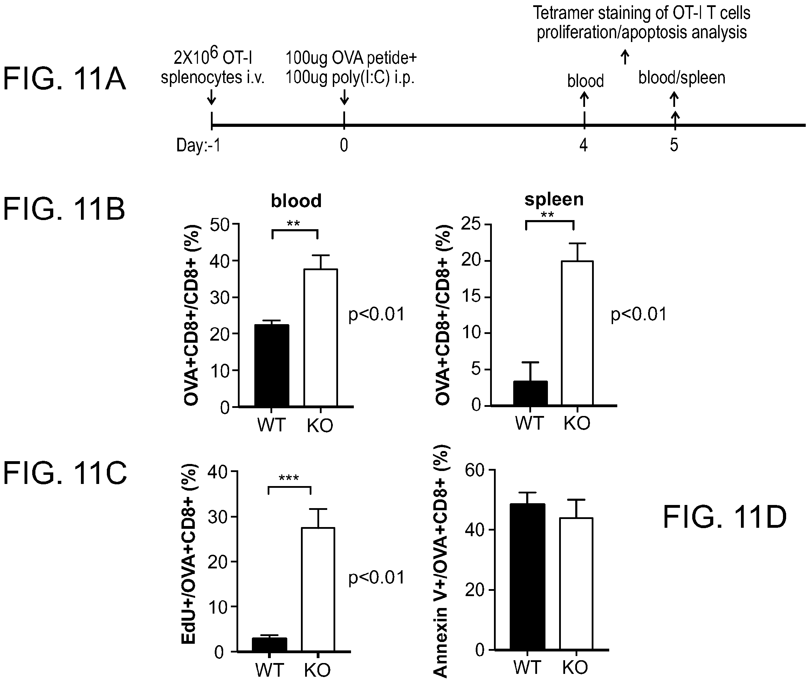

**Figure 11A** is a schematic diagram of the OT-1 *in vivo* activation model described in Example 8. **Figure 11B** depicts the percentage of OT-1 cells in the total CD8 T cell population in the blood (left panel) and spleen (right panel) of wild type (WT) or Siglec 15 whole body knockout (KO) mice following intravenous transfer of splenocytes from OT-1/RagKO mice. The percentage of OT-1 cells was monitored by FACS staining using OT-1 tetramer. **Figure 11C** depicts the proliferation of blood OT-1 cells at day 5 determined by anti-EdU staining, and calculated as the percentage of EdU positive OT-1 cells/total OT-1 positive cells. **Figure 11D** depicts apoptosis in splenocytes that were isolated and cultured overnight without stimulation, and stained for annexin V. Apoptosis was calculated as the percentage of annexin V-positive OT-1 cells/total OT-1 positive cells.
**Figure 12A** depicts the percentage of OT-1 cells in the total CD8 T cell population in the blood of mice at various times following intravenous transfer of splenocytes from OT-1/RagKO mice in accordance with the schedule described in Figure 11A. WT, wild-type mouse; KO, Siglec 15 whole-body knock out mouse; LysM-Cre KO, macrophage-specific Siglec 15 knock out mouse. **Figure 12** **B** depicts the level of IL-10 in the plasma of whole body KO and LysM-Cre KO mice assessed during OT-1 T cell reaction, compared with wild-type.
**Figure 13** describes the percentage of myeloid cells, CD8+ cells, and CD4+ cells in the blood of wild-type (WT) or Siglec 15 knock out (S15KO) mice at 11 months of age.
**Figure 14** depicts cytokine levels in the supernatant of macrophages co-cultured with 293T cells that overexpress mock plasmid (control), full-length LRRC4C, or Siglec 15.
**Figure 15** depicts that Siglec 15 antibody S15m02 acted as a blocking antibody that disrupted Siglec 15 from binding to MAG or to LRRC4C, whereas Siglec 15 antibody S15m03 did not affect the interaction between Siglec 15 and MAG or LRRC4C.
**Figure 16** depicts that addition of a blocking antibody of Siglec 15, S15m02, caused EAE mice to develop more severe disease symptoms than their counterparts injected with control mAb or receiving S15m03 antibody.
**Figure 17** depicts that addition of a Siglec 15-Fc fusion protein to EAE (experimental autoimmune encephalomyelitis) mice caused inflammation in those mice to be accelerated.
**Figure 18** depicts that Siglec 15 knockout (KO) mice (upper line) exhibited more severe EAE disease symptoms than wild-type (WT) mice (lower line), indicating an inhibitory role of Siglec 15 in the regulation of brain inflammatory responses.

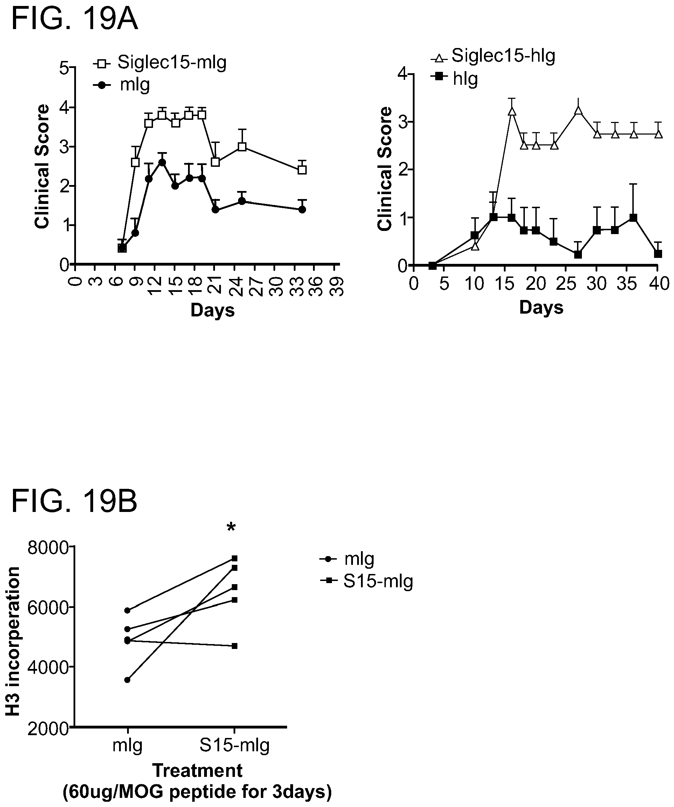

**Figure 19A** depicts the EAE clinical scores of WT mice immunized with MOG peptide and boosted with pertussis toxin at day 0 and day 1, followed by treatment with 100 µg of Siglec 15-mIg fusion protein (Siglec15-mIg) or control mIg (left panel), or a Siglec 15-hIg fusion protein (Siglec15-hIg) or control hIg (right panel) twice per week starting at day 6, for a total of 4 doses. **Figure 19B** depicts ³H-thymidine incorporation in splenocytes obtained from control mIg treated mice at day 12, which were re-stimulated with MOG peptide (60 µg/ml) for 3 days, in the presence of 5ug/ml Siglec15-mIg (S15-mIg) or control mIg (mIg).
**Figure 20** depicts that blocking of Siglec by Siglec 15-Fc treatment significantly reduced tumor size in mice with glioblastoma.
**Figure 21** depicts that blocking of Siglec by Siglec 15-Fc treatment significantly prolonged survival of mice with glioblastoma.
**Figure 22** depicts the synergistic effect of Siglec15-Fc and anti-PDL1 treatment on reducing tumor size and promoting survival benefit of mice with glioblastoma.
**Figure 23A** depicts luciferase activity in the brain of wild type (WT) or Siglec 15 knock-out (Siglec15 KO) mice inoculated intra-cranially at day 0 with GL261 glioblastoma cells containing a luciferase reporter. **Figure 23B** presents images of luciferase activity in the brain of WT and KO mice on days 13 and 18 after inoculation with GL261 cells. **Figure 23C** presents a survival curve of WT and KO mice inoculated with GL261.

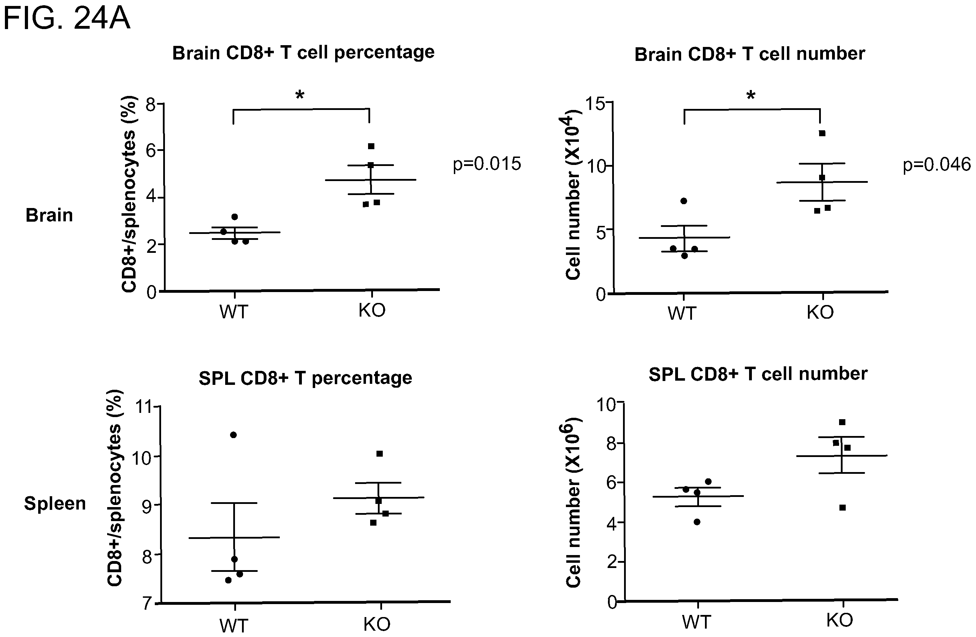

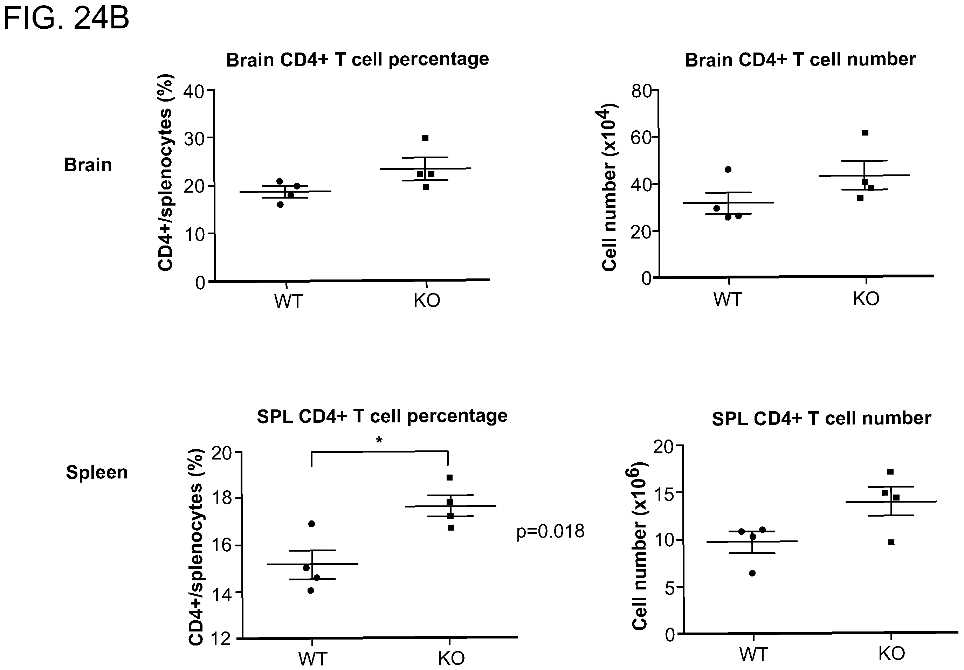

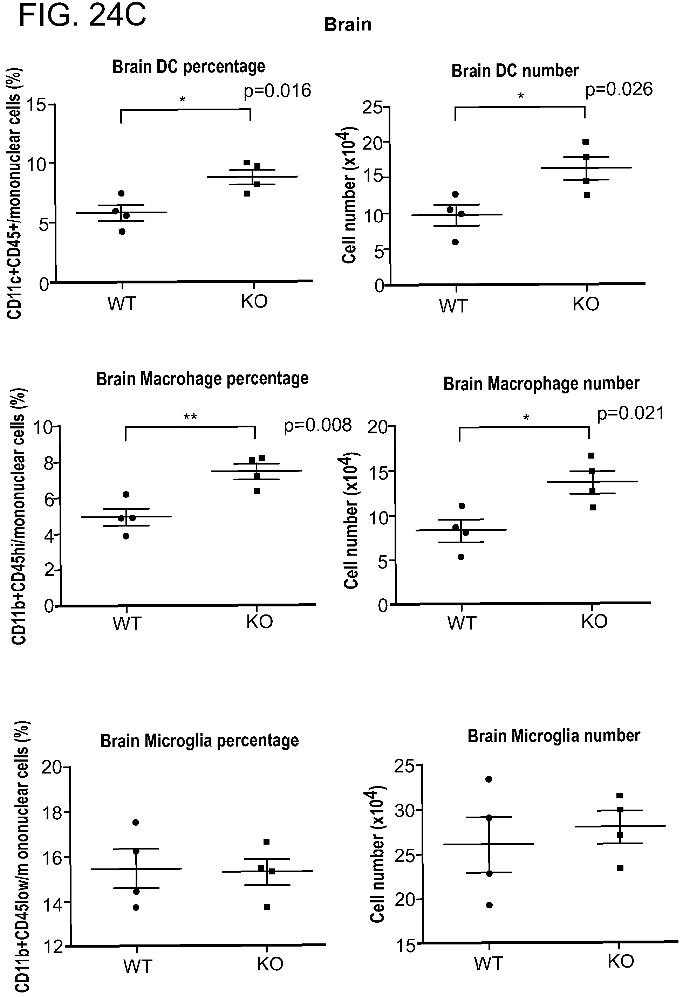

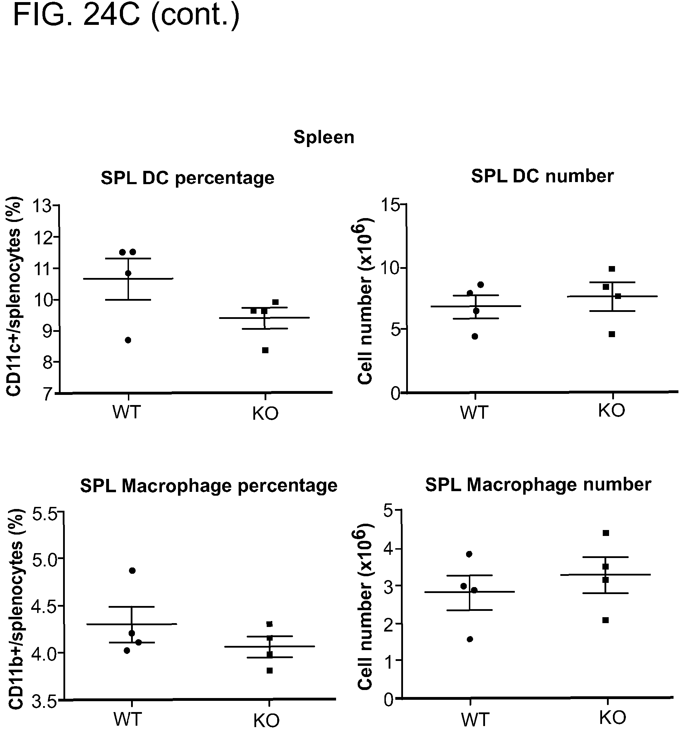

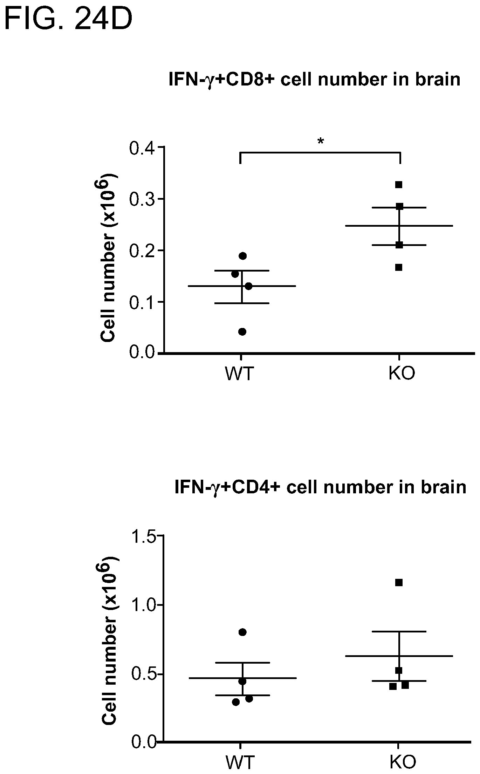

**Figure 24A** depicts the percentage and total number of CD8 T cells in the brain or spleen of both groups (WT and KO) at day 14. **Figure 24B** depicts the percentage and total number of CD4 T cells in the brain or spleen of both groups (WT and KO) at day 14. **Figure 24C** depicts the percentage and total number of dendritic cells (DC), macrophages, and microglia in the brain or spleen of both groups (WT and KO) at day 14. **Figure 24D** depicts the percentage and total number of IFN-γ positive CD8 or CD4 T cells in brain lymphocytes from tumor bearing WT or KO mice obtained at day 14, and re-stimulated with GL-261 tumor cells overnight.
**Figure 25A** depicts Siglec 15 expression on MC38-S15- and MC38-S15+ cells, determined by FACS staining with an anti-Siglec 15 monoclonal antibody (Figure 25A). **Figure 25B** depicts tumor growth of MC38-S15- and MC38-S15+ cells following inoculation by subcutaneous injection in B6 mice (Figure 25B).
**Figure 26** depicts the average tumor size in B6 mice inoculated with an MC38-S15+ stable cell line, and treated with a control antibody, anti-Siglec 15 antibody m01, or Siglec 15-mIg fusion protein. Average tumor size in each group is shown.
**Figure 27A** summarizes a proposed mechanism of action of Siglec 15, serving as a ligand. **Figure 27B** summarizes a proposed mechanism of action of Siglec 15, serving as a receptor.

### DETAILED DESCRIPTION OF THE INVENTION

The present invention is based, at least in part, on the discovery that Siglec 15 plays a critical role in suppression of the immune responses to cancer, thus, providing a rationale to manipulate the Siglec 15 pathway for future development of therapeutic cancer drugs. In particular, it has been discovered that a decrease in expression of Siglec 15 reduces tumor size and prolongs the survival of mice having brain tumors. In addition, inhibition of Siglec 15 activity by blocking the interaction between Siglec 15 and its ligands in a brain inflammation mouse model, as well as abolishing the expression of Siglec 15 in Siglec 15 knockout mice exacerbated the brain inflammation. Accordingly, the present invention provides methods of treating a cancer, methods of treating an autoimmune disease, methods of regulating an immune response to cancer, and methods of regulating an inflammation response in subjects by modulating the expression and/or activity of Siglec 15 as well as its binding partners in the same pathway.

### I. Definitions

In order that the present invention may be more readily understood, certain terms are first defined. Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition. Recitation of ranges of values herein are merely intended to serve as a shorthand method of referring individually to each separate value recited or falling within the range, unless otherwise indicated herein, and each separate value is incorporated into the specification as if it were individually recited.

In the following description, for purposes of explanation, specific numbers, materials and configurations are set forth in order to provide a thorough understanding of the invention. It will be apparent, however, to one having ordinary skill in the art that the invention may be practiced without these specific details. In some instances, well-known features may be omitted or simplified so as not to obscure the present invention. Furthermore, reference in the specification to phrases such as "one embodiment" or "an embodiment" means that a particular feature, structure or characteristic described in connection with the embodiment is included in at least one embodiment of the invention. The appearances of phrases such as "in one embodiment" in various places in the specification are not necessarily all referring to the same embodiment.

The use of the terms "a" and "an" and "the" and similar referents in the context of describing the invention (especially in the context of the following claims) are to be construed to cover both the singular and the plural (*i.e.*, one or more), unless otherwise indicated herein or clearly contradicted by context. By way of example, "an element" means one element or more than one element. The terms "comprising, "having," "including," and "containing" are to be construed as open-ended terms (*i*.*e*., meaning "including, but not limited to") unless otherwise noted.

As used herein, the term "Siglec15", also known as sialic acid binding Ig-like lectin 15, CD33 antigen-like 3, CD33 molecule-like 3, CD33L3 and HsT1361, is a member of the lectin family proteins which contains a sialic acid binding component and an Ig-like molecule. The sequence of a human Siglec15 mRNA can be found, for example, at GenBank Accession GI: 225637536 (NM_213602.2; SEQ ID NO: 1). The sequence of a human Siglec15 polypeptide sequence can be found, for example, at GenBank Accession No. GI:4 7106069 (NP_998767.1; SEQ ID NO: 2).

As used herein, the term "MAG", also known as myelin associated glycoprotein, Sialic Acid Binding Ig-Like Lectin 4A, SIGLEC4A or GMA, is a type I membrane protein and a member of the immunoglobulin superfamily. MAG is a functional receptor of NOGO-66 and is thought to participate in neuron myelination. MAG is also present in myeloid cells and especially in microglia. MAG knock-out (KO) mice have problems with phagocytosis. MAG binds to sialylated glycoconjugates and mediates certain myelin-neuron cell-cell interactions. Three alternatively spliced transcripts encoding different isoforms have been described for this gene. The sequence of a human MAG mRNA can be found, for example, at GenBank Accession GI: 312836849 (NM_002361.3; SEQ ID NO: 3). The sequence of a human MAG polypeptide sequence can be found, for example, at GenBank Accession No. GI: 11225258 (NP_002352.1; SEQ ID NO: 4).

As used herein, the term "LRRC4C", also known as leucine rich repeat (LRR) containing 4C, netrin-G1 ligand, NGL1, or KIAA1580, LRRC4C is an LRR family molecule associated with neuron-related growth, dendrite formation, and axon extension. LRRC4C is mainly localized to the postsynaptic side of excitatory synapses and interact with the presynaptic ligand, netrin-G1, to regulate excitatory synapse formation. The sequence of a human LRRC4C mRNA can be found, for example, at GenBank Accession GI: 385724810 (NM_020929.2; SEQ ID NO: 5). The sequence of a human LRRC4C polypeptide sequence can be found, for example, at GenBank Accession No. GI: 51317373 (NP_065980.1; SEQ ID NO: 6).

As used herein, the term "Sialyl-Tn", also known as "STn", "Sialyl-Tn antigen", and "Neu5Acα2-6GalNAcα-*O*-Ser/Thr", refers to the sialic acid-substituted form of Tn antigen. Tn antigen is an N-acetylgalactosamine (GalNAc) oligosaccharide linked to serine or threonine by a glycosidic bond, as an O-glycan. Sialyl-Tn is a truncated O-glycan containing a sialic acid α-2,6 linked to GalNAc α-*O*-Ser/Thr. Sialyl-Tn expression is caused by activation of an aberrant glycosylation pathway, and typically occurs in tumor cells. Biosynthesis of Sialyl-Tn has been linked to expression of the sialyltransferase ST6GalNAc1, and to mutations of COSMC (core 1 β3-Gal-T-specific molecular chaperone). Sialyl-Tn expression has been reported in over 80% of human carcinomas, including gastric, colon, breast, lung, oesophageal, prostate, and endometrial cancer, and is linked to poor prognosis (Munkley, Int. J. Mol. Sci. (2016), 17(3):275).

It should be noted that throughout, molecule names, *e*.*g*., Siglec15, MAG or LLRC4C, include both the gene and protein, unless otherwise specified. Thus, the term "Siglec15" when used in reference to the molecule includes both the Siglec15 protein and the Siglec15 gene.

The term "level of Siglec 15" includes levels of Siglec 15 mRNA or cDNA, and/or protein concentration, expression, activity, function, or stability of Siglec 15 protein, DNA, mRNA, or cDNA. In one embodiment, the term "level" as used herein refers to the measurable quantity of Siglec 15. The amount may be either (a) an absolute amount as measured in molecules, moles or weight per unit volume or cells or (b) a relative amount, *e*.*g*., measured by densitometric analysis.

As used herein, a "modulator of Siglec 15" refers to any compound or molecule that modulates the mRNA expression and/or protein expression of Siglec 15; and/or the mRNA and/or protein stability of Siglec 15; and/or the biological activity of Siglec 15. A modulator can modulate the expression and/or activity of Siglec 15 either directly or indirectly. A modulator of Siglec 15 acts directly on Siglec 15, *e.g.*, an antibody which binds to Siglec 15 and inhibits or activates its function. In another embodiment, the modulator of a Siglec 15 acts indirectly on Siglec 15 (*e.g.*, through another molecule, *e.g.*, a binding partner of Siglec 15) resulting in an increase or a decrease in the activity of Siglec 15. Exemplary agents suitable for use in the methods of the invention include interfering nucleic acid molecules (*e*.*g*., antisense RNAs, sdRNAs, and siRNAs), intracellular antibodies, recombinant fusion proteins, inhibitory peptides, or small molecules. Agents suitable for use in the methods of the invention are discussed in detail below.

As used herein, the various forms of the term "modulate" include stimulation (*e.g.*, increasing or up-regulating a particular response or activity) and inhibition (*e.g.*, decreasing or down-regulating a particular response or activity). In some embodiments, the modulator is an inhibitor of Siglec 15. In some embodiments, the modulator is an activator of Siglec 15.

As used herein, the term "inhibit" refers to a decrease in expression, stability, and/or a biological activity of Siglec 15. For example, the term "inhibit" refers to the ability to decrease or down-regulate the expression, stability, and/or activity of Siglec 15 as described herein.

As used herein, the term "stimulate" refers to an increase in expression, stability and/or a biological activity of Siglec 15. For example, the term "stimulate" refers to the ability to increase or up-regulate the expression, stability, and/or activity of Siglec 15 as described herein.

An "inhibitor of Siglec 15 activity," or a "Siglec 15 antagonist," as used herein, include any molecule that partially or fully blocks, inhibits, or neutralizes a biological activity mediated by Siglec 15. In some embodiments, a Siglec 15 antagonist inhibits an Siglec 15 polypeptide. In other embodiments, a Siglec 15 antagonist inhibits a ligand of Siglec 15, *e.g.*, MAG, LRRC4C, or Sialyl-Tn.

An "agonist of Siglec 15 activity," or a "Siglec 15 agonist," as used herein, includes any molecule that partially or fully stimulates or augments the biological activity of the antigen to which it binds. Exemplary Siglec 15 agonists bind to Siglec 15 and stimulate transduction of a signal through Siglec 15 upon binding, mimicking the interaction of Siglec 15 with a natural ligand, *e.g.*, MAG, LRRC4C, or Sialyl-Tn. Other exemplary agonists of Siglec 15 activity bind to and transduce a signal through a Siglec 15 ligand, *e.g.*, MAG, LRRC4C, or Sialyl-Tn, mimicking the interaction of the ligand with Siglec 15.

The term "antibody", as used herein, is intended to refer to immunoglobulin molecules comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH 1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino terminus to carboxy-terminus in the following order: FR1, CDR1, FR1, CDR2, FR3, CDR3, FR4.

The term "antigen-binding portion" or "antigen-binding fragment" of an antibody (or simply "antibody portion"), as used herein, refers to a portion of a full-length antibody, generally the target binding or variable region. Examples of antibody fragments include Fab, Fab', F(ab')₂ and Fv fragments. The phrase "functional fragment" of an antibody is a compound having qualitative biological activity in common with a full-length antibody. For example, a functional fragment of an antagonistic anti-Siglec 15 antibody can bind to Siglec 15 in such a manner so as to block, inhibit, or neutralize a biological activity mediated by Siglec 15. As used herein, "functional fragment" with respect to antibodies, refers to Fv, scFv, diabody, F(ab) and F(ab')₂ fragments. An "Fv" fragment is the minimum antibody fragment which contains a complete target recognition and binding site. This region consists of a dimer of one heavy and one light chain variable domain in a tight, non-covalent association (VH-VL dimer). It is in this configuration that the three CDRs of each variable domain interact to define a target binding site on the surface of the VH-VL dimer. An scFv contains one heavy and one light chain variable domain connected by a linker peptide of a size that permits the VH and VL domains to interact to form the target binding site. Collectively, the six CDRs confer target binding specificity to the antibody or antibody fragment. However, even a single variable domain (or half of an Fv comprising only three CDRs specific for a target) can have the ability to recognize and bind target, although at a lower affinity than the entire binding site.

The terms "antagonist antibody" or "blocking antibody" as used herein refer to an antibody which inhibits or reduces the biological activity of the antigen to which it binds. Exemplary antagonist antibodies substantially or completely inhibit the biological activity of the antigen.

The term "agonist antibody" as used herein refer to an antibody which stimulates or augments the biological activity of the antigen to which it binds. Exemplary agonist antibodies stimulate transduction of a signal through the antigen upon binding, mimicking the interaction of the antigen with a natural ligand.

The term "subject" is used herein to refer to an animal, such as a mammal, including a primate (such as a human, a non-human primate, *e*.*g*., a monkey, and a chimpanzee), a non-primate (such as a cow, a pig, a camel, a llama, a horse, a goat, a rabbit, a sheep, a hamster, a guinea pig, a cat, a dog, a rat, a mouse, and a whale), a bird (*e*.*g*., a duck or a goose), and a shark. In an embodiment, the subject is a human, such as a human being treated or assessed for a disease, disorder or condition, a human at risk for a disease, disorder or condition, a human having a disease, disorder or condition, and/or human being treated for a disease, disorder or condition as described herein. In some embodiments, the subject does not suffer from an ongoing autoimmune disease. In one embodiment, the subject is about 1, 2, 3, 4, 5, 6, 7, 8, 9, or 10 years of age. In another embodiment, the subject is about 5-10, 10-15, 15-20, 20-25, 25-30, 30-35, 35-40, 40-45, 45-50, 50-55, 55-60, 60-65, 65-70, 70-75, 75-80, 80-85, 85-90, 90-95, 95-100 years of age. Values and ranges intermediate to the above recited ranges are also intended to be part of this invention. In addition, ranges of values using a combination of any of the above-recited values as upper and/or lower limits are intended to be included.

As used herein, the terms "treating" or "treatment" refer to a beneficial or desired result including, but not limited to, alleviation or amelioration of one or more symptoms, diminishing the extent of a disorder, stabilized (*i*.*e*., not worsening) state of a disorder, amelioration or palliation of the disorder, whether detectable or undetectable. "Treatment" can also mean prolonging survival as compared to expected survival in the absence of treatment.

As used herein, the term "effective amount" refers to the amount of a therapy, which is sufficient to reduce or ameliorate the severity and/or duration of a disorder or one or more symptoms thereof, inhibit or prevent the advancement of a disorder, cause regression of a disorder, inhibit or prevent the recurrence, development, onset or progression of one or more symptoms associated with a disorder, detect a disorder, or enhance or improve the prophylactic or therapeutic effect(s) of another therapy (e.g., prophylactic or therapeutic agent). An effective amount can require more than one dose.

### II. Methods of the Invention

The present invention is based, at least in part, on the discovery that Siglec 15 has a critical role in immune regulation. Siglec 15 has been extensively studied in osteoclast differentiation, where it acts in a non-immunological role. In addition to its role in osteoclast differentiation, Siglec 15 regulates the immune response by suppressing the activity of immune cells, including T cells and macrophages, as described herein. Accordingly, Siglec 15 upregulation in cancer cells or in the tumor microenvironment may repress a subject's immune response against the cancer. Moreover, deficiency in Siglec 15 may contribute to conditions related to autoimmunity.

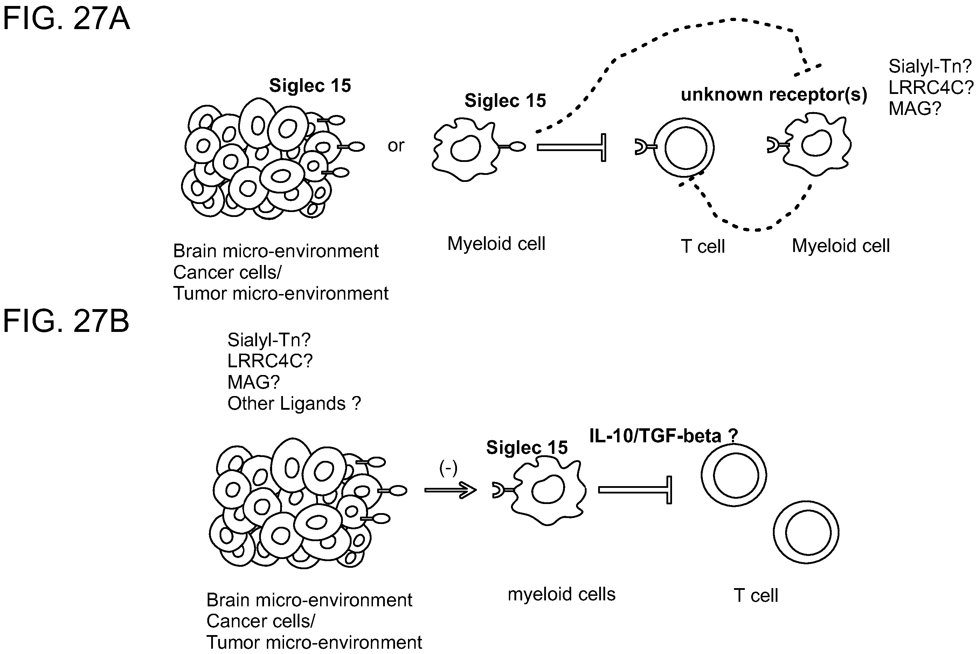

Without wishing to be bound by theory, it is proposed that Siglec 15 can serve an immunomodulatory function by acting as a ligand, and/or as a receptor (Figure 27). Siglec 15 is expressed in myeloid cells, and is over-expressed in the brain microenvironment, in cancer cells, and in the tumor microenvironment. When over-expressed in the brain, cancer cells, or in the tumor microenvironment, Siglec 15 may function as a ligand, where it can affect T cell responses directly, or indirectly through myeloid cells. Both T cells and myeloid cells may express receptor(s) responsible for Siglec 15's immune repressive function (Figure 27A). In addition or in the alternative, the expression of Siglec 15 ligands in the brain microenvironment, in cancer cells, or in the tumor microenvironment may induce signal transduction through Siglec 15 acting as a receptor (Figure 27B). Accordingly, Siglec 15 may indirectly affect T cell responses through myeloid:T cell interactions, or immune suppressive cytokines, such as IL-10 and TGF-beta.

In various aspects, the present invention is directed to methods of modulating immune function by promoting or inhibiting the activity of Siglec 15.

### A. Methods of Increasing Immune Response by Decreasing the Expression and/or Activity of Siglec 15

In one aspect, the present invention provides methods for increasing an immune or inflammatory response in a subject, by administering to the subject an effective amount of a modulator of Siglec 15, wherein the modulator decreases the expression and/or activity of Siglec 15 in the subject. In one embodiment, this method can be used to increase the number of T cells, *e*.*g*., CD4 T cells and/or CD8 T cells, in a subject. In another embodiment, this method can be used to increase the activity of T cells, *e*.*g*., CD4 T cells and/or CD8 T cells, in a subject.

The foregoing method can be used to treat a disorder which would benefit from increasing or augmenting the immune response in a subject. For example, a modulator of Siglec 15 that decreases the expression and/or activity of Siglec 15 can be used to treat a cancer in a subject in need thereof. Such methods can include administering to a subject in need thereof, an effective amount of a modulator of Siglec 15, wherein the modulator decreases the expression and/or activity levels of Siglec 15 in a subject, thereby treating a cancer in the subject.

In another aspect, the present invention provides methods for reducing a tumor size in a subject in need thereof. The methods include administering to a subject in need thereof, an effective amount of a modulator of Siglec 15, wherein the modulator decreases the expression and/or activity levels of Siglec 15 in a subject, thereby reducing the tumor size in the subject.

In one aspect, the present invention features methods for prolonging the survival of a subject in need thereof. The methods include administering to a subject in need thereof, an effective amount of a modulator of Siglec 15, wherein the modulator decreases the expression and/or activity levels of Siglec 15 in a subject, thereby prolonging the survival of the subject.

In another aspect, the present invention features methods for increasing an immune response against a tumor in a subject in need thereof. The methods include administering to a subject in need thereof, an effective amount of a modulator of Siglec 15, wherein the modulator decreases the expression and/or activity levels of Siglec 15 in a subject, thereby increasing an immune response against the tumor in the subject.

The modulator of Siglec 15 suitable for use in the methods of the present invention includes any compound or molecule that can regulate the expression and/or activity of Siglec 15, for example, the mRNA expression and/or protein expression of Siglec 15; the mRNA and/or protein stability of Siglec 15; and/or the biological activity of Siglec 15. A modulator can modulate the expression and/or activity of Siglec 15 either directly or indirectly. In some embodiment, the modulators inhibit the expression and/or activity of Siglec 15. Inhibitory modulators of Siglec 15 can act directly on Siglec 15, *e.g.*, an antagonist antibody which binds to Siglec 15 and inhibits its function. Alternatively, inhibitory modulators of a Siglec 15 act indirectly on Siglec 15 (*e.g.*, through another molecule, *e.g.*, a binding partner of Siglec 15) resulting in a decreased activity. Exemplary modulators suitable for use in the methods of the invention include small molecule inhibitors, antagonist antibodies, or antigen-binding fragment thereof, recombinant fusion proteins (*e*.*g*., soluble Siglec 15 fusion proteins, for example, soluble Siglec 15-Fc), inhibitory peptides, or interfering nucleic acid molecules (*e*.*g*., antisense RNAs, sdRNAs, and siRNAs). Modulators suitable for use in the methods of the invention are discussed in detail below.

The modulator of Siglec 15 of the present invention may also block interaction between Siglec 15 and a binding ligand. Binding ligands of Siglec 15 can be identified by any methods known in the art. For example, protein-protein interaction between Siglec 15 and binding ligands can be identified by co-immunoprecipitation in which the binding of a pair of proteins of interest is determined by forming a co-precipitate with an antibody *in vitro.* Alternatively, the yeast two-hybrid or phage display approach may be employed to screen for binding ligands of Siglec 15. In some embodiments, chemical cross-linking assays followed by mass spectrometry analysis can be used to identify interacting proteins.

In some embodiment, the binding ligands for Siglec 15 suitable for use in the present invention can be identified by a Receptor Array Technology. The Receptor Array Technology is a well-established technology for screening counter-receptors of target proteins (Zhu Y et al, Nat Commun, 4: 2043, 2013; Yao S et al, Immunity, 34(5): 729-740, 2011). Briefly, the receptor array comprises a solid support structure comprising a plurality of wells wherein each of the plurality of wells comprises a cell that has been transfected with a gene encoding a receptor protein. A target gene (encoding a secreted protein) or the extracellular domain of the target gene (encoding a transmembrane protein, *e.g.*, an immune modulator) is genetically fused to a tag gene (mouse IgG2a Fc, human IgG1 Fc, FLAG, or 6xHIS), to prepare fusion genes as described previously (Chapoval, AI. et al., Mol Biotechnol 21(3): 259-264, 2002). Upon transfection of individual fusion genes into 293T cells, the purified recombinant fusion protein is used to screen against the Receptor Array. A fluorescence-labeled secondary antibody against the tag is applied to detect the binding of the target protein, *e.g.*, an immune modulator identified based on the methods of the present invention, to the transfected 293T cells and is screened using the Applied Biosystems 8200 Cellular Detection System and analyzed by CDS 8200 software. The entire contents of the foregoing references are incorporated herein by reference.

In some embodiments, the binding ligand of Siglec 15 is MAG. In other embodiments, the binding ligand of Siglec 15 is LRRC4C. In other embodiments, the binding ligand of Siglec 15 is Sialyl-Tn.

Binding sites on Siglec 15 for its ligands, *e.g.*, MAG, LRRC4C, and/or Sialyl-Tn, may be determined by any methods known in the art. For example, Siglec 15 mutant proteins with domain deletions can be generated and purified using standard methods known in the art. In some embodiments, the modulator suitable for use in the methods of the present invention is a mutant Siglec 15 protein with a reduced activity for ligand binding. For example, a modulator can be a mutant Siglec protein with a single substitution mutation at residue 143. In other embodiments, a modulator suitable for use in the methods of the present invention is a mutant Siglec 15 protein that has a deletion of the IgV domain.

The modulators of Siglec 15 suitable for use in the methods of the present invention may increase an immune response against a tumor by inhibiting the activity involved in the Siglec 15 pathway, for example, by decreasing the expression and/or activity of any binding ligands of Siglec 15, *e.g.*, MAG, LRRC4C, or Sialyl-Tn. In some embodiments, the modulator suitable for use in the methods of the present invention is a small molecule inhibitor of MAG or LRRC4C, an antagonist antibody for MAG or LRRC4C, or antigen-binding fragment thereof, a recombinant MAG fusion protein that is inhibitory in nature, a recombinant LRRC4C fusion protein that is inhibitory in nature, or an inhibitory protein or nucleic acid targeting MAG or LRRC4C. In other embodiments, the modulator is an antibody, or antigen-binding fragment thereof, that specifically binds Sialyl-Tn. Such an antibody, or antigen-binding fragment thereof, can bind Sialyl-Tn and mask its association with Siglec 15. In other embodiments, the modulator is a soluble Sialyl-Tn molecule, optionally coupled to a scaffold, *e.g.*, a scaffold peptide. Modulators suitable for use in the methods of the invention are discussed in detail below.

The foregoing methods can be used to treat a disorder which would benefit from increasing or augmenting the immune response in a subject. Such disorders include, but are not limited to, cancer. Administration of a Siglec 15 modulator that decreases the expression and/or activity of Siglec 15 can be used, for example, to stimulate an immune response against a cancer (*e*.*g*., stimulate a T cell response against a cancer), reduce tumor size, and/or prolong survival of a subject having cancer.

As described herein, the term "cancer" refers to one of a group of diseases caused by the uncontrolled, abnormal proliferation of cells that can spread to adjoining tissues or other parts of the body. Cancer cells can form a solid tumor, in which the cancer cells are massed together, or exist as dispersed cells, as in leukemia. Types of cancer that are suitable to be treated by decreasing the expression or activity of Siglec 15 include, but are not limited to, solid tumors and/or hematological cancers. In one embodiment, the cancer is of epithelial origin. Exemplary types of cancer that can be treated by the foregoing methods include, but are not limited to, adrenal cancer, anal cancer, bile duct cancer, bladder cancer, bone cancer, brain/CNS tumors, breast cancer, castleman disease, cervical cancer, colon/rectum cancer, endometrial cancer, esophagus cancer, eye cancer, gallbladder cancer, gastrointestinal cancer, kidney cancer, laryngeal and hypopharyngeal cancer, leukemia, liver cancer, lung cancer, lymphoma, lymphoma of the skin, malignant mesothelioma, multiple myeloma, myelodysplastic syndrome, nasal cavity and paranasal sinus cancer, nasopharyngeal cancer, neuroblastoma, non-hodgkin lymphoma, oral cavity and oropharyngeal cancer, osteosarcoma, ovarian cancer, pancreatic cancer, penile cancer, pituitary tumors, prostate cancer, retinoblastoma, rhabdomyosarcoma, salivary gland cancer, skin cancer, small intestine cancer, stomach cancer, testicular cancer, thymus cancer, thyroid cancer, uterine sarcoma, vaginal cancer, vulvar cancer, waldenstrom macroglobulinemia, and wilms tumor. In some embodiments, the cancer is selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma.

In some embodiments, the cancer is colon cancer, endometriod cancer, kidney cancer (*e*.*g*., kidney papillary cell carcinoma, kidney clear cell carcinoma), liver cancer, thyroid cancer, lung cancer (*e*.*g*., lung adenocarcinoma, lung squamous cell carcinoma), head and neck cancer, breast cancer, cervical cancer, prostate cancer, bladder cancer, glioblastoma, rectal cancer, or bile duct cancer. In one embodiment, the cancer is brain cancer. In one embodiment, the cancer is glioblastoma. In one embodiment, the cancer is not a blood-derived cancer. In one embodiment, the cancer is not leukemia. In one embodiment, the cancer is not acute myeloid leukemia (AML).

Use of an "effective amount" of the modulators of the present invention (and therapeutic compositions comprising such modulators) is an amount effective, at dosages and for periods of time necessary to achieve the desired result. For example, an effective amount of a modulator may vary according to factors such as the disease state, age, sex, reproductive state, and weight, and the ability of the agent to elicit a desired response in the organism. Dosage regimens may be adjusted to provide the optimum response. For example, several divided doses may be provided daily or the dose may be proportionally reduced as indicated by the exigencies of the situation.

"Treat," as used herein, refers to an intervention that imparts a benefit to a patient, *e*.*g*., a patient afflicted with or at risk for developing a disease. Treating includes actions taken and actions refrained from being taken for the purpose of improving the condition of the patient, *e*.*g*., the relief of one or more symptoms, or delay in the onset or progression of the disease.

In one embodiment, the invention relates to a combination therapy for treating cancer, wherein a Siglec 15 modulator that decreases the expression and/or activity of Siglec 15 is administered to a subject in combination with a checkpoint inhibitor, *e*.*g*., an antagonist of PD-L1, or an antagonist of PD-1. PD-1 is a checkpoint protein on T cells, which keeps T cells from attacking cells in the body that express PD-L1. Some cancer cells overexpress PD-L1, which enables them to evade detection by T cells. Inhibitors of PD-L1 and PD-1 can boost the immune response against cancer cells, and can synergistically promote tumor cell killing when used in conjunction with an antagonist of Siglec 15 activity. Exemplary anti-PD-L1 inhibitory antibodies that may be used in conjunction with an antagonist of Siglec 15 activity include, but are not limited to, atezolizumab (Genentech), avelumab (Pfizer), and durvalumab (AstraZeneca). Exemplary anti-PD-1 inhibitory antibodies that may be used in conjunction with an antagonist of Siglec 15 activity include, but are not limited to, pembrolizumab (Merck) and nivolumab (Bristol-Myers Squibb).

In some embodiments, the foregoing methods further comprise a screening step, wherein patients having a cancer are screened for upregulation of Siglec 15 in the cancer or in the tumor microenvironment. For example, in one embodiment, a biological sample containing cancer cells is obtained from the subject, and Siglec 15 expression is determined, and compared to a suitable control, *e*.*g*., a comparable sample obtained from a normal subject, a reference value indicative of the level of expression in a normal subject, etc. Upregulation of Siglec 15 in the biological sample containing cancer cells can indicate that the subject would benefit from an agent that decreases the expression or activity of Siglec 15.

In some embodiments, the foregoing methods can comprise a screening step, wherein patients with an ongoing autoimmune disease are excluded from treatment with a Siglec 15 modulator. In other embodiments, the modulator of Siglec 15 suitable for use in the methods of the present invention will not cause a non-specific autoimmune disease in a subject. Subjects receiving the treatment comprising the Siglec 15 modulator may be selected such that they will not develop an autoimmune disease spontaneously.

### B. Methods of Decreasing Inflammation by Increasing the Expression and/or Activity of Siglec 15

Another aspect of the present invention provides methods for decreasing an unwanted immune or inflammatory response in a subject, by administering to the subject an effective amount of a modulator of Siglec 15, wherein the modulator increases the expression and/or activity of Siglec 15 in the subject. In one embodiment, this method can be used to decrease the number of T cells, *e.g.*, CD4 T cells and/or CD8 T cells, in a subject. In another embodiment, this method can be used to decrease the activity of T cells, *e.g.*, CD4 T cells and/or CD8 T cells, in a subject.

The foregoing method can be used to treat a disorder which would benefit from reducing an immune response or an inflammatory response in a subject. For example, a modulator of Siglec 15 that increases the expression and/or activity of Siglec 15 can be used to treat an autoimmune disease in a subject in need thereof. The methods include administering to the subject an effective amount of a modulator of Siglec 15, wherein the modulator increases the expression and/or activity of Siglec 15 in the subject, thereby treating an autoimmune disease in the subject.

Another aspect of the present invention provides methods of treating an autoimmune disease in a subject in need thereof. The methods include administering to the subject an effective amount of Siglec 15 protein, or a nucleic acid encoding the Siglec 15 protein, thereby treating an autoimmune disease in the subject.

In one aspect, the present invention features methods of decreasing a brain inflammatory response in a subject in need thereof. The methods include administering to the subject an effective amount of a modulator of Siglec 15, wherein the modulator increases the expression and/or activity levels of Siglec 15, thereby decreasing a brain inflammatory response in the subject.

In another aspect, the present invention provides methods of decreasing a brain inflammatory response in a subject in need thereof. The methods include administering to the subject an effective amount of Siglec 15 protein, or a nucleic acid encoding the Siglec 15 protein, thereby decreasing a brain inflammatory response in the subject.

The modulator of Siglec 15 suitable for use in the foregoing methods includes any compound or molecule that can positively regulate the expression and/or activity of Siglec 15, for example, by modulating the mRNA expression and/or protein expression of Siglec 15; the mRNA and/or protein stability of Siglec 15; and/or the biological activity of Siglec 15. A modulator can modulate the expression and/or activity of Siglec 15 either directly or indirectly. In some embodiments, the modulators increase the expression and/or activity of Siglec 15. Stimulatory modulators of Siglec 15 can act directly on Siglec 15, *e.g.*, an agonist antibody which binds to Siglec 15 and stimulates its function. In other embodiments, the stimulatory modulator of a Siglec 15 acts indirectly on Siglec 15 (*e.g.*, through another molecule, *e.g.*, a binding partner of Siglec 15) resulting in an increased activity. Exemplary modulators suitable for use in the methods of the invention include small molecule activators, agonist antibodies, or antigen-binding fragment thereof, or a protein and a nucleic acid that activates the transcription and/or translation of Siglec 15. Alternatively, a modulator that increases the expression and/or activity of Siglec 15 for use in the methods of the present invention includes a Siglec 15 protein, or a nucleic acid encoding the Siglec 15 protein. In some embodiments, the Siglec 15 protein is selected from the group consisting of a full-length Siglec 15 protein, a functional fragment of Siglec 15, or an IgV domain of Siglec 15. Modulators suitable for use in the methods of the invention are discussed in detail below.

The modulator of Siglec 15 of the present invention may also promote interaction between Siglec 15 and a binding ligand. Binding ligands of Siglec 15 can be identified by any methods known in the art. For example, protein-protein interaction between Siglec 15 and binding ligands can be identified by an co-immunoprecipitation assay in which the binding of a pair of proteins of interest is determined by forming a co-precipitate with an antibody *in vitro.* Alternatively, yeast two-hybrid or phage display assays may be used to screen for binding ligands for Siglec 15. In some embodiments, chemical cross-linking assays followed by mass spectrometry can be used to analyze interacting proteins.

In some embodiments, the binding ligands for Siglec 15 suitable for use in the present invention can be identified by a Receptor Array Technology as described herein.

In some embodiments, the binding ligand of Siglec 15 is MAG. In other embodiments, the binding ligand of Siglec 15 is LRRC4C. In other embodiments, the binding ligand of Siglec 15 is Sialyl-Tn.

Exemplary modulators of Siglec 15 suitable for use in practicing certain embodiments of the methods described herein may decrease inflammation by stimulating an activity involved in the Siglec 15 pathway, for example, by increasing the expression and/ activity of binding ligands of Siglec 15, *e.g.*, MAG, LRRC4C, and/or Sialyl-Tn. In some embodiments, the modulator suitable for use in the methods of the present invention is a small molecule activator of MAG or LRRC4C, an agonist antibody for MAG or LRRC4C, or antigen-binding fragment thereof, a protein or a nucleic acid that activates the transcription and/or translation of MAG or LRRC4C. In other embodiments, the modulator suitable for use in the methods of the present invention is a MAG protein or a nucleic acid encoding the MAG protein. In another embodiment, the modulator suitable for use in the methods of the present invention is an LRRC4C protein or a nucleic acid encoding the LRRC4C protein. In one embodiment, the modulator is a synthetic Sialyl-Tn molecule. For example, Sialyl-Tn can be administered alone or coupled, *e.g.*, covalently or non-covalently conjugated, to a scaffold molecule. In one embodiment, the modulator is a synthetic peptide bearing Sialyl-Tn. Sialyl-Tn can bind to and activate Siglec 15, serving as an agonist of Siglec 15 activity. Modulators that increase the expression and/or activity of Siglec 15 suitable for use in the methods of the invention are discussed in detail below.

Diseases that may be treated with the methods of the present invention include, but are not limited to, autoimmune diseases or cancer. "Treat" refers to any type of treatment that imparts a benefit to a patient, *e.g.*, a patient afflicted with or at risk for developing a disease. Treating includes actions taken and actions refrained from being taken for the purpose of improving the condition of the patient, *e.g.*, the relief of one or more symptoms, or delay in the onset or progression of the disease.

As described herein, "autoimmune diseases" are those diseases that arise from an abnormal immune response of the body against substances and tissues normally present in the body, and include, but are not limited to, rheumatoid arthritis (RA), juvenile chronic arthritis (JCA), thyroiditis, graft versus host disease (GVHD), scleroderma, diabetes mellitus, Graves' disease, allergy, acute or chronic immune disease associated with an allogenic transplantation, such as, but not limited to, renal transplantation, cardiac transplantation, bone marrow transplantation, liver transplantation, pancreatic transplantation, small intestine transplantation, lung transplantation and skin transplantation. In some embodiments, the autoimmune disease is an inflammatory brain disease. In other embodiments, the inflammatory brain disease is multiple sclerosis. In other embodiments, the inflammatory brain disease is experimental autoimmune encephalomyelitis (EAE).

### III. Pharmaceutical Formulations

Pharmaceutical formulations comprising Siglec 15 or a modulator of Siglec 15 of the present invention may be prepared by mixing the protein or nucleic acid having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*., Zn-protein complexes); and/or non-ionic surfactants such as Tween^{™}, Pluronics^{™} or polyethylene glycol (PEG).

The formulations herein may also contain more than one active compound as necessary for the particular indication being treated. For example, if an autoimmune disease is to be treated, the formulations of the invention containing Siglec 15 or a modulator of Siglec 15 may be combined with drugs that are known to treat autoimmune diseases, such as methylprednisolone, kenalog, medrol, prednisolone, cortef, hydrocortisone, cortisone, triamcinolone acetonide, celestone soluspan, methylprednisolone acetate, orapred ODT, veripred 20, Solu-Medrol or methylprednisolone sodium. If cancer is to be treated, the formulations of the invention containing Siglec 15 or a modulator of Siglec 15 may be combined with drugs that are known to treat cancer, such as Abiraterone Acetate, ABITREXATE (Methotrexate), ABRAXANE (Paclitaxel Albumin-stabilized Nanoparticle Formulation), ADCETRIS (Brentuximab Vedotin), Ado-Trastuzumab Emtansine, ADRIAMYCIN (Doxorubicin Hydrochloride), ADRUCII, (Fluorouracil), Afatinib Dimaleate, AFINITOR (Everolimus), ALDARA (Imiquimod), Aldesleukin, Alemtuzumab, ALIMTA (Pemetrexed Disodium), ALOXI (Palonosetron Hydrochloride), AMBOCHLORIN (Chlorambucil), AMBOCLORIN (Chlorambucil), Aminolevulinic Acid, Anastrozole, Aprepitant, AREDIA (Pamidronate Disodium), ARIMIDEX (Anastrozole), AROMASIN (Exemestane), ARRANON (Nelarabine), Arsenic Trioxide, ARZERRA (Ofatumumab), Asparaginase Erwinia chrysanthemi, AVASTIN (Bevacizumab), Axitinib, Azacitidine, Bendamustine Hydrochloride, Bevacizumab, Bexarotene, BEXXAR (Tositumomab and I 131 Iodine Tositumomab), Bleomycin, Bortezomib, BOSULIF (Bosutinib), Cabazitaxel, Cabozantinib-S-Malate, CAMPATH (Alemtuzumab), CAMPTOSAR (Irinotecan Hydrochloride), Capecitabine, Carboplatin, Carfilzomib, CEENU (Lomustine), CERUBIDINE (Daunorubicin Hydrochloride), Cetuximab, Chlorambucil, Cisplatin, CLAFEN (Cyclophosphamide), Clofarabine, COMETRIQ (Cabozantinib-S-Malate), COSMEGEN (Dactinomycin), Crizotinib, Cyclophosphamide, CYFOS (Ifosfamide), Cytarabine, Dabrafenib, Dacarbazine, DACOGEN (Decitabine), Dactinomycin, Dasatinib, Daunorubicin Hydrochloride, Decitabine, Degarelix, Denileukin Diftitox, Denosumab, Dexrazoxane Hydrochloride, Docetaxel, Doxorubicin Hydrochloride, EFUDEX (Fluorouracil), ELITEK (Rasburicase), ELLENCE (Epirubicin Hydrochloride), ELOXATIN (Oxaliplatin), Eltrombopag Olamine, EMEND (Aprepitant), Enzalutamide, Epirubicin Hydrochloride, ERBITUX (Cetuximab), Eribulin Mesylate, ERIVEDGE (Vismodegib), Erlotinib Hydrochloride, ERWINAZE (Asparaginase Erwinia chrysanthemi), Etoposide, Everolimus, EVISTA (Raloxifene Hydrochloride), Exemestane, FARESTON (Toremifene), FASLODEX (Fulvestrant), FEMARA (Letrozole), Filgrastim, FLUDARA (Fludarabine Phosphate), Fludarabine Phosphate, FLUOROPLEX (Fluorouracil), Fluorouracil, Folinic acid, FOLOTYN (Pralatrexate), Fulvestrant, Gefitinib, Gemcitabine Hydrochloride, Gemtuzumab Ozogamicin, GEMZAR (Gemcitabine Hydrochloride), GILOTRIF (Afatinib Dimaleate), GLEEVEC (Imatinib Mesylate), HALAVEN (Eribulin Mesylate), HERCEPTIN (Trastuzumab), HYCAMTIN (Topotecan Hydrochloride), Ibritumomab Tiuxetan, ICLUSIG (Ponatinib Hydrochloride), Ifosfamide, Imatinib Mesylate, Imiquimod, INLYTA (Axitinib), INTRON A (Recombinant Interferon Alfa-2b), Iodine 131 Tositumomab and Tositumomab, Ipilimumab, IRESSA (Gefitinib), Irinotecan Hydrochloride, ISTODAX (Romidepsin), Ixabepilone, JAKAFI (Ruxolitinib Phosphate), JEVTANA (Cabazitaxel), Kadcyla (Ado-Trastuzumab Emtansine), KEOXIFENE (Raloxifene Hydrochloride), KEPIVANCE (Palifermin), KYPROLIS (Carfilzomib), Lapatinib Ditosylate, Lenalidomide, Letrozole, Leucovorin Calcium, Leuprolide Acetate, Lomustine, LUPRON (Leuprolide Acetate, MARQIBO (Vincristine Sulfate Liposome), MATULANE (Procarbazine Hydrochloride), Mechlorethamine Hydrochloride, MEGACE (Megestrol Acetate), Megestrol Acetate, MEKINIST (Trametinib), Mercaptopurine, Mesna, METHAZOLASTONE (Temozolomide), Methotrexate, Mitomycin, MOZOBIL (Plerixafor), MUSTARGEN (Mechlorethamine Hydrochloride), MUTAMYCIN (Mitomycin C), MYLOSAR (Azacitidine), MYLOTARG (Gemtuzumab Ozogamicin), Nanoparticle Paclitaxel (Paclitaxel Albumin-stabilized Nanoparticle Formulation), NAVELBINE (Vinorelbine Tartrate), Nelarabine, NEOSAR (Cyclophosphamide), NEUPOGEN (Filgrastim), NEXAVAR (Sorafenib Tosylate), Nilotinib, NOLVADEX (Tamoxifen Citrate), NPLATE (Romiplostim), Ofatumumab, Omacetaxine Mepesuccinate, ONCASPAR (Pegaspargase), ONTAK (Denileukin Diftitox), Oxaliplatin, Paclitaxel, Paclitaxel Albumin-stabilized Nanoparticle Formulation, Palifermin, Palonosetron Hydrochloride, Pamidronate Disodium, Panitumumab, Pazopanib Hydrochloride, Pegaspargase, Peginterferon Alfa-2b, PEG-INTRON (Peginterferon Alfa-2b), Pemetrexed Disodium,, Pertuzumab, PLATINOL (Cisplatin), PLATINOL-AQ (Cisplatin), Plerixafor, Pomalidomide, POMALYST (Pomalidomide), Ponatinib Hydrochloride, Pralatrexate, Prednisone, Procarbazine Hydrochloride, PROLEUKIN (Aldesleukin), PROLIA (Denosumab), PROMACTA (Eltrombopag Olamine), PROVENGE (Sipuleucel-T), PURINETHOL (Mercaptopurine), Radium 223 Dichloride, Raloxifene Hydrochloride, Rasburicas, Recombinant Interferon Alfa-2b, Regorafenib, REVLIMID (Lenalidomide), RHEUMATREX (Methotrexate), Rituximab, Romidepsin, Romiplostim, RUBIDOMYCIN (Daunorubicin Hydrochloride), Ruxolitinib Phosphat, Sipuleucel-T, Sorafenib Tosylate, SPRYCEL (Dasatinib), STIVARGA (Regorafenib), Sunitinib Malate, SUTENT (Sunitinib Malate), SYLATRON (Peginterferon Alfa-2b), SYNOVIR (Thalidomide), SYNRIBO (Omacetaxine Mepesuccinate), TAFINLAR (Dabrafenib), Tamoxifen Citrate, TARABINE PFS (Cytarabine), TARCEVA (Erlotinib Hydrochloride), TARGRETIN (Bexarotene), TASIGNA (Nilotinib), TAXOL (Paclitaxel), TAXOTERE (Docetaxel), TEMODAR (Temozolomide), Temozolomide, Temsirolimus, Thalidomide, TOPOSAR (Etoposide), Topotecan Hydrochloride, Toremifene, TORISEL (Temsirolimus), Tositumomab and I 131 Iodine Tositumomab, TOTECT (Dexrazoxane Hydrochloride), Trametinib, Trastuzumab, TREANDA (Bendamustine Hydrochloride), TRISENOX (Arsenic Trioxide), TYKERB (Lapatinib Ditosylate), Vandetanib, VECTIBIX (Panitumumab), VelP, VELBAN (Vinblastine Sulfate), VELCADE (Bortezomib), VELSAR (Vinblastine Sulfate), Vemurafenib, VEPESID (Etoposide), VIADUR (Leuprolide Acetate), VIDAZA (Azacitidine), Vinblastine Sulfate, Vincristine Sulfate, Vinorelbine Tartrate, Vismodegib, VORAXAZE (Glucarpidase), Vorinostat, VOTRIENT (Pazopanib Hydrochloride), WELLCOVORIN (Leucovorin Calcium), XALKORI (Crizotinib), XELODA (Capecitabine), XGEVA (Denosumab), XOFIGO (Radium 223 Dichloride), XTANDI (Enzalutamide), YERVOY (Ipilimumab), ZALTRAP (Ziv-Aflibercept), ZELBORAF (Vemurafenib), ZEVALIN (Ibritumomab Tiuxetan), ZINECARD (Dexrazoxane Hydrochloride), Ziv- Aflibercept, Zoledronic Acid, ZOLINZA (Vorinostat), ZOMETA (Zoledronic Acid), and ZYTIGA (Abiraterone Acetate). In one embodiment, the modulator of Siglec 15 can be combined with a PD-L1 antagonist, *e*.*g*., an anti-PD-L1 antagonist antibody, or an antigen-binding portion thereof. In another embodiment, he modulator of Siglec 15 can be combined with a PD-1 antagonist, *e*.*g*., an anti-PD-1 antagonist antibody, or an antigen-binding portion thereof.

The active ingredients may also be packaged in a microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Generally, the ingredients of compositions are supplied either separately or mixed together in unit dosage form, for example, as a dry lyophilized powder or water free concentrate in a hermetically sealed container such as an ampoule or sachet indicating the quantity of active agent. Where the mode of administration is infusion, composition can be dispensed with an infusion bottle containing sterile pharmaceutical grade water or saline. Where the mode of administration is by injection, an ampoule of sterile water for injection or saline can be provided so that the ingredients may be mixed prior to administration. In an alternative embodiment, one or more of the pharmaceutical compositions of the invention is supplied in liquid form in a hermetically sealed container indicating the quantity and concentration of the agent.

The active agent can be incorporated into a pharmaceutical composition suitable for parenteral administration, typically prepared as an injectable solution. The injectable solution can be composed of either a liquid or lyophilized dosage form in a flint or amber vial, ampule or pre-filled syringe. The liquid or lyophilized dosage may further comprise a buffer (*e*.*g*., L-histidine, sodium succinate, sodium citrate, sodium phosphate or potassium phosphate, sodium chloride), a cryoprotectant (*e*.*g*., sucrose trehalose or lactose, a bulking agent (*e.g*., mannitol), a stabilizer (e.g., L-Methionine, glycine, arginine), an adjuvant (hyaluronidase).

The compositions of this invention, *e*.*g*., a Siglec 15 or a modulator of Siglec 15, may be in a variety of forms. These include, for example, liquid, semi-solid and solid dosage forms, such as liquid solutions (*e*.*g*., injectable and infusible solutions), microemulsion, dispersions, liposomes or suspensions, tablets, pills, powders, liposomes and suppositories. The preferred form depends on the intended mode of administration and therapeutic application. Pharmaceutical compositions comprising Siglec 15 or a modulator of Siglec 15 described herein may be formulated for administration to a particular tissue. For example, in certain embodiments, it may be desirable to administer Siglec 15 or a modulator of Siglec 15 to connective tissue, brain tissue, and/or tumor sites in a variety of organs.

Siglec 15 and the modulators of Siglec 15 suitable for use in the methods of the present invention can be administered by any suitable means, including parenteral administration (*e*.*g*., injection, infusion), and may be administered by subcutaneous, intraperitoneal, intrapulmonary, and intranasal, and, if desired for local treatment, intralesional administration, *e*.*g*., intratumoral administration. Parenteral infusions include intravenous, intraarterial, intraperitoneal, intramuscular, intradermal or subcutaneous administration. In addition, Siglec 15 and modulators of Siglec 15 can be suitably administered by pulse infusion, particularly with declining doses. The dosing can be given by injections, such as intravenous or subcutaneous injections. The route of administration can be selected according to various factors, such as whether the administration is brief or chronic. Other administration methods are contemplated, including topical, particularly transdermal, transmucosal, rectal, oral or local administration e.g. through a catheter placed close to the desired site. Injection, especially intravenous, is of interest.

In some embodiments, the methods of the present invention comprise administering a therapeutically effective amount of Siglec 15 as described herein to the subject. In some embodiments, the methods of the present invention comprise administering a therapeutically effective amount of a modulator of a Siglec 15 to the subject. The therapeutically effective amount of Siglec 15 or a modulator of Siglec 15 is an amount sufficient to treat disease, *e*.*g*., an autoimmune disease, or a cancer, in a subject. The therapeutically effective dosage of Siglec 15 or a modulator of Siglec 15 as described herein will vary somewhat from subject to subject, and will depend upon factors such as the age, weight, and condition of the subject and the route of delivery. Such dosages can be determined in accordance with procedures known to those skilled in the art. In an exemplary embodiment, a therapeutically effective amount of a Siglec 15 antagonist is an amount effective to reduce the level or activity of Siglec 15 in a subject. In another exemplary embodiment, a therapeutically effective amount of a Siglec 15 agonist is an amount effective to increase the level or activity of Siglec 15 in a subject.

In one embodiment, the therapeutic methods described herein are performed on a human.

### IV. Siglec 15 Modulators For Use in the Methods of the Invention

As described above, a decrease in expression of Siglec 15 reduces tumor size and prolongs the survival of mice having brain tumors, whereas a decrease in the expression and/or activity of Siglec 15 exacerbated brain inflammation, suggesting that Siglec 15 plays a critical role as an immune modulatory molecule in the regulation of immune responses to cancer and inflammation. Accordingly, molecules which modulate, *e*.*g*., inhibit or activate, the expression and/or activity of Siglec 15, and/or molecules which modulate, *e*.*g*., inhibit or activate, the expression and/or activity of Siglec 15 binding partners, *e*.*g*., MAG or LRRC4C, are useful in the methods of the present invention. Exemplary modulators can modulate, *e*.*g*., inhibit or promote, the association between Siglec 15 and a Siglec 15 ligand (*e.g.*, MAG, LRRC4C, or Sialyl-Tn). An inhibitory modulator (*i*.*e*., inhibitor) or an activating modulator (*i*.*e*., an activator) can be a nucleic acid, a polypeptide, an antibody, or a small molecule compound. In some embodiments, the inhibitor or activator functions at a level of transcription, mRNA stability, translation, protein stability/degradation, protein modification, and protein binding.

### A. Inhibitory Modulators

In some embodiments, a modulator for use in the methods of the invention is an inhibitory modulator. In some embodiments, an inhibitory modulator is a small molecule, *e.g.*, a small molecule inhibitor for Siglec 15, a small molecule inhibitor for binding ligands of Siglec 15, *e.g.*, MAG, LRRC4C, and/or Sialyl-Tn. In other embodiments, an inhibitory modulator for use in the methods of the invention is an intracellular binding molecule that acts to specifically inhibit the expression, stability, and/or activity of Siglec 15 and/or its binding ligands, *e.g.*, MAG, LRRC4C, and/or Sialyl-Tn. In other embodiments, the inhibitory modulator is an antagonist antibody for Siglec 15, and/or its binding ligands, *e.g.*, MAG or LRRC4C, or antigen-binding fragment thereof. In some embodiments, the inhibitory modulator is a recombinant fusion protein for Siglec 15, and/or its binding ligands, *e.g.*, MAG or LRRC4C. In other embodiments, the Siglec 15 fusion protein is a Siglec 15-Fc fusion protein. In some embodiments, an inhibitory modulators for use in the methods of the invention is a nucleic acid molecule which acts to specifically decrease the expression, stability, and/or activity of Siglec 15 and/or its binding ligands, *e.g.*, MAG or LRRC4C.

As used herein, the term "intracellular binding molecule" is intended to include molecules that act intracellularly to inhibit the expression or activity of a protein by binding to the protein or to a nucleic acid (*e.g.*, an mRNA molecule) that encodes the protein.

The modulator of Siglec 15 of the present invention may also modulate, *e*.*g*., block the interaction between Siglec 15 and a binding ligand. In some embodiments, the binding ligand of Siglec 15 is selected from the group consisting of MAG and LRRC4C. In some embodiments, the modulator is a mutant Siglec 15 protein that has a single substitution mutation at residue 143. In other embodiments, the modulator is a mutant Siglec 15 protein that has a deletion of the IgV domain. In some embodiments, the modulator of Siglec 15 binds to the IgV domain of Siglec 15. In other embodiments, the modulator of Siglec 15 binds to residue 143 of Siglec 15. In some embodiments, the modulator of Siglec 15 binds to the region in the ligand of Siglec 15, *e.g.*, MAG or LRRC4C, where interaction between Siglec 15 and its ligand occurs.

### i. Inhibitory Nucleic Acids

In one embodiment, the methods described herein include targeting Siglec 15 and/or its binding ligands, *e.g.*, MAG or LRRC4C, using inhibitory nucleic acids. A nucleic acid inhibitor can encode a small interference RNA (*e.g.*, an RNAi agent) that targets Siglec 15, and/or its binding ligands, *e.g.*, MAG or LRRC4C, and inhibits its expression or activity. The term "RNAi agent" refers to an RNA, or analog thereof, having sufficient sequence complementarity to a target RNA to direct RNA interference. Examples also include a DNA that can be used to make the RNA.

*RNA Interference*: RNA interference (RNAi) refers to a sequence-specific or selective process by which a target molecule (*e.g.*, a target gene, protein or RNA) is down-regulated. Generally, an interfering RNA ("RNAi") is a double stranded short-interfering RNA (siRNA), short hairpin RNA (shRNA), or single-stranded micro-RNA (miRNA) that results in catalytic degradation of specific mRNAs, and also can be used to lower or inhibit gene expression. RNA interference (RNAi) is a process whereby double-stranded RNA (dsRNA) induces the sequence-specific regulation of gene expression in animal and plant cells and in bacteria (Aravin and Tuschl, FEBS Lett. 26:5830-5840, 2005; Herbert et al., Curr. Opin. Biotech. 19:500-505, 2008; Sharp, Genes Dev., 15:485-490, 2001; Valencia-Sanchez et al. Genes Dev. 20:515-524, 2006). In mammalian cells, RNAi can be triggered by 21-nucleotide (nt) duplexes of small interfering RNA (siRNA) (Chiu et al., Mol. Cell. 10:549-561, 2002), by microRNA (miRNA), functional small-hairpin RNA (shRNA), or other dsRNAs which are expressed in vivo using DNA templates with RNA polymerase II or III promoters (Zeng et al., Mol. Cell 9:1327-1333, 2002; Paddison et al., Genes Dev. 16:948-958, 2002; Denti, et al., Mol. Ther. 10:191-199, 2004; Lee et al., Nature Biotechnol. 20:500-505, 2002; Paul et al., Nature Biotechnol. 20:505-508, 2002; Rossi, Human Gene Ther. 19:313-317, 2008; Tuschl, T., Nature Biotechnol. 20:440-448, 2002; Yu et al., Proc. Natl. Acad. Sci. USA 99(9):6047-6052, 2002)

*siRNA Molecules*: The term "short interfering RNA" or "siRNA" (also known as "small interfering RNAs") refers to an RNA agent, preferably a double-stranded agent, of about 10-50 nucleotides in length, preferably between about 15-25 nucleotides in length, more preferably about 17, 18, 19, 20, 21, 22, 23, 24, or 25 nucleotides in length, the strands optionally having overhanging ends comprising, for example 1, 2 or 3 overhanging nucleotides (or nucleotide analogs), which is capable of directing or mediating RNA interference. Naturally-occurring siRNAs are generated from longer dsRNA molecules (e.g., >25 nucleotides in length) by a cell's RNAi machinery.

In general, the methods described herein can use dsRNA molecules comprising 16-30, *e.g.*, 16, 17, 18, 19, 20, 21, 22, 23, 24, 25, 26, 27, 28, 29, or 30 nucleotides in each strand, wherein one of the strands is substantially identical, *e.g.*, at least 80% (or more, *e.g.*, 85%, 90%, 95%, or 100%) identical, *e.g.*, having 3, 2, 1, or 0 mismatched nucleotide(s), to a target region in the mRNA, and the other strand is complementary to the first strand. The dsRNA molecules can be chemically synthesized, or can be transcribed in vitro or in vivo, *e*.*g*., shRNA, from a DNA template. The dsRNA molecules can be designed using any method known in the art. Negative control siRNAs should not have significant sequence complementarity to the appropriate genome. Such negative controls can be designed by randomly scrambling the nucleotide sequence of the selected siRNA; a homology search can be performed to ensure that the negative control lacks homology to any other gene in the appropriate genome. In addition, negative control siRNAs can be designed by introducing one or more base mismatches into the sequence.

The methods described herein can use both siRNA and modified siRNA derivatives, *e*.*g*., siRNAs modified to alter a property such as the specificity and/or pharmacokinetics of the composition, for example, to increase half-life in the body, *e.g.*, crosslinked siRNAs. Thus, the invention includes methods of administering siRNA derivatives that include siRNA having two complementary strands of nucleic acid, such that the two strands are crosslinked. The oligonucleotide modifications include, but are not limited to, 2'-O-methyl, 2'-fluoro, 2'-O-methyoxyethyl and phosphorothioate, boranophosphate, 4'-thioribose. (Wilson and Keefe, Curr. Opin. Chem. Biol. 10:607-614, 2006; Prakash et al., J. Med. Chem. 48:4247-4253, 2005; Soutschek et al., Nature 432:173-178, 2004).

In some embodiments, the siRNA derivative has at its 3' terminus a biotin molecule (*e.g.*, a photocleavable biotin), a peptide (*e.g.*, a Tat peptide), a nanoparticle, a peptidomimetic, organic compounds (*e*.*g*., a dye such as a fluorescent dye), or dendrimer. Modifying siRNA derivatives in this way may improve cellular uptake or enhance cellular targeting activities of the resulting siRNA derivative as compared to the corresponding siRNA, are useful for tracing the siRNA derivative in the cell, or improve the stability of the siRNA derivative compared to the corresponding siRNA.

The inhibitory nucleic acid compositions can be unconjugated or can be conjugated to another moiety, such as a nanoparticle, to enhance a property of the compositions, *e*.*g*., a pharmacokinetic parameter such as absorption, efficacy, bioavailability, and/or half-life. The conjugation can be accomplished by methods known in the art, *e.g.*, using the methods of Lambert et al., Drug Deliv. Rev.:47(1), 99-112, 2001; Fattal et al., J. Control Release 53(1-3):137-43, 1998; Schwab et al., Ann. Oncol. 5 Suppl. 4:55-8, 1994; and Godard et al., Eur. J. Biochem. 232(2):404-10, 1995). The inhibitory nucleic acid molecules can also be labeled using any method known in the art; for instance, the nucleic acid compositions can be labeled with a fluorophore, *e.g.*, Cy3, fluorescein, or rhodamine. The labeling can be carried out using a kit, *e.g.*, the SILENCER^{™} siRNA labeling kit (Ambion). Additionally, the siRNA can be radiolabeled, *e*.*g*., using ³H, ³²P, or other appropriate isotope.

*siRNA Delivery*: Direct delivery of siRNA in saline or other excipients can silence target genes in tissues, such as the eye, lung, and central nervous system (Bitko et al., Nat. Med. 11:50-55 (2005); Shen et al., Gene Ther. 13:225-234 (2006); Thakker et al., Proc. Natl. Acad. Sci. U.S.A. (2004)). In adult mice, efficient delivery of siRNA can be accomplished by "high-pressure" delivery technique, a rapid injection (within 5 seconds) of a large volume of siRNA containing solution into animal via the tail vein (Lewis, Nature Genetics 32:107-108, 2002).

Liposomes and nanoparticles can also be used to deliver siRNA into animals. Delivery methods using liposomes, *e*.*g*. stable nucleic acid-lipid particles (SNALPs), dioleoyl phosphatidylcholine (DOPC)-based delivery system, as well as lipoplexes, *e*.*g*. Lipofectamine 2000, TransIT-TKO, have been shown to effectively repress target mRNA (de Fougerolles, Human Gene Ther. 19:125-132 (2008); Landen et al., Cancer Res. 65:6910-6918 (2005); Luo et al., Mol. Pain 1:29 (2005); Zimmermann et al., Nature 441:111-114 (2006)). Conjugating siRNA to peptides, RNA aptamers, antibodies, or polymers, *e*.*g*. dynamic polyconjugates, cyclodextrin-based nanoparticles, atelocollagen, and chitosan, can improve siRNA stability and/or uptake. (Howard et al., Mol. Ther. 14:476-484 (2006); Hu-Lieskovan et al., Cancer Res. 65:8984-8992 (2005); Kumar, et al., Nature 448:39-43; McNamara et al., Nat. Biotechnol. 24:1005-1015 (2007); Rozema et al., Proc. Natl. Acad. Sci. U.S.A. 104:12982-12987 (2007); Song et al., Nat. Biotechnol. 23:709-717 (2005); Wolfrum et al., Nat. Biotechnol. 25:1149-1157 (2007)).

Viral-mediated delivery mechanisms can also be used to induce specific silencing of targeted genes through expression of siRNA, for example, by generating recombinant adenoviruses harboring siRNA under RNA Pol II promoter transcription control. Infection of HeLa cells by these recombinant adenoviruses allows for diminished endogenous target gene expression. Injection of the recombinant adenovirus vectors into transgenic mice expressing the target genes of the siRNA results in in vivo reduction of target gene expression. *Id.* In an animal model, whole-embryo electroporation can efficiently deliver synthetic siRNA into post-implantation mouse embryos (Calegari et al., Proc. Natl. Acad. Sci. USA 99(22):14236-40 (2002)).

*Uses of Engineered RNA Precursors to Induce RNAi*: Engineered RNA precursors, introduced into cells or whole organisms as described herein, will lead to the production of a desired siRNA molecule. Such an siRNA molecule will then associate with endogenous protein components of the RNAi pathway to bind to and target a specific mRNA sequence for cleavage, destabilization, and/or translation inhibition destruction. In this fashion, the mRNA to be targeted by the siRNA generated from the engineered RNA precursor will be depleted from the cell or organism, leading to a decrease in the concentration of the protein encoded by that mRNA in the cell or organism.

*Antisense*: An "antisense" nucleic acid can include a nucleotide sequence that is complementary to a "sense" nucleic acid encoding a protein, *e*.*g*., complementary to the coding strand of a double-stranded cDNA molecule or complementary to a target mRNA sequence. The antisense nucleic acid can be complementary to an entire coding strand of a target sequence, or to only a portion thereof (for example, the coding region of a target gene). In another embodiment, the antisense nucleic acid molecule is antisense to a "noncoding region" of the coding strand of a nucleotide sequence encoding the selected target gene (*e.g.*, the 5' and 3' untranslated regions).

An antisense nucleic acid can be designed such that it is complementary to the entire coding region of a target mRNA but can also be an oligonucleotide that is antisense to only a portion of the coding or noncoding region of the target mRNA. For example, the antisense oligonucleotide can be complementary to the region surrounding the translation start site of the target mRNA, *e*.*g*., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (*e*.*g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e*.*g*., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

Based upon the sequences disclosed herein relating to Siglec 15 and/or its binding ligands, *e.g.*, MAG and LRRC4C, one of skill in the art can easily choose and synthesize any of a number of appropriate antisense molecules for use in accordance with the present invention. For example, a "gene walk" comprising a series of oligonucleotides of 15-30 nucleotides spanning the length of a target nucleic acid can be prepared, followed by testing for inhibition of target gene expression. Optionally, gaps of 5-10 nucleotides can be left between the oligonucleotides to reduce the number of oligonucleotides synthesized and tested. In some embodiments, the antisense molecules target the IgV domain of Siglec 15. In other embodiments, the antisense molecules target the region in MAG or LRRC4C where the interaction with Siglec 15 occurs. In some embodiments, the antisense molecules target the region spanning residue 143 of Siglec 15.

The antisense nucleic acid molecules of the invention are typically administered to a subject (*e*.*g*., by direct injection at a tissue site), or generated in situ such that they hybridize with or bind to cellular mRNA and/or genomic DNA encoding a target protein to thereby inhibit expression of the protein, *e.g.*, by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, *e*.*g*., by linking the antisense nucleic acid molecules to peptides or antibodies that bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter can be used.

*CRISPR Technology:* The expression of a target polynucleotide (*e*.*g*., DNA or RNA of Siglec 15 and/or its binding ligands, *e.g.*, MAG and LRRC4C) can be modified by allowing a CRISPR complex to bind to the polynucleotide, wherein the CRISPR complex comprises a CRISPR enzyme complexed with a guide sequence hybridized to a target sequence within the target polynucleotide, wherein the guide sequence is linked to a tracr mate sequence which in turn hybridizes to a tracr sequence. In some embodiment, binding of CRISPR complex to a target polynucleotide results in an increased expression of the target polynucleotide. In another embodiment, binding of CRISPR complex to a target polynucleotide results in a decreased expression of the target polynucleotide (*e*.*g*., DNA or RNA of Siglec 15 and/or its binding ligands, *e.g.*, MAG and LRRC4C).

The clustered, regularly interspaced, short palindromic repeat (CRISPR) technology is included in the invention as an approach for generating RNA-guided nuclease with customizable specificities for targeted genome editing. Genome editing mediated by these nucleases has been used to rapidly, easily and efficiently modify endogenous genes in a wide variety of biomedically important cell types and in organisms that have traditionally been challenging to manipulate genetically.

In general, the term "CRISPR system" refers collectively to transcripts and other/elements involved in the expression of or directing the activity of CRISPR-associated ("Cas") genes, including sequences encoding a Cas gene, a tracr (transactivating CRISPR) sequence (*e*.*g*. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In embodiments of the invention the terms guide sequence and guide RNA are used interchangeably. In some embodiments, one or more elements of a CRISPR system is derived from a type I, type II, or type III CRISPR system. In some embodiments, one or more elements of a CRISPR system is derived from a particular organism comprising an endogenous CRISPR system, such as Streptococcus pyogenes. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides (*e*.*g*., DNA or RNA of Siglec 15 and/or its binding ligands, *e.g.*, MAG and LRRC4C). In some embodiments, a target sequence is located in the nucleus or cytoplasm of a cell.

In preferred embodiments of the invention, the CRISPR/Cas system is a type II CRISPR system and the Cas enzyme is Cas9, which catalyzes DNA cleavage. Enzymatic action by Cas9 derived from Streptococcus pyogenes or any closely related Cas9 generates double stranded breaks at target site sequences which hybridize to 20 nucleotides of the guide sequence and that have a protospacer-adjacent motif (PAM) sequence NGG following the 20 nucleotides of the target sequence. CRISPR activity through Cas9 for site-specific DNA recognition and cleavage is defined by the guide sequence, the tracr sequence that hybridizes in part to the guide sequence and the PAM sequence. More aspects of the CRISPR system are described in Karginov and Hannon, The CRISPR system: small RNA-guided defense in bacteria and archae, Mol. Cell 2010, 37(1): 7.

The type II CRISPR locus from Streptococcus pyogenes SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30 bp each). In this system, targeted DNA double-strand break (DSB) is generated in four sequential steps. First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer. Several aspects of the CRISPR system can be further improved to increase the efficiency and versatility of CRISPR targeting. Optimal Cas9 activity may depend on the availability of free Mg2+ at levels higher than that present in the mammalian nucleus (see *e.g.* Jinek et al., 2012, Science, 337:816), and the preference for an NGG motif immediately downstream of the protospacer restricts the ability to target on average every 12-bp in the human genome.

The foregoing inhibitory nucleic acid strategies can also be used to diminish levels of Sialyl-Tn. For example, RNA interference, siRNA molecules, antisense nucleic acids, and/or CRISPR technology can be used to modulate the expression level of enzymes responsible for the formation of Sialyl-Tn. As noted herein, Sialyl-Tn is a truncated O-glycan containing a sialic acid α-2,6 linked to GalNAc α-*O*-Ser/Thr. Exemplary enzymes involved in synthesis of Sialyl-Tn include sialytransferase ST6GalNAc1 and COSMC (core 1 β3-Gal-T-specific molecular chaperone). Modulation of enzymes involved in Sialyl-Tn production, such as sialytransferase ST6GalNAc1 or COSMC, *e.g.,* using inhibitory nucleic acids, can be used to reduce levels of Sialyl-Tn *in vivo* or *in vitro.*

### ii. Antagonist Antibodies

The invention further contemplates methods and compositions comprising an antagonist antibody which inhibits Siglec 15 and/or its binding ligands, *e.g.*, MAG, LRRC4C, and/or Sialyl-Tn, thereby decreasing expression and/or activity of Siglec 15 and promoting an immune response against a cancer.

In some embodiments, the antagonist antibody of Siglec 15 and/or its binding ligands, *e.g.*, MAG, LRRC4C, and/or Sialyl-Tn, or antigen binding portion thereof, decreases the expression and/or activity of Siglec 15 and/or its binding ligands, *e.g.*, MAG, LRRC4C, and/or Sialyl-Tn.

In some embodiments, an antagonist antibody of Siglec 15 and/or an antagonist antibody of Siglec 15 ligands, *e.g.*, MAG, LRRC4C, and/or Sialyl-Tn, or an antigen binding portion thereof, block the interaction between Siglec 15 and its binding ligand, *e.g.*, MAG, LRRC4C, and/or Sialyl-Tn. In some embodiments, the antagonist antibody of Siglec 15 and/or its binding ligands, *e.g.*, MAG, LRRC4C, and/or Sialyl-Tn, or antigen binding portion thereof, binds to the IgV domain of Siglec 15. In other embodiments, the antagonist antibody of Siglec 15 and/or its binding ligands, *e.g.*, MAG, LRRC4C, and/or Sialyl-Tn, or antigen binding portion thereof, binds to residue 143 of Siglec 15. In some embodiments, the antagonist antibody of Siglec 15 and/or its binding ligands, *e.g.*, MAG, LRRC4C, and/or Sialyl-Tn, or antigen binding portion thereof, binds to the region in the ligand of Siglec 15, *e.g.*, MAG, LRRC4C, and/or Sialyl-Tn, where interaction between Siglec 15 and its ligands occurs.

In one embodiment, an antagonist antibody, or antigen-binding portion thereof, binds an extracellular epitope of Siglec 15. For example, the antagonist antibody, or antigen-binding portion thereof, can bind Siglec 15 at an extracellular epitope responsible for the interaction between Siglec 15 and a Siglec 15 ligand, *e.g.*, MAG, LRRC4C, and/or Sialyl-Tn. Such antibodies may partially or completely block the interaction between Siglec 15 and a Siglec 15 ligand, *e.g.*, MAG, LRRC4C, and/or Sialyl-Tn. In an exemplary embodiment, an anti-Siglec 15 antagonist antibody, or antigen-binding portion thereof, binds Siglec 15 at an epitope that includes residue 143. In one embodiment, an antagonist anti-Siglec 15 antibody does not transduce a signal through Siglec 15. In one embodiment, an antagonist anti-Siglec 15 antibody induces endocytosis of Siglec 15. In another embodiment, an antagonist anti-Siglec 15 antibody does not induce endocytosis of Siglec 15.

Anti-Siglec 15 antibodies can be screened for their ability to antagonize the function of Siglec 15 using standard methods. Exemplary anti-Siglec 15 antagonist antibodies include S15m03 and S15m02, described herein. Antibodies, or antigen-binding fragments thereof, derived from S15m03 or S15m02 can also be useful as Siglec 15 antagonists. For example, a derivative of S15m03 or S15m02 can be a chimeric, humanized, or human variant of S15m03 or S15m02. An exemplary derivative of S 15m03 or S 15m02 comprises one, two, three, four, five, or six of the complementary determining regions (CDRs) of S15m03 or S15m02. For example, a derivative of S15m03 or S15m02 can contain the heavy chain and/or light chain CDR3 of S15m03 or

S15m02. In another example, a derivative of S15m03 or S15m02 can contain the heavy chain and/or light chain CDR2 of S15m03 or S15m02. In another example, a derivative of S15m03 or S15m02 can contain the heavy chain and/or light chain CDR1 of S15m03 or S15m02. In another example, a derivative of S15m03 or S15m02 can contain the heavy chain and/or light chain CDR1, CDR2, and CDR3 of S15m03 or S15m02. In preferred embodiments, derivatives of S15m03 or S15m02 maintain the antigen-binding specificity ofS15m03 or S15m02 for Siglec 15.

In one embodiment, the anti-Siglec 15 antibody is an antibody set forth in U.S. Provisional Patent Application No. 62/397,794, filed September 21, 2016, the entire contents of which are incorporated herein by reference.

Other exemplary anti-Siglec 15 antibodies that may be useful as Siglec 15 antagonists include, but are not limited to, mAb A9E8 (described in US 9,447,192), mAb DS-1501 (Daiichi Sankyo), mAb 32A1 (described in US 8,575,316), mAb AB-25E9 (Alethia Biotherapeutics), mAb 25B8, mAb 25E6, and mAb 25E9 (described in US 9,388,242). The entire contents of US 9,447,192, US 8,575,316, and US 9,388,242 are incorporated herein by reference. Other exemplary anti-Siglec 15 antibodies include antibodies that compete for binding with the foregoing anti-Siglec 15 antibodies, and/or which bind the same or an overlapping epitope in Siglec 15 as the foregoing anti-Siglec 15 antibodies. In one embodiment, the Siglec 15 antagonist is not mAb A9E8, or an antigen-binding fragment thereof. In another embodiment, the Siglec 15 antagonist is not mAb DS-1501, or an antigen-binding fragment thereof. In another embodiment, the Siglec 15 antagonist is not mAb AB-25E9, or an antigen-binding fragment thereof.

In one embodiment, an antagonist antibody, or antigen-binding portion thereof, binds an extracellular epitope of MAG. For example, the antagonist antibody, or antigen-binding portion thereof, can bind MAG at an extracellular epitope responsible for the interaction between MAG and Siglec 15. Such antibodies may partially or completely block the interaction between MAG and Siglec 15. In an exemplary embodiment, an anti-MAG antagonist antibody, or antigen-binding portion thereof, binds MAG at an epitope that interacts with residue 143 of Siglec 15. In one embodiment, an antagonist anti-MAG antibody does not transduce a signal through MAG.

In one embodiment, an antagonist antibody, or antigen-binding portion thereof, binds an extracellular epitope of LRRC4C. For example, the antagonist antibody, or antigen-binding portion thereof, can bind LRRC4C at an extracellular epitope responsible for the interaction between LRRC4C and Siglec 15. Such antibodies may partially or completely block the interaction between LRRC4C and Siglec 15. In an exemplary embodiment, an anti-LRRC4C antagonist antibody, or antigen-binding portion thereof, binds LRRC4C at an epitope that interacts with residue 143 of Siglec 15. In one embodiment, an antagonist anti-LRRC4C antibody does not transduce a signal through LRRC4C.

In one embodiment, an antagonist antibody, or antigen-binding portion thereof, binds Sialyl-Tn. Such antibodies may partially or completely block the interaction between Sialyl-Tn and Siglec 15. In an exemplary embodiment, an anti-Sialyl-Tn antagonist antibody, or antigen-binding portion thereof, binds Sialyl-Tn at an epitope that interacts with Siglec 15. In one embodiment, an antagonist anti-Sialyl-Tn antibody does not transduce a signal through a polypeptide containing Sialyl-Tn.

Antagonist antibodies suitable for use in the methods of the present invention may be identified, screened for (*e.g.*, using phage display), or characterized for their physical/chemical properties and/or biological activities by various assays known in the art (see, for example, Antibodies: A Laboratory Manual, Second edition, Greenfield, ed., 2014). Binding specificity of an antibody for its antigen can be tested by known methods in the art such as ELISA, Western blot, or surface plasmon resonance.

Antibodies may be produced using recombinant methods and compositions known in the art, *e.g.*, as described in U.S. Pat. No. 4,816,567, incorporated by reference herein. An isolated nucleic acid encoding, for example, an anti-Siglec 15 antibody is used to transform host cells for expression. Such nucleic acid may encode an amino acid sequence comprising the VL and/or an amino acid sequence comprising the VH of the antibody (*e*.*g*., the light and/or heavy chains of the antibody). In a further embodiment, one or more vectors (*e*.*g*., expression vectors) comprising such nucleic acid are provided. In a further embodiment, a host cell comprising such nucleic acid is provided. In one such embodiment, a host cell comprises (*e*.*g*., has been transformed with): a vector comprising a nucleic acid that encodes an amino acid sequence comprising the VL of the antibody and an amino acid sequence comprising the VH of the antibody, or a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, *e.g.* a Chinese Hamster Ovary (CHO) cell or lymphoid cell (*e.g.*, Y0, NS0, Sp20 cell).

For recombinant production of an anti-Siglec 15 antibody, a nucleic acid encoding an antibody is isolated and inserted into one or more vectors for further cloning and/or expression in a host cell. Such nucleic acid may be readily isolated and sequenced using conventional procedures (*e*.*g*., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, *e*.*g*., U.S. Pat. Nos. 5,648,237, 5,789,199, and 5,840,523. After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.*, in Graham et al., J. Gen Virol. 36:59 (1977)); baby hamster kidney cells (BHK); mouse sertoli cells (TM4 cells as described, *e*.*g*., in Mather, Biol. Reprod. 23:243-251 (1980)); monkey kidney cells (CV1); African green monkey kidney cells (VERO-76); human cervical carcinoma cells (HELA); canine kidney cells (NMCK; buffalo rat liver cells (BRL 3A); human lung cells (W138); human liver cells (Hep G2); mouse mammary tumor (MMT 060562); TR1 cells, as described, *e*.*g*., in Mather et al., Annals N.Y. Acad. Sci. 383:44-68 (1982); MRC 5 cells; and FS4 cells. Other useful mammalian host cell lines include Chinese hamster ovary (CHO) cells, including DHFR.sup.- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, *e*.*g*., Yazaki and Wu, Methods in Molecular Biology, Vol. 248 (B. K. C. Lo, ed., Humana Press, Totowa, N.J.), pp. 255-268 (2003).

### iii. Small Molecule Inhibitors

Other inhibitory modulators that can be used to specifically inhibit the activity of an Siglec 15 protein are chemical compounds that directly inhibit Siglec 15 activity or inhibit the interaction between Siglec 15 and its binding ligands, *e.g.*, MAG, LRRC4C, and/or Sialyl-Tn. Such compounds can be either natural products or members of a combinatorial chemistry library, and can be identified using screening assays, as described in detail below.

In some embodiments, the small molecule inhibitors of the present invention may block the interaction between Siglec 15 and its binding ligands. For example the small molecule inhibitors bind to the IgV domain of Siglec 15. In other embodiments, the small molecule inhibitors of the present invention may bind to the region in MAG, LRRC4C, or Sialyl-Tn that interacts with the IgV domain of Siglec 15, thereby blocking the interaction between Siglec 15 and its ligand, *e*.*g*., MAG, LRRC4C, or Sialyl-Tn.

In some embodiments, the small molecule inhibitors are selected to bind domains sharing homology to a domain of the Siglec 15 For example, a small molecule of the present invention may be directed toward a domain which is at least 50% identical, at least 60% identical, at least 70% identical, at least 80% identical, at least 90% identical, or at least 95% or 99% identical to the IgV domain of Siglec 15. Such a small molecule would be capable of binding protein domains, possibly in Siglec 15, which are functionally similar to, for example, the IgV domain of Siglec 15.

The small molecule inhibitors of the present invention may also bind to a particular motif or consensus sequence derived from an IgV domain of Siglec 15, allowing the small molecule inhibitors to specifically bind domains which are shared among members of the Siglec family. In another embodiment, small molecules of the present invention bind protein motifs or consensus sequences which represent the three dimensional structure of the protein. Such motifs or consensus sequences would not represent a contiguous string of amino acids, but a non-linear amino acid arrangement that results from the three-dimensional folding of Siglec 15 (*i.e.*, a structural motif). An example of such a motif would be a motif designed based on the IgV domain of Siglec 15. Such motifs and consensus sequences may be designed according to the any methods known in the art.

In some embodiments the small molecule binds to specific sequences of the Siglec 15 protein, for example, residue 143 of Siglec 15.

### iv. Fusion Proteins or Mutant Proteins

Variants of Siglec 15 protein or variants of Siglec 15's binding ligands (e.g., MAG and LRRC4C) that function as antagonists and inhibit the activity of Siglec 15 can be used in the methods of the present invention. For example, a Fc-fusion protein of Siglec 15 which functions as a blocking protein in a similar manner as an anti-Siglec 15 antagonist antibody can be used as an inhibitory modulator in the present invention. In an exemplary embodiment, the Siglec 15-Fc fusion protein antagonist is a soluble Siglec 15-Fc fusion protein. Alternatively, Siglec 15 mutants that have a reduced binding activity for its ligands would also function as an inhibitory modulator for use in the methods of the invention. For example, a Siglec 15 mutant that has a signle substitution mutation at residue 143, or a Siglec 15 mutant without the IgV domain can be used as an inhibitory modulator in the present invention.

In some embodiments, variants of Siglec 15's binding ligands (e.g., MAG and LRRC4C) blocks the interaction between Siglec 15 and its binding ligand. In some embodiments, a Fc-fusion protein of MAG or LRRC4C which functions as a blocking protein in a similar manner as an anti-Siglec 15 antagonist antibody can be used as an inhibitory modulator in the present invention. In another example, a protein containing Sialyl-Tn can function as a blocking agent, by binding and sequestering Siglec 15. Alternatively, MAG or LRRC4C mutants that have a reduced binding activity for Siglec 15 would also function as an inhibitory modulator for use in the methods of the invention. For example, a MAG or LRRC4C mutant that has a mutation that abolish the interaction with the IgV domain of Siglec 15 can be used as an inhibitory modulator in the present invention.

A recombinant fusion protein for Siglec 15 and/or its binding ligands, *e.g,* MAG or LRRC4C, for use in the methods of the present invention may be generated from a recombinant vector according to methods know in the art. The recombinant vectors can comprise a nucleic acid encoding a Siglec 15 protein in a form suitable for expression of the nucleic acid in a host cell. In some embodiments, a nucleic acid sequence encoding the Fc domain of IgG can be operably linked in an expression vector to a nucleic acid molecule encoding a protein of interest, such as Siglec 15 and/or its binding ligands, *e.g*., MAG and LRRC4C, or a functional segment thereof. The resulting fusion protein can then be readily purified from the cells by art recognized methods, such as with an anti-Fc antibody.

In some embodiments, the recombinant vectors may include one or more regulatory sequences, selected on the basis of the host cells to be used for expression, which is operably linked to the nucleic acid sequence to be expressed (i.e., a recombinant expression vector). Within a recombinant expression vector, "operably linked" is intended to mean that the nucleotide sequence of interest is linked to the regulatory sequence(s) in a manner which allows for expression of the nucleotide sequence (*e.g*., in an *in vitro* transcription/translation system or in a host cell when the vector is introduced into the host cell). The term "regulatory sequence" is intended to include promoters, enhancers and other expression control elements (*e.g*., polyadenylation signals). Such regulatory sequences are described, for example, in Goeddel, Methods in Enzymology: Gene Expression Technology vol.185, Academic Press, San Diego, CA (1991). Regulatory sequences include those which direct constitutive expression of a nucleotide sequence in many types of host cell and those which direct expression of the nucleotide sequence only in certain host cells (*e.g*., tissue-specific regulatory sequences). It will be appreciated by those skilled in the art that the design of the expression vector can depend on such factors as the choice of the host cell to be transformed, the level of expression of protein desired, and the like. The expression vectors of the invention can be introduced into host cells to thereby produce proteins or peptides, including fusion proteins or peptides, encoded by nucleic acids as described herein.

The recombinant expression vectors of the invention can be designed for expression of a polypeptide, or functional fragment thereof, in prokaryotic (*e.g., E. coli*) or eukaryotic cells (*e.g*., insect cells using baculovirus expression vectors, yeast cells or mammalian cells). Suitable host cells may include, but not limited to *E. coli* cells, *Bacillus* cells, *Saccharomyces* cells, *Pochia* cells, NS0 cells, COS cells, Chinese hamster ovary (CHO) cells, myeloma cells, or cells as described herein.

Another aspect of the invention pertains to host cells into which a recombinant vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

Vector DNA can be introduced into prokaryotic or eukaryotic cells *via* conventional transformation or transfection techniques. As used herein, the terms "transformation" and "transfection" are intended to refer to a variety of art-recognized techniques for introducing foreign nucleic acid into a host cell, including calcium phosphate or calcium chloride co-precipitation, DEAE-dextran-mediated transfection, lipofection, or electroporation.

### v. Siglec 15-Derived Peptides

An inhibitory modulator for use in the methods of the present invention is a peptidic compound derived from the amino acid sequence of Siglec 15 and/or its binding ligands *(e.g.,* the sequences disclosed herein as SEQ ID NOs: 2, 4 and 6). In particular, the inhibitory compound comprises a portion of Siglec 15 (or a mimetic thereof) that mediates interaction of Siglec 15 with a binding ligand, *e.g.,* MAG, LRRC4C, or Sialyl-Tn, such that contact of Siglec 15 with this peptidic compound competitively inhibits the interaction of Siglec 15 with its binding ligand, *e.g.*, MAG, LRRC4C, or Sialyl-Tn. For example, a peptide derived from Siglec 15 that comprises amino acid sequence in the IgV domain may serve as an inhibitory modulator. Alternatively, a peptide derived from Siglec 15 that comprises amino acid sequence surrounding residue 143 of Siglec 15 may serve as an inhibitory modulator. In other embodiments, a peptide derived from MAG or LRRC4C that comprises amino acid sequence in the region where interaction with Siglec 15 occurs may serve as an inhibitory modulator.

The peptidic compounds of the invention can be made intracellularly in cells by introducing into the cells an expression vector encoding the peptide. Such expression vectors can be made by standard techniques. The peptide can be expressed in intracellularly as a fusion with another protein or peptide *(e.g.,* a GST fusion). Alternative to recombinant synthesis of the peptides in the cells, the peptides can be made by chemical synthesis using standard peptide synthesis techniques. Synthesized peptides can then be introduced into cells by a variety of means known in the art for introducing peptides into cells (*e.g*., liposome and the like).

### B. Stimulatory Modulators

In some embodiments, a modulator for use in the methods of the invention is a stimulatory modulator, which increases the expression and/or activity of Siglec 15 and/or its binding ligands, *e.g.,* MAG, LRRC4C, or Sialyl-Tn, and thereby decreasing inflammation in a subject in need thereof. Examples of such stimulatory modulators include proteins, nucleic acid molecules, *e.g*., expression vectors comprising nucleic acid molecules, and small molecules that stimulate expression and/or activity of Siglec 15 and/or its binding ligands, *e.g.,* MAG, LRRC4C, or Sialyl-Tn, in a cell.

As used herein, the term "nucleic acid molecule" is intended to include DNA molecules (*e.g.,* cDNA or genomic DNA) and RNA molecules (*e.g.,* mRNA) and analogs of the DNA or RNA generated using nucleotide analogs. The nucleic acid molecule can be single-stranded or double-stranded, but preferably is double-stranded DNA. A nucleic acid molecule used in the methods of the present invention can be isolated using standard molecular biology techniques. In some embodiments, a stimulatory modulator suitable for use in the methods of the present invention is a Siglec 15 protein. For example, a stimulatory modulator is a full-length Siglec 15 protein, a functional fragment of Siglec 15, or an IgV domain of Siglec 15. A stimulatory modulators promotes the interaction between Siglec 15 and its binding ligands, *e.g.,* MAG, LRRC4C, or Sialyl-Tn. In other embodiments, the stimulatory modulator suitable for use in the methods of the present invention is a nucleic acid molecule encoding a Siglec 15 protein. For example, a cDNA (full length or partial cDNA sequence) is cloned into a recombinant expression vector and the vector is transfected into cells using standard molecular biology techniques. The cDNA can be obtained, for example, by amplification using the polymerase chain reaction (PCR) or by screening an appropriate cDNA library.

In some embodiments, a stimulatory modulator suitable for use in the methods of the present invention is a binding ligand of a Siglec 15 protein, *e.g.,* MAG, LRRC4C, or Sialyl-Tn. A stimulatory modulator promotes the interaction between Siglec 15 and its binding ligands. In other embodiments, the stimulatory modulator suitable for use in the methods of the present invention is a nucleic acid molecule encoding a binding ligand of a Siglec 15 protein, *e.g.,* MAG and LRRC4C.

In some embodiments, the stimulatory modulator for use in the methods of the invention is an intracellular binding molecule that acts to specifically activates the expression, stability, and/or activity of Siglec 15 and/or its binding ligands, *e.g.,* MAG, LRRC4C, or Sialyl-Tn. As used herein, the term "intracellular binding molecule" is intended to include molecules that act intracellularly to increase the expression or activity of a protein by binding to the protein or to a nucleic acid *(e.g.,* an mRNA molecule) that encodes the protein.

In other embodiments, the stimulatory modulator is an agonist antibody for Siglec 15, and/or its binding ligands, *e.g.,* MAG or LRRC4C, or antigen-binding fragment thereof. Such agonist antibodies for Siglec 15, and/or its binding ligands, *e.g.,* MAG or LRRC4C, can be identified and generated using methods as described herein.

In one embodiment, the stimulatory modulator is a Siglec 15 fusion protein. In exemplary embodiments, the stimulatory Siglec 15 fusion protein is immobilized, *e.g.*, on a cell, or on a solid support, *e.g.,* a bead. For example, a membrane-anchored Siglec 15-Fc fusion protein can increase the activity of Siglec 15 or Siglec 15 binding ligands, and can function as a Siglec 15 agonist. In another embodiment, the agonist is a Siglec-Fc fusion protein containing mutations in FcR binding.

In some embodiment, a stimulatory nucleic acid can be used to activate the expression and/or activity of Siglec 15 and/or its binding ligands, *e.g.,* MAG and LRRC4C, based on CRISPR technology as described herein.

Other stimulatory modulators that can be used to specifically activate the activity of a Siglec 15 protein and/or its binding ligands, *e.g.,* MAG, LRRC4C, or Sialyl-Tn, are chemical compounds that directly activates Siglec 15 activity or promotes the interaction between Siglec 15 and/or its binding ligands, *e.g.,* MAG, LRRC4C, or Sialyl-Tn. Such compounds can be identified using screening assays that select for such compounds, as described in detail below.

### V. Screening Assays

Modulators that affect Siglec 15 activity can be known (*e.g*., antibodies that interfere with Siglec 15 activity, or Siglec 15 mutant proteins) or can be identified using the methods described herein. The invention provides methods (also referred to herein as "screening assays") for identifying other modulators, *i.e*., candidate or test compounds or modulators (*e.g*., peptides, small molecules or other drugs) which modulate the expression and/or activity of Siglec 15 and for testing or optimizing the activity of other modulators.

In some embodiments, molecules which bind Siglec 15 and/or its binding ligands, *e.g.,* MAG, LRRC4C, or Sialyl-Tn, or have a stimulatory or inhibitory effect on the expression and/or activity of Siglec 15 or its binding ligands, *e.g.,* MAG, LRRC4C, or Sialyl-Tn, can be identified.

In one embodiment, the ability of a compound to directly modulate the expression, post-translational modification, or activity of Siglec 15 or its binding ligands, *e.g.,* MAG, LRRC4C, or Sialyl-Tn, is measured as an indicator using a screening assay of the invention.

Modulators that are capable of inhibiting the expression, stability, and/or activity of Siglec 15 or its binding ligands, *e.g.,* MAG, LRRC4C, or Sialyl-Tn, as identified by the methods of the invention, are useful as candidate compounds useful to treat a cancer in a subject in need thereof, to reduce a tumor size or prolong the survival of a subject in need thereof, or to increase an immune response against a tumor in a subject in need thereof.

Modulators that are capable of increasing the expression, stability, and/or activity of Siglec 15 or its binding ligands, *e.g.,* MAG, LRRC4C, or Sialyl-Tn, as identified by the methods of the invention, are useful as candidate compounds useful to treat an autoimmune disease, or to decrease an inflammatory response in a subject in need thereof.

For example, in one aspect, the present invention provides methods for identifying a compound useful for treating an autoimmune disease or a cancer in a subject. The methods include providing a test compound (or a plurality of test compounds), determining the effect of the test compound on the expression and/or activity of Siglec 15, and selecting a compound which modulates the expression and/or activity of Siglec 15, thereby identifying a compound useful for treating an autoimmune disease or a cancer in the subject. In some embodiments, an increase in the expression and/or activity of Siglec 15 indicates that the compound is useful for treating an autoimmune disease. In other embodiments, a decrease in the expression and/or activity of Siglec 15 indicates that the compound is useful for treating a cancer.

In another aspect, the present invention provides methods for identifying a compound useful for increasing an immune response against a tumor in a subject in need thereof. The methods include providing a test compound (or a plurality of test compounds), determining the effect of the test compound on the expression and/or activity of Siglec 15, and selecting a compound which decreases the expression and/or activity of Siglec 15, thereby identifying a compound useful for increasing an immune response against the tumor in the subject.

In yet another aspect, the present invention provides methods for identifying a compound useful for decreasing a brain inflammatory response in a subject in need thereof. The methods include providing a test compound (or a plurality of test compounds), determining the effect of the test compound on the expression and/or activity of Siglec 15, and selecting a compound which increases the expression and/or activity of Siglec 15, thereby identifying a compound useful for decreasing a brain inflammatory response in the subject.

Examples of modulators, candidate compounds or test compounds include, but are not limited to, nucleic acids (*e.g*., DNA and RNA), carbohydrates, lipids, proteins, peptides, peptidomimetics, small molecules and other drugs. Modulators can be obtained using any of the numerous approaches in combinatorial library methods known in the art, including: biological libraries; spatially addressable parallel solid phase or solution phase libraries; synthetic library methods requiring deconvolution; the "one-bead onecompound" library method; and synthetic library methods using affinity chromatography selection. The biological library approach is limited to peptide libraries, while the other four approaches are applicable to peptide, non-peptide oligomer or small molecule libraries of compounds (Lam (1997) Anticancer Drug Des. 12:145; U.S. Patent No. 5,738,996; and U.S. Patent No. 5,807,683, the entire contents of each of the foregoing references are incorporated herein by reference).

Examples of methods for the synthesis of molecular libraries can be found in the art, for example in: DeWitt et al. (1993) Proc. Natl. Acad. Sci. USA 90:6909; Erb et al. (1994) Proc. Natl. Acad. Sci. USA 91:11422; Zuckermann et al. (1994) J. Med. Chem. 37:2678; Cho et al. (1993) Science 261: 1303; Carrell et al. (1994) Angew. Chem. Int. Ed. Engl. 33:2059; Carell et al. (1994) Angew. Chem. Int. Ed. Engl. 33:2061; and Gallop et al. (1994) J. Med. Chem. 37:1233, the entire contents of each of the foregoing references are incorporated herein by reference. Libraries of compounds may be presented, *e.g.,* presented in solution (*e.g.,* Houghten (1992) Bio/Techniques 13:412-421), or on beads (Lam (1991) Nature 354:82- 84), chips (Fodor (1993) Nature 364:555-556), bacteria (U.S. Patent No. 5,223,409), spores (Patent Nos. 5,571,698; 5,403,484; and 5,223,409), plasmids (Cull et al. (1992) Proc. Natl. Acad. Sci. USA 89: 1865-1869) or phage (Scott and Smith (19900 Science 249:386-390; Devlin (1990) Science 249:404-406; Cwirla et al. (1990) Proc. Natl. Acad. Sci. USA 87:6378-6382; and Felici (1991) J. Mol. Biol. 222:301-310). The entire contents of each of the foregoing references are incorporated herein by reference.

The test compound can be contacted with a cell that expresses the Siglec 15 protein or a molecule with which Siglec 15 directly interacts, *e.g*., MAG or LRRC4C. For example, the test compound can be contacted with a cell that naturally expresses or has been engineered to express the protein(s) by introducing into the cell an expression vector encoding the protein.

Alternatively, the test compounds can be subjected to a cell-free composition that includes the protein(s) (*e.g.,* a cell extract or a composition that includes *e.g*., purified natural or recombinant protein).

Compounds that modulate expression and/or activity of Siglec 15, or a binding ligand of Siglec 15, *e.g*., MAG, LRRC4C, or Sialyl-Tn, can be identified using various "read-outs. "

For example, a cell can be transfected with an expression vector, incubated in the presence and in the absence of a test compound, and the effect of the compound on the expression of Siglec 15 or on a biological response regulated by Siglec 15 can be determined. The biological activities of Siglec 15 include activities determined *in vivo,* or *in vitro,* according to standard techniques. Activity can be a direct activity, such as an association with a binding ligand, *e.g.,* MAG, LRRC4C, or Sialyl-Tn. Alternatively, the activity is an indirect activity, such as an increase in a brain inflammation response.

To determine whether a test compound modulates Siglec 15 protein expression, *in vitro* transcriptional assays can be performed. To determine whether a test compound modulates Siglec 15 mRNA expression, various methodologies can be performed, such as quantitative or real-time PCR.

A variety of reporter genes are known in the art and are suitable for use in the screening assays of the invention. Examples of suitable reporter genes include those which encode chloramphenicol acetyltransferase, beta-galactosidase, alkaline phosphatase, green fluorescent protein, or luciferase. Standard methods for measuring the activity of these gene products are known in the art.

A variety of cell types are suitable for use as an indicator cell in the screening assay. Preferably a cell line is used which expresses low levels of endogenous Siglec 15 and is then engineered to express recombinant protein. Cells for use in the subject assays include eukaryotic cells. For example, in one embodiment, a cell is a fungal cell, such as a yeast cell. In another embodiment, a cell is a plant cell. In yet another embodiment, a cell is a vertebrate cell, *e.g.,* an avian cell or a mammalian cell (*e.g.,* a murine cell, or a human cell).

Recombinant expression vectors that can be used for expression of , *e.g.,* Siglec 15, are known in the art. For example, the cDNAis first introduced into a recombinant expression vector using standard molecular biology techniques. A cDNA can be obtained, for example, by amplification using the polymerase chain reaction (PCR) or by screening an appropriate cDNA library. The nucleotide sequences of cDNAs for or a molecule in a signal transduction pathway involving (*e.g*., human, murine and yeast) are known in the art and can be used for the design of PCR primers that allow for amplification of a cDNA by standard PCR methods or for the design of a hybridization probe that can be used to screen a cDNA library using standard hybridization methods.

In another embodiment, the test compounds can be subjected to a cell-free composition that includes the protein(s) (*e.g.*, a cell extract or a composition that includes *e.g.,* either purified natural or recombinant protein). Siglec 15 expressed by recombinant methods in a host cells or culture medium can be isolated from the host cells, or cell culture medium using standard methods for protein purification. For example, ionexchange chromatography, gel filtration chromatography, ultrafiltration, electrophoresis, and immunoaffinity purification with antibodies can be used to produce a purified or semi-purified protein that can be used in a cell free composition. Alternatively, a lysate or an extract of cells expressing the protein of interest can be prepared for use as cell-free composition.

In one embodiment, compounds that specifically modulate Siglec 15 activity or the activity of a binding ligand in a signal transduction pathway involving Siglec 15 are identified based on their ability to modulate the interaction of Siglec 15 with its binding ligand. The binding ligand can be a mRNA molecule or a protein molecule, *e.g.,* MAG or LRRC4C. Suitable assays are known in the art that allow for the detection of proteinprotein interactions (*e.g*., immunoprecipitations, two-hybrid assays and the like) or that allow for the detection of interactions between Siglec 15 and an mRNA (*e.g*., electrophoretic mobility shift assays, DNAse I footprinting assays and the like). By performing such assays in the presence and absence of test compounds, these assays can be used to identify compounds that modulate (*e.g*., inhibit or enhance) the activity of Siglec 15 with a binding ligand.

Compounds identified in the subject screening assays can be used in methods of modulating one or more of the biological responses regulated by Siglec 15. It will be understood that it may be desirable to formulate such compound(s) as pharmaceutical compositions as described herein prior to contacting them with cells.

Once a test compound is identified that directly or indirectly modulates, *e.g*., Siglec 15 expression or activity by one of the variety of methods described hereinbefore, the selected test compound (or "compound of interest") can then be further evaluated for its effect on cells, for example by contacting the compound of interest with cells either *in vivo* (*e.g.,* by administering the compound of interest to an organism) or *ex vivo* (*e.g.,* by isolating cells from an organism and contacting the isolated cells with the compound of interest or, alternatively, by contacting the compound of interest with a cell line) and determining the effect of the compound of interest on the cells, as compared to an appropriate control (such as untreated cells or cells treated with a control compound, or carrier, that does not modulate the biological response).

In another aspect, the invention pertains to a combination of two or more of the assays described herein. For example, a modulator can be identified using a cell-based or a cell-free assay, and the ability of the modulators to increase or decrease the activity of Siglec 15 or a protein with which Siglec 15 interacts can be confirmed *in vivo, e.g.,* in an animal, such as, for example, an animal model for, *e.g.,* a glioblastoma tumor model.

Moreover, a modulator of Siglec 15 or a molecule in a signaling pathway involving Siglec 15 identified as described herein (*e.g*., an antisense nucleic acid molecule, or a specific antibody, or a small molecule) can be used in an animal model to determine the efficacy, toxicity, or side effects of treatment with such a modulator. Alternatively, a modulator identified as described herein can be used in an animal model to determine the mechanism of action of such a modulator.

In another embodiment, it will be understood that similar screening assays can be used to identify compounds that indirectly modulate the activity and/or expression of Siglec 15 *e.g.,* by performing screening assays such as those described above using molecules with which Siglec 15 interacts, *e.g*., MAG, LRRC4C, or Sialyl-Tn, or any molecules that act either upstream or downstream of Siglec 15 in the pathway.

Compounds identified by the screening assays of the present invention are considered as candidate therapeutic compounds useful for treating diseases, *e.g*., cancer, or autoimmune disease, as described herein. Thus, the invention also includes compounds identified in the screening assays, and methods for their administration and use in the treatment, prevention, or delay of development or progression of diseases described herein.

It is to be understood that this invention is not limited to particular assay methods, or test agents and experimental conditions described, as such methods and agents may vary. It is also to be understood that the terminology used herein is for the purpose of describing particular embodiments only, and is not intended to be limiting.

The present invention is further illustrated by the following examples, which are not intended to be limiting in any way. The entire contents of all references, patents and published patent applications cited throughout this application, as well as the Figures, are hereby incorporated herein by reference.

### EXAMPLES

### Example 1. Identification of Siglec 15 by Genome-Scale T-Cell Activity Array Technology

In order to conduct human genome-wide searches of targets for immunotherapy, a genome-scale T cell activity array (GS-TCAA) was developed. 6402 human membrane cDNAs, covering 90% of the human membrane genome, were prepared using Qiagen miniprep kits, quantified and diluted for GS-TCAA construction.

A set of human receptor arrays containing four 1536-well plates was spun down (600 g, 2 min), followed by reverse transfection. Briefly, 1536-well array plates were dispensed with 2 µl optiMEM containing lipofectamine 3000 (7 µl lipo/ml) per well using a robotic dispenser (Multidrop Combi, Thermo Scientific), and quickly shook for 1 min by an ultra-speed orbital shaker. All plates were stored in room temperature for 20 min, followed by the addition of 293T.2A.m.anti-CD3 cells for T cell stimulation (2000 cells in 4 µl per well) by Multidrop. After that, the plates were further spin down (1000 g, 4 min) to get rid of the bubble inside each well before incubation at 37 degrees. The T cell reporter cell, or primary T cells (4000 cells), such as engineered Jurkat cell lines with different GFP reporters, were loaded into the array plates 24 hours after transfection. Plate imaging was performed 12 hours after co-culture of T cells with 293T based T cell stimulators by an InCell image analyzer (GE). After optimal imaging analysis using Cellprofiler software, gene candidates with potential regulatory functions were identified. For example, if a molecule is not effective in modulating the T cell activity, the GFP signal will remain similar. If the molecule acts as an stimulatorgy signal for the T cell activity, then a relateively stronger GFP signal will be detected. Alternatively, if the molecule is inhibitory, a much weaker GFP signal will be observed. Based on the genome-scale T cell activity array, Siglec 15 was identified as an immune modulator, suggesting that Siglec 15 may have an immunolgocial function.

### Example 2. Brain and Myeloid-related Expression of Siglec 15

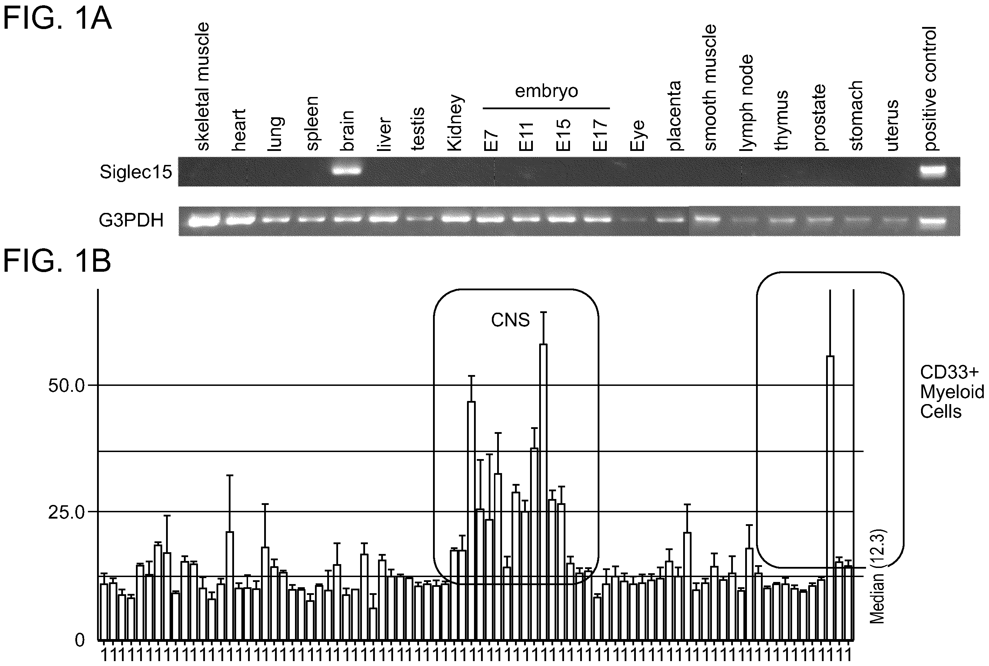

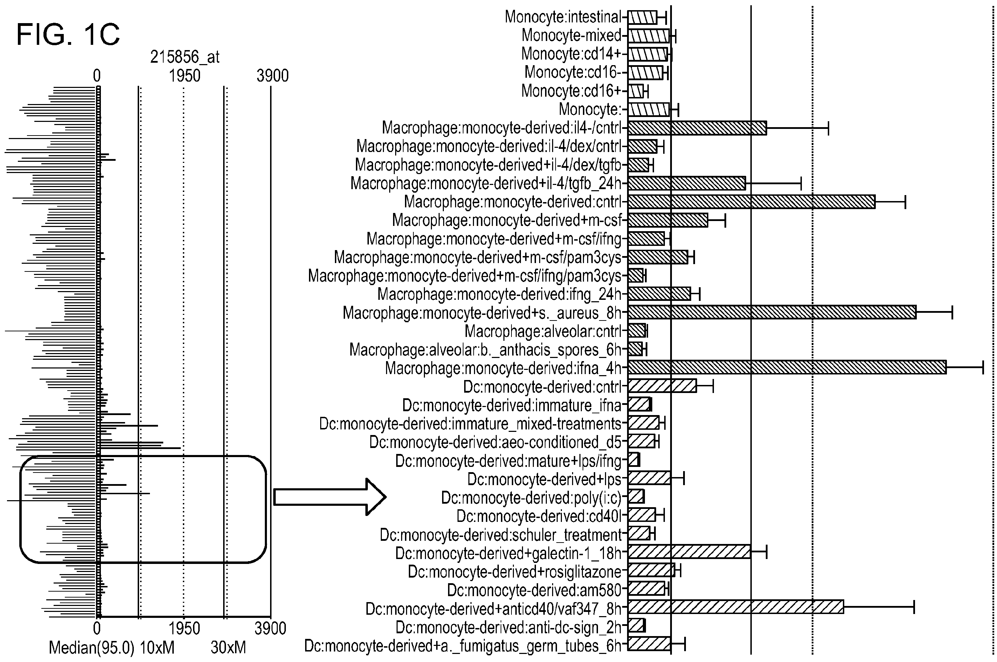

Siglec 15 expression was evaluated in different mouse tissues using RT-PCR with a mouse cDNA library (Clontech Laboratories). A Siglec 15 plasmid (Origene) was used as a positive control. Results are shown in Figure 1A. Microarray analysis of Siglec 15 RNA expression in human tissues (BioGPS.org) is shown in Figure 1B. This data indicates that Siglec 15 is expressed in brain and myeloid-related cells in both human and mouse, under normal conditions. Microarray data from BioGPS is presented in Figure 1C, demonstrating that Siglec 15 is expressed on monocytes, macrophages, dendritic cells, B cells, and osteoclasts. Accordingly, Siglec 15 may have a physiological function in brain-related diseases, and/or regulation of immune response.

### Example 3. Expression of Siglec 15 in Cancer

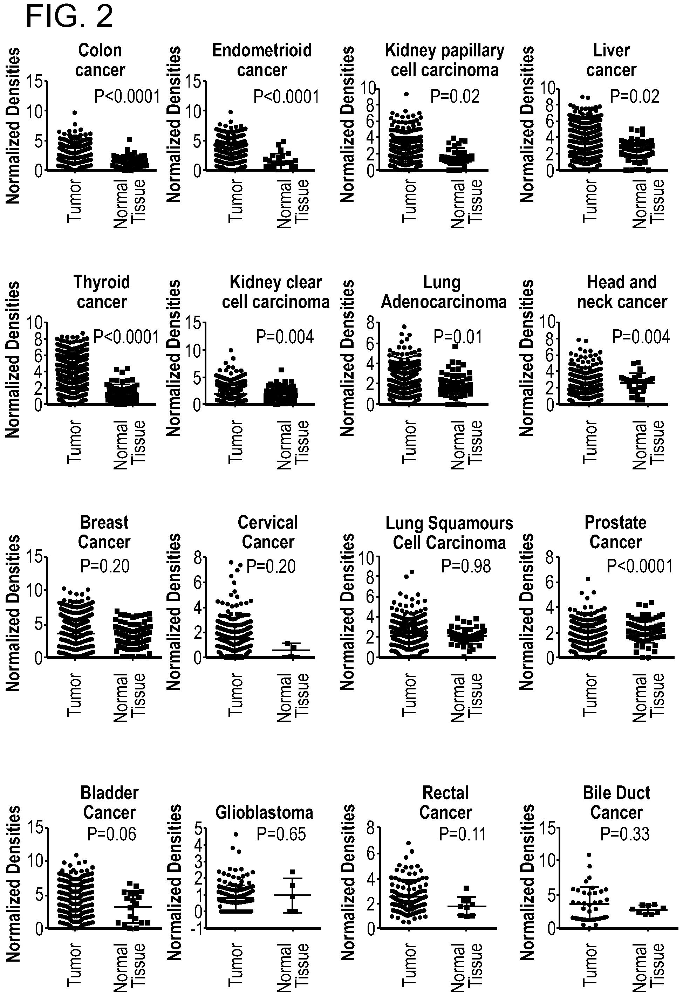

A meta-analysis of Siglec 15 expression in human cancer was performed using the TCGA cancer microarray database. Siglec 15 mRNA expression in many human cancers was compared to counterpart normal tissue (Figure 2). Original datasets were normalized using the UCSC Cancer Genomics Browser software (https://genome-cancer.ucsc.edu), and were analyzed using the R program. This data indicates that Siglec 15 is overexpressed in many human cancers, and may play a role in cancer development and progression.

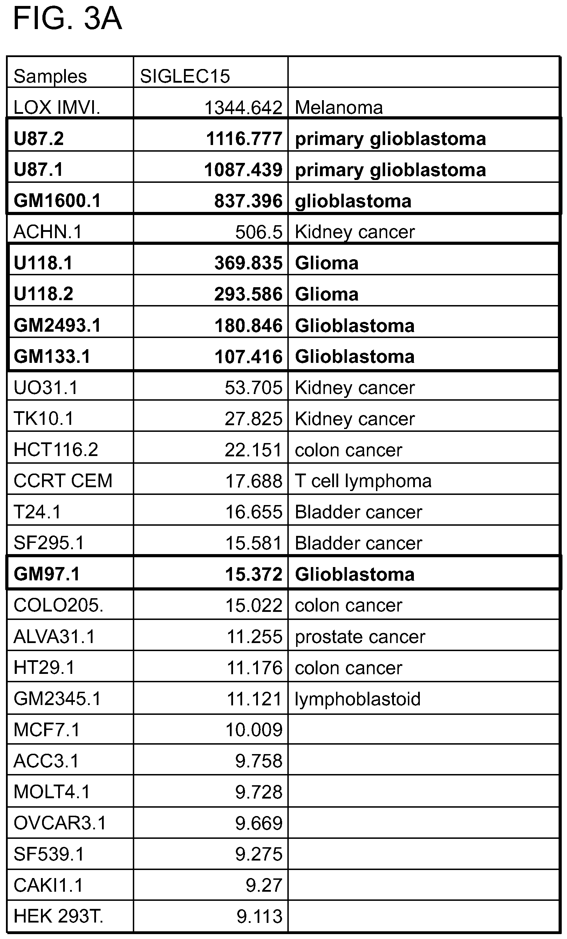

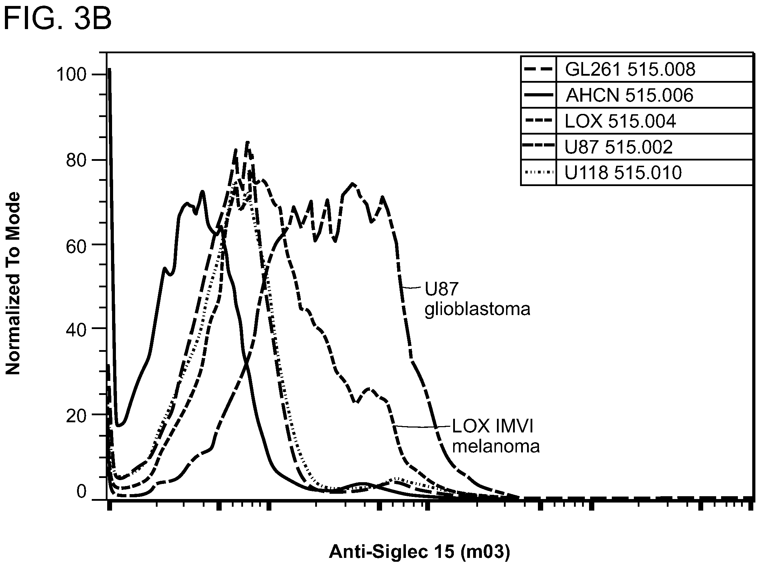

Siglec 15 expression was also evaluated in several human cancer cell lines. Expression of Siglec 15 in human cancer cell lines was determined by NCI60 microarray (BioGPS) (Figure 3A, expression values are shown in arbitrary units). Several human cancer cell lines were stained with anti-Siglec 15 antibody m03. Antibody m03 was generated by immunization of NZB/W F1 mice with a mouse Siglec15 ectodomain fusion protein, and cross-reacts with human Siglec 15. Stained cells were analyzed for Siglec 15 expression using FACS (Figure 3B). This data indicates that Siglec 15 is also upregulated in human tumor cell lines.

### Example 4. Identification of MAG or LRRC4C as Ligands for Siglec 15 by Receptor Array Technology

To identify the ligand for Siglec 15, a Siglec 15-Fc fusion protein was generated by fusing the extracellular domain of each molecule with a mouse or human Fc tag (Dong H, et al., Nat Med. 1999; 5(12): 1365-9) and screened in the newly established genome scale human receptor array (Yao, S et al., Immunity 2011, 34(5):729-40). Briefly, the complete human membrane gene library containing over 6,200 genes was collected, maintained, and diluted by OPTI-MEM media and placed individually into four 1,536-well plates at 40 ng/well, through a robotic system. Lipofectamine 2000 was added to each well and mixed with plasmids for 30 minutes. Two thousand HEK293T cells were added subsequently to each well to perform transient transfection. Eight hours after transfection, 10 ng of human Siglec 15-Fc and anti-Fc FMAT blue secondary antibody were added to each well. The plates were read 24 hours after transfection using the Applied Biosystems 8200 cellular detection system and analyzed using the CDS 8200 software. Human Fc Receptor genes served as internal positive controls for the assay.

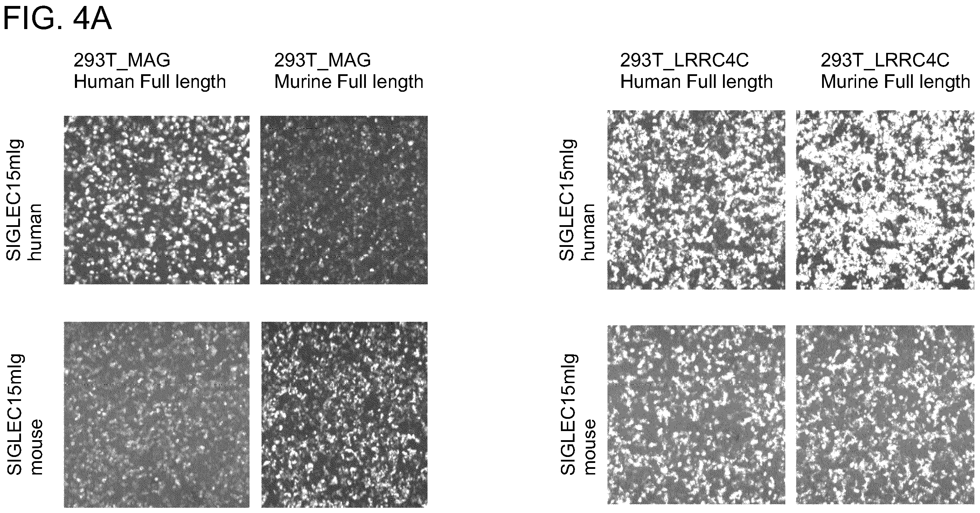

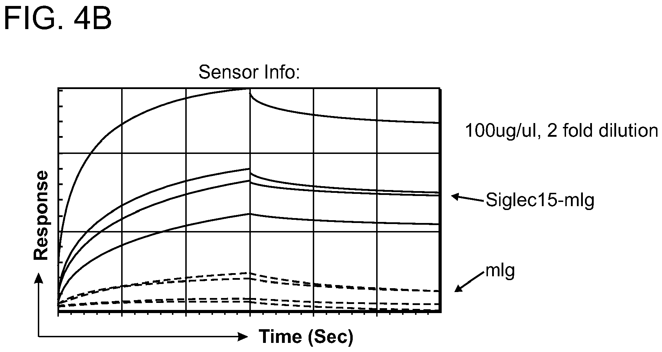

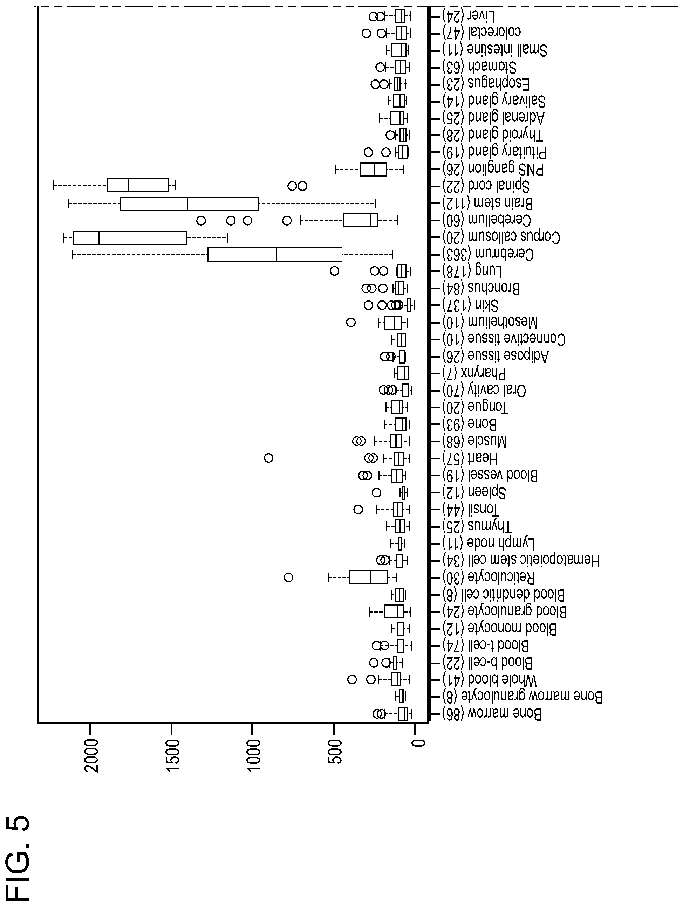

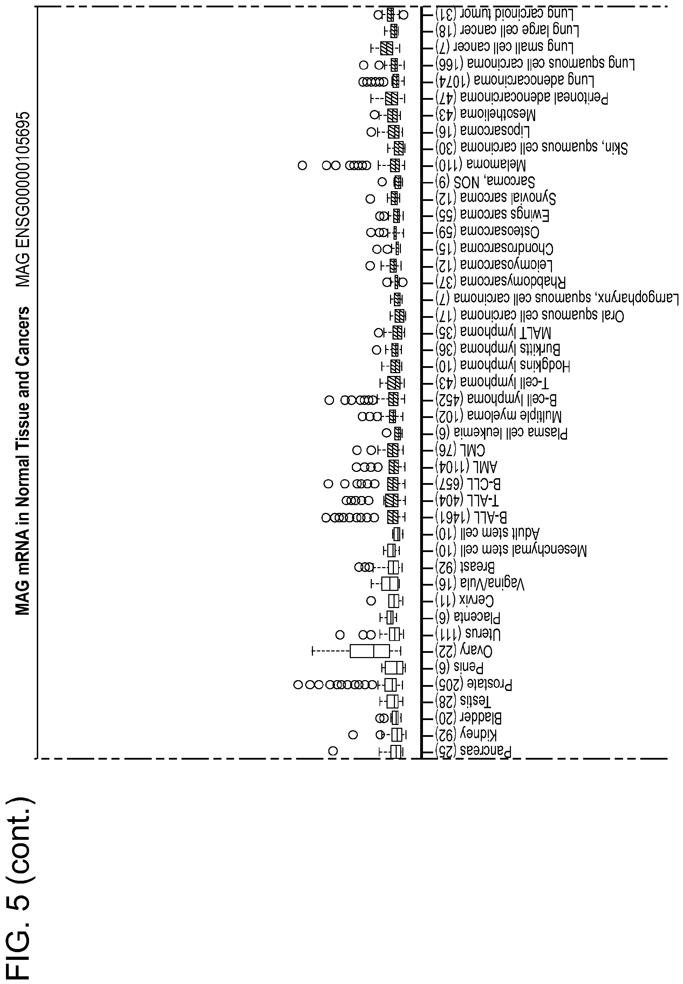

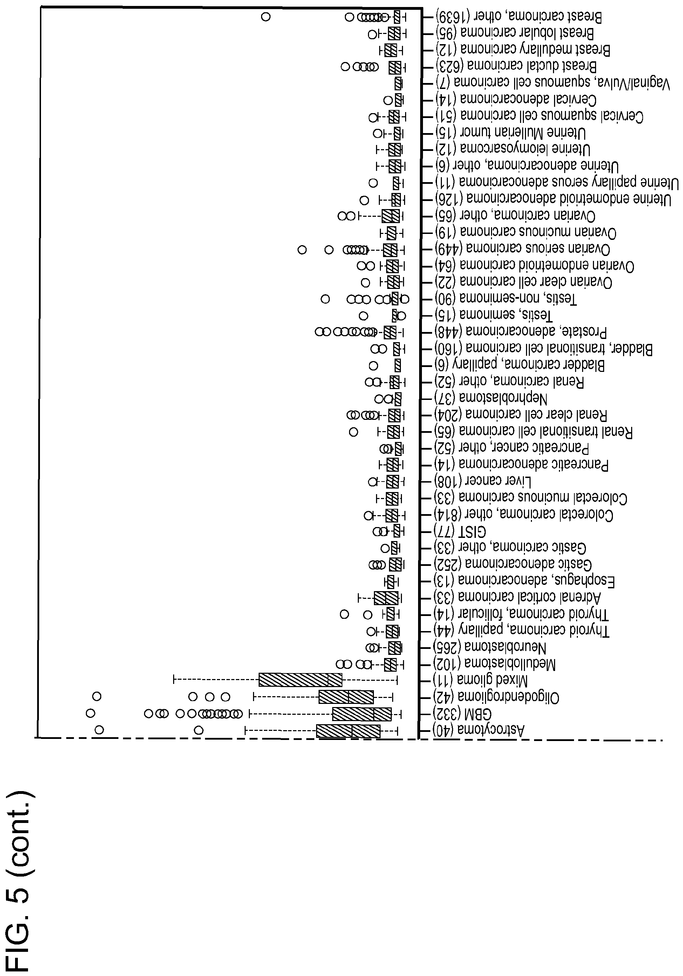

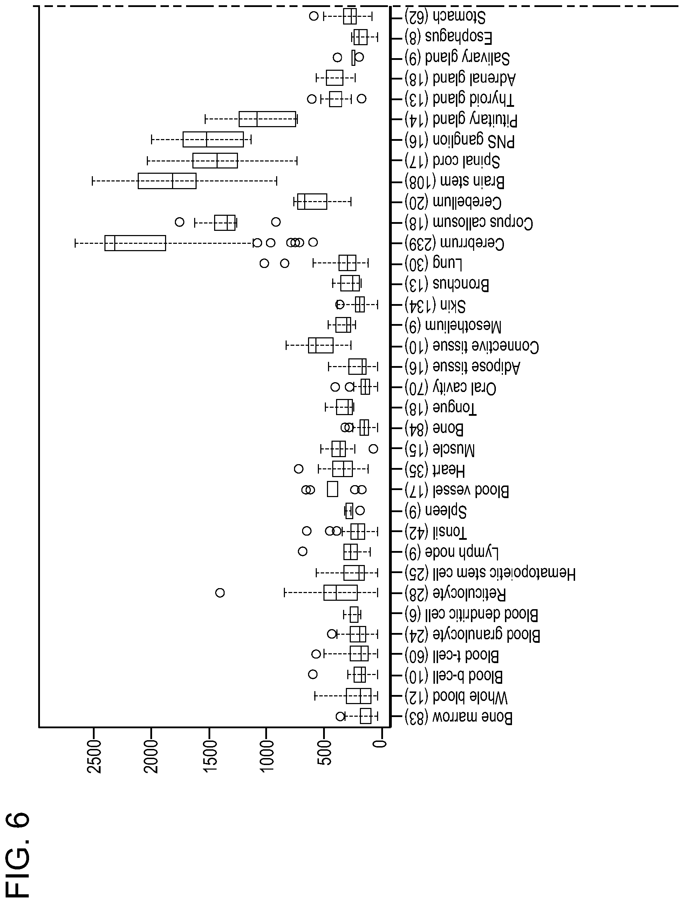

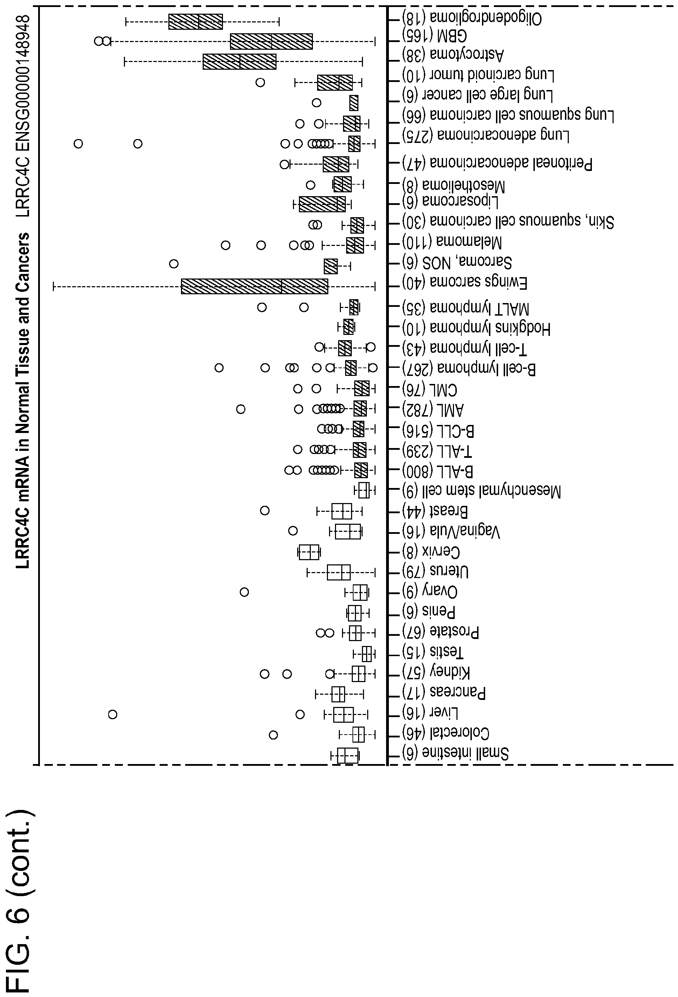

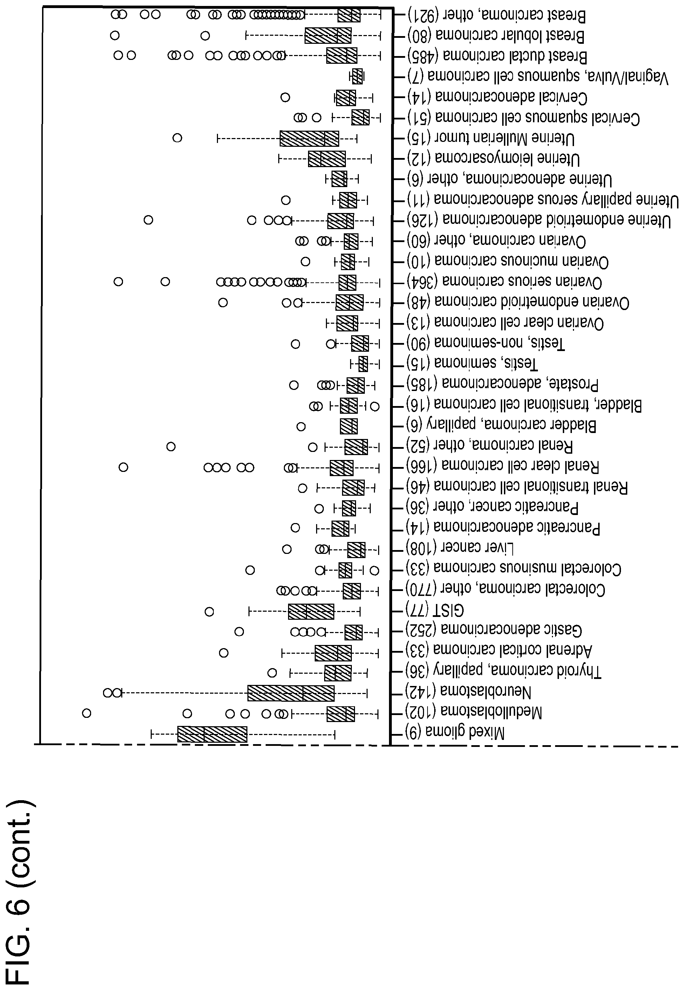

Two positive hits were identified for ligands for Siglec 15: myelin-associated glycoprotein (MAG) and leucine rich repeat containing 4C (LRRC4C) (Figure 4A). MAG, also named Siglec 4, is primarily found in the brain and participates in neuron myelination. It is also present in myeloid cells and especially in microglia. MAG knockout (KO) mice have problems with phagocytosis. LRRC4C is an LRR family molecule associated with neuron-related growth, dendrite formation, and axon extension. LRRC4C is mainly localized to the postsynaptic side of excitatory synapses and interacts with the presynaptic ligand, netrin-G1, to regulate excitatory synapse formation. Expression of MAG was shown to be enriched in brain or brain related tumor (Figure 5), and LRRC4C mRNA levels were also up-regulated in several kinds of cancers such as brain cancer, breast cancer, ovarian cancer, renal cell carcinoma and Ewing sarcoma (Figure 6). It has previously been reported that Siglec 15 interacts with Sialyl-Tn. To confirm this observation, binding of Sialyl-Tn antigen Neu5Ac α2-6GalNAc to Siglec 15 fusion protein (Siglec 15-mIg) was measured using Octet streptavidin biosensors (ForteBio). The Octet streptavidin biosensors were pre-loaded with NeuAa-2-6 GalNac-Biotin (Glycotech, 50 µg/ml), and responses to either Siglec15-mIg or control mIg (2-fold serial dilution starting from 100 µg/ml) over time were determined (Figure 4B). Sialyl-Tn expression has been documented in many human and mouse cancers. Accordingly, Sialyl-Tn may be an additional binding partner that serves as a ligand/receptor for Siglec15.

The specificity of interaction between Siglec 15 and MAG or LRRC4C was further verified by flow cytometry analysis. All antibodies for flow cytometry staining were purchased from BD Bioscience (San Jose, CA) or eBioscience (San Diego, CA). Siglec 15 fusion protein bound strongly to HEK293T cells transfected with MAG or LRRC4C, but did not bind to control cells, and inclusion of anti-Siglec15 mAb completely blocked this interaction. Indeed, the interaction between Siglec 15 and MAG or LRRC4C is well conserved between mouse and human. As demonstrated in Figure 4A, human Siglec 15 could bind both human and mouse MAG, and mouse Siglec 15 could also recognize both mouse and human LRRC4, suggesting a cross-species interaction.

### Example 5. Identification of the Binding Domain of Siglec 15 for MAG and LRRC4C

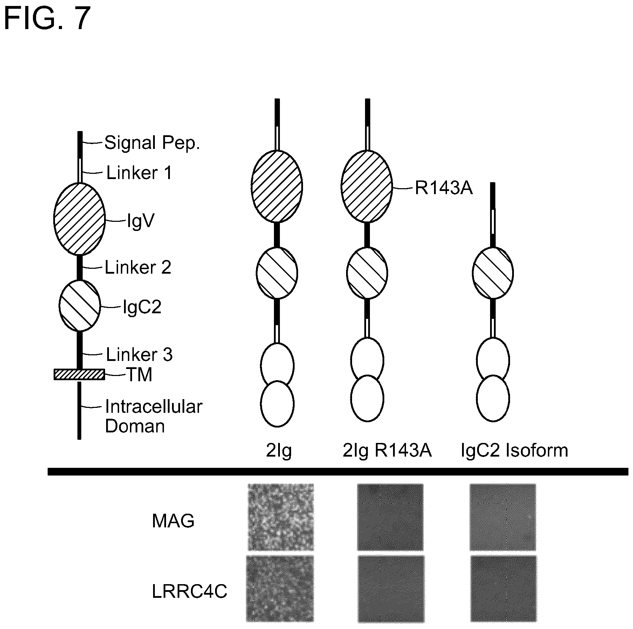

In order to determine the binding domain of Siglec 15 for MAG or LRRC4C, Siglec 15 mutants with domain deletions were constructed and purified. Siglec 15 contains an intracellular domain, a transmembrane domain, an IgC domain and an IgV domain. Human or mouse Siglec 15 was made via a PCR method similar to what was previously described (Sedy JR, et al., Nat Immunol. 2005; 6(1): 90-8). Siglec 15 point mutations were selected according to previous publications and generated using PCR (Cheung TC, et al., Proc Natl Acad Sci USA. 2005; 102(37): 13218-23; Compaan DM, et al., J Biol Chem. 2005; 280(47): 39553-61). As demonstrated in Figure 7, deletion of the IgV domain from Siglec 15 completely abolished its interaction with MAG and LRRC4C. In addition, a point mutation at residue 143 of the IgV domain (R143A mutation) was able to eliminate the interaction between Siglec 15 and MAG or LRRC4C, suggesting that the IgV domain of Siglec 15 is required for interaction with MAG or LRRC4C, more specifically, the interaction involves the R143 residue in the IgV domain.

### Example 6. Siglec 15 Directly Inhibits T Cell Activities

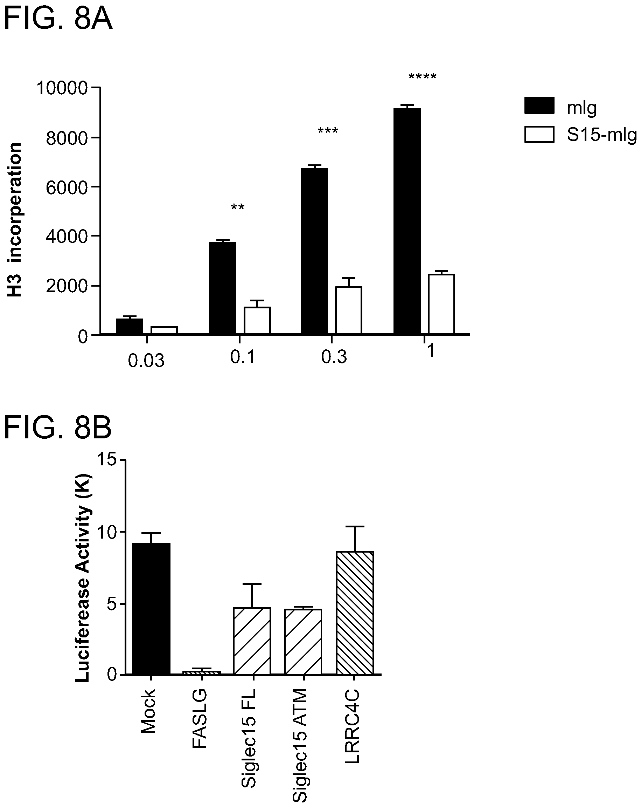

To determine the effect of Siglec 15 on human T cell activity, human PBMCs were stimulated with immobilized anti-CD3 antibody (ranging from 0.03 µg/ml to 1 µg/ml), and either immobilized human Siglec15-mIgG fusion protein (S15-mIg) or control mouse IgG (mIg) (5 µg/ml), for 72 hours. ³H Thymidine incorporation in proliferated T cells was analyzed 16 hours later (Figure 8A). Cells exposed to S15-mIg had significantly reduced levels of ³H thymidine incorporation, indicating that contact with Siglec 15 as a ligand reduced T cell proliferation.

A membrane-associated OKT3 expression construct was generated by fusing nucleic acid encoding an scFv fragment of the anti-human CD3 antibody OKT3 with a nucleic acid encoding CD14. This construct was transfected into 293T cells to generate 293T.m.OKT3 cells, which express the OKT3 scFv on the cell surface. Jurkat NF-AT luciferase reporter cells were co-cultured for 12 hours with 293T.m.OKT3 cells overexpressing mock plasmid, full length FASLG (Fas ligand), LRRC4C, Siglec15, or a gene encoding the Siglec15 ectodomain with the B7-H6 transmembrane domain (Siglec 15 ATM). Luciferase activity was monitored 4 hours after the co-culture (Figure 8B). In this system, over-expression of FASLG nearly eliminated the NF-AT signal compared to the Mock plasmid. In addition, we observed that either Siglec15 full length or Siglec15 ATM expression could similarly inhibit NF-AT luciferase signals significantly.

The foregoing experiments indicate that human Siglec 15, acting as a ligand, can inhibit T cell activities.

To confirm that murine Siglec 15 similarly inhibits the activity of murine T cells, ³H thymidine incorporation in mouse splenocytes was analyzed following exposure to murine Siglec 15. Mouse splenocytes were stimulated with immobilized anti-CD3 (ranging from 0 µg/ml to 2µg/ml), and were exposed to immobilized or soluble mouse Siglec15-mIgG fusion protein (S15-mIg) or control mouse IgG (mIg) (5ug/ml), for 72 hours. Thymidine incorporation in proliferated T cells was analyzed 16 hrs later. ³H Thymidine incorporation in cells exposed to immobilized Siglec15-mIgG is shown in Figure 9A. ³H Thymidine incorporation in cells exposed to soluble Siglec15-mIgG is shown in Figure 9B. Cells exposed to murine S15-mIg had reduced levels of³H thymidine incorporation, indicating that contact with Siglec 15 ligand reduced proliferation of mouse splenocytes.

293T cells were transfected with plasmids encoding H2-Kb fused with OVA peptide (SIINFEKL) to generate 293T-Kb-OVA cells. The cells were then infected with a lentiviral vector encoding full-length Siglec 15, to generate 293T-Kb-OVA-S15 cells. Several cell lines with variable Siglec 15 expression were isolated by FACS screening. OT-1 mice transgenic for an OVA-specific TCR recognizing the H-2kb OVA SIINFEKL peptide were obtained from The Jackson Laboratory. Splenocytes from OT-1 transgenic mice were pre-activated with SIINFEKL peptide plus IL-2 for 3 days. Activated cells were then co-cultured with 293T-KbOVA cells over-expressing full-length mouse Siglec 15 (KbOVA-S15) or a mock transfected control (KbOVA-control), for 3 days. ³H thymidine incorporation in proliferated T cells was analyzed 16 hrs later (Figure 9C). Splenocytes exposed to Siglec 15 in the context of 293T-KbOVA cells had reduced levels of ³H thymidine incorporation, indicating that contact with cell-based Siglec 15 reduced proliferation of mouse splenocytes.

Different levels of mouse Siglec15 expression were identified on three 293T-KbOVA cell lines over-expressing mouse Siglec15, as indicated by FACS analysis following staining with anti-Siglec15 antibody m03 (Figure 9D). Splenocytes from OT-1 transgenic mice were pre-activated with SIINFEKL peptide and IL-2, as described above. Activated splenocytes were then co-cultured with 293T-KbOVA cell lines having increasing levels of Siglec15 expression as shown in Figure 9D, for 3 days. The IFN-γ levels (Figure 9E), and TNF-α levels (Figure 9F) in the supernatant were measured by Cytometric Bead Array (CBA) (BD Pharmingen). IFN-γ and TNF-α production was reduced with increasing levels of Siglec 15 expression, indicating that Siglec 15 inhibits T cell function in a dose-dependent manner.

### Example 7. Cell-Associated Siglec 15 Reduces T Cell Cytotoxicity

An adherence-based cytotoxicity assay was used to examine the effect of Siglec 15 on T cell cytotoxicity and the relevance to tumor killing.

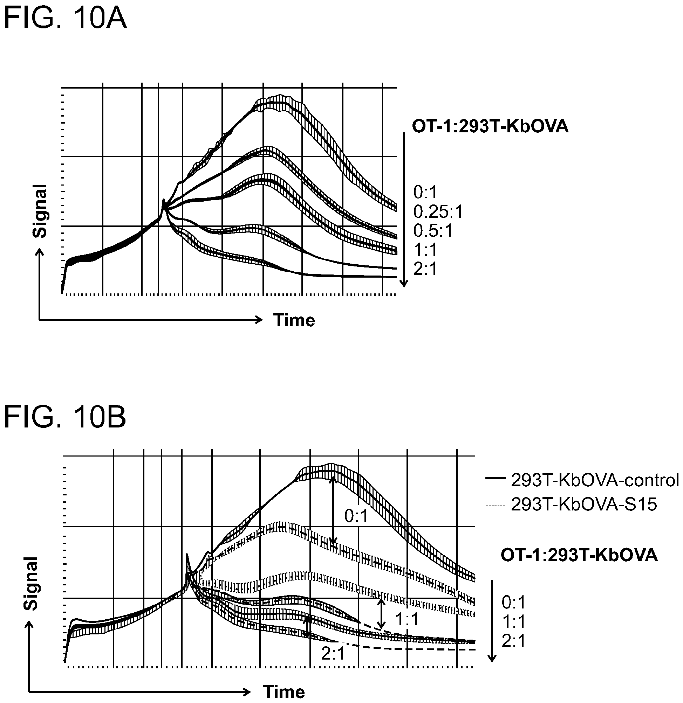

293T-KbOVA target cells were placed in a 384 E-Plate (ACEA Biosciences) at a density of 10⁴ cells/well, and were grown overnight. Cells adherent to the plate generate increasing electrical signals. If left undisturbed, the electrical signal would increase over time, and then would decline due to over-growth of the cells. To test the effect of antigen-specific T cells in this system, the 293T-KbOVA target cells were co-cultured with different ratios of pre-activated OT-1 T cells (ranging from 0:1 to 2:1). The adherence signal of target cells growing over time is shown in Figure 10A. A doesresponse relationship was observed with respect to the level of OT-1 T cell killing of 293T-KbOVA target cells. The higher the ratio of OT-1 T cells:293T-KbOVA target cells present in the assay, the less signal was detected, indicating that fewer live 293T-KbOVA target cells were detected with increasing amounts of OT-1 T cells.

In this system, T cell killing of target cells with Siglec15 overexpression (293T-KbOVA-S15) was compared with T cell killing of target cells that are Siglec 15- negative (293T-KbOVA-control), in the presence of varying ratios of OT-1 T cells. The signal of 293T-KbOVA cells overexpressing Siglec15 was weaker than control cells in the absence of T cells (0:1 ratio). However, cells overexpressing Siglec15 generated a higher signal in the presence of T cells (1:1 or 2:1 ratio), which indicates resistance to T cell killing (Figure 10B). This data indicates that cell-associated Siglec 15 (*e.g*., on Siglec 15 expressing tumor cells) may render cells resistant to T cell cytotoxicity.

### Example 8. Deficiency of Siglec 15 Elicits Increased T Cell Responses

Wild type (WT) or Siglec15 whole body knockout (KO) mice intravenously (i.v.) received splenocytes from OT-1/RagKO mice according to the schedule in Figure 11A. Briefly, mice received 2×10⁶ splenocytes at day -1, and were boosted intraperitoneally (i.p.) with 100 µg OVA peptide plus 100µg poly i:c at day 0. The percentage of OT-1 cells relative to the total CD8 T cell population was assessed in the blood at day 4, and in the spleen at day 5 (Figure 11B). The percentage of OT-1 cells was determined by FACS staining using OT-1 tetramer.

The percentage of antigen-specific CD8 T cells in the blood and in the spleen was increased in the Siglec 15 KO mice compared to WT mice. This result suggests that removing the inhibitory effect of Siglec 15 allows T cells to respond more robustly to antigen priming.

OT-1 T cells were injected into WT or S15KO mice at day -1, followed by peptide stimulation at day 0, as shown in Figure 11A. The mice were fed with 5-ethynyl-2'-deoxyuridine (EdU) (0.8 mg/ml) in the drinking water starting at day 0, and changed every two days. The proliferation of blood OT-1 cells was analyzed on day 5 by anti-EdU staining, and calculated as the percentage of EdU positive OT-1 cells/total OT-1 positive cells (Figure 11C). Splenocytes were also isolated and cultured overnight without stimulation and staining for annexin V. Apoptosis was calculated by annexin V-positive OT-1 cells/total OT-1 positive cells (Figure 11D).

### Example 9. Myeloid-derived Siglec 15 is Largely Responsible for Siglec 15 Function In Vivo

To confirm the cell population responsible for the Siglec15 response, a macrophage-specific Siglec15 knock out mouse (LysM-Cre KO) was developed, which permitted an analysis of which cells are expressing Siglec15 and inhibiting T cells. Siglec 15 conditional knockout mice purchased from MMRRC (Mutant Mouse Resource & Research Centers) (Strain B6.Cg-*Siglec15^{tm1.1Cfg}*/Mmucd; Reference no. MMRRC:032723-UCD) were backcrossed with LysM-Cre mice (Jax Labs) to generate LysM-Cre Siglec 15 knockout mice. These mice were later backcrossed with C57BL6 mice for over 6 generations before performing experiments.

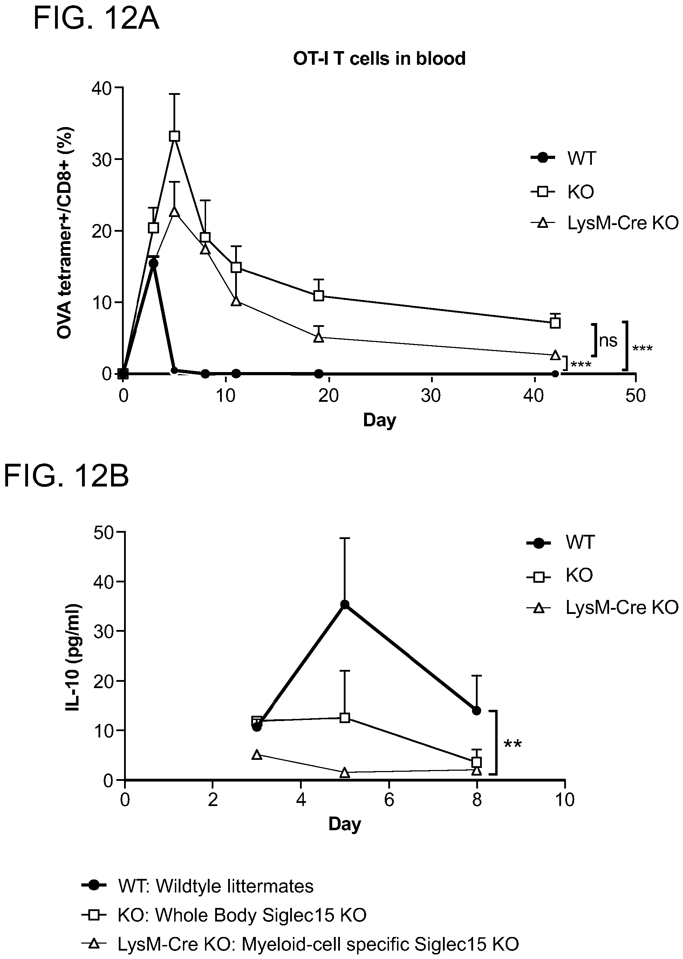

The OT-1 T cell transfer system described in Example 8 was used to compare the expansion of OT-1 T cells in the Siglec 15 whole-body knockout mouse (KO), and the macrophage-specific Siglec 15 knock out mouse (LysM-Cre KO). Blood was collected at different time points, and the percentage of OT-1 cells in the total CD8 T cell population was determined (Figure 12A). Expansion of OT-1 T cells peaks at day 3 for WT, and later for the two types of KO mice. The contraction phase, the phase where the T cells contract after their initial expansion, remains higher in both KO models, suggesting that in the absence of Siglec15 inhibition, T cells are maintained at higher numbers. The Siglec15 whole-body KO and LysM-Cre KO behave similarly, suggesting that macrophages may be largely responsible for the inhibitory effect of the Siglec15 molecule. IL-10 level in the plasma of whole body KO and LysM-Cre KO mice was also assessed during OT-1 T cell reaction, and compared with wild-type (Figure 12B). A lower level of IL-10 was identified in the plasma of whole-body KO and LysM-Cre KO mice, relative to wild-type.

### Example 10. Deficiency of Siglec 15 has Little Effect on Endogenous Immune Cell Pools

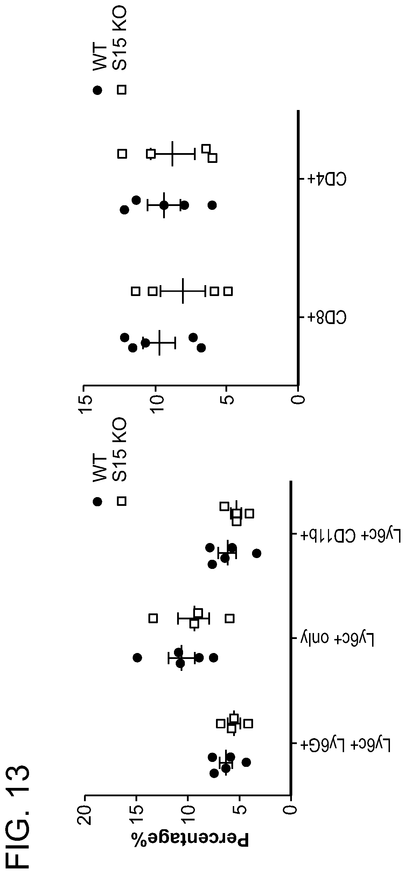

To determine whether the deficiency of Siglec 15 in knock out mice affects the composition of endogenous pools of immune cells, the percentage of myeloid, CD8, or CD4 populations in the blood of wild-type (WT) and Siglec15 knockout (S15KO) mice was determined at 11 months of age (Figure 13).

These data demonstrate that although antigen-specific T cell responses are significantly enhanced in Siglec15 KO mice, these mice do not have obvious inherited immune phenotypes (either myeloid immune cells or T cells) under normal conditions. This finding indicates that Siglec15 may operate in an induced pattern of expression and/or functionality.

### Example 11. Cell-based Siglec 15 Inhibits Macrophage Responses

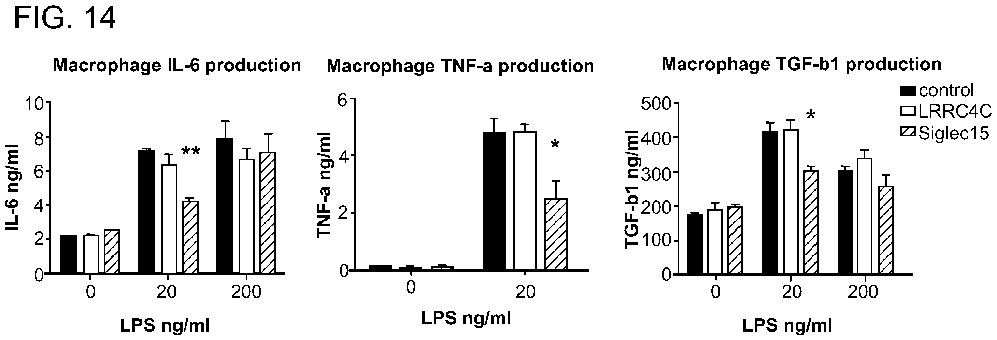

Mouse peritoneal macrophages were co-cultured with 293T cells over-expressing mock plasmid (Control), full-length LRRC4C, or Siglec15, in the presence of different doses of LPS for 24 hrs. Cytokine levels in the supernatant were measured by Cytometric Bead Array (CBA) (BD Pharmingen). Macrophage production of IL-6, TNF-α, and TGF-β1 was reduced when the cells were co-cultured with cells over-expressing Siglec 15 (Figure 14), This data indicates that cell-based Siglec 15 directly inhibits myeloid cell responses. Accordingly, as a ligand, Siglec 15 may act through both myeloid cells and T cells to impose inhibitory activities on the immune system.

### Example 12. Effect of Blocking the Interaction between Siglec 15 and MAG or LRRC4C in EAE mice

To determine the role of the interaction between Siglec 15 and MAG or LRRC4C in regulating brain inflammatory diseases, a mouse model of experimental autoimmune encephalomyelitis (EAE) was used. EAE is an inflammation model of encephalitis and demyelination, which affects the spinal cord and the brain causing paralysis. The EAE model is widely used as a model of the human inflammatory demyelinating disease multiple sclerosis (MS). The degree of paralysis can be quantitated via an EAE score.

Briefly, female C57BL/6 mice (6-10 weeks old) were purchased from the National Cancer Institute, NIH (Frederick, MD). C57BL/6 mice at 8-12 weeks of age were immunized subcutaneously on day 0 with 100 µg MOG peptide (35-55) emulsified in complete Freund's adjuvant (CFA) (Difco) to induce EAE. 400 ng Pertussis toxin (Sigma) in 200 µl PBS was injected twice, on days 0 and 2. Each mouse was injected intraperitoneally with 200 µg Siglec 15 mAb (S15m02, S15m03) or control antibody, or fusion protein as indicated, at day 7 and day 10. The mouse anti-mouse Siglec 15 mAbs were generated by immunizing a NZB/W F1 mouse with mouse Siglec 15-Ig fusion protein. All fusion proteins were generated by fusing the extracellular domain of each molecule with a mouse or human Fc tag (Dong H, et al., Nat Med. 1999; 5(12): 1365-9).

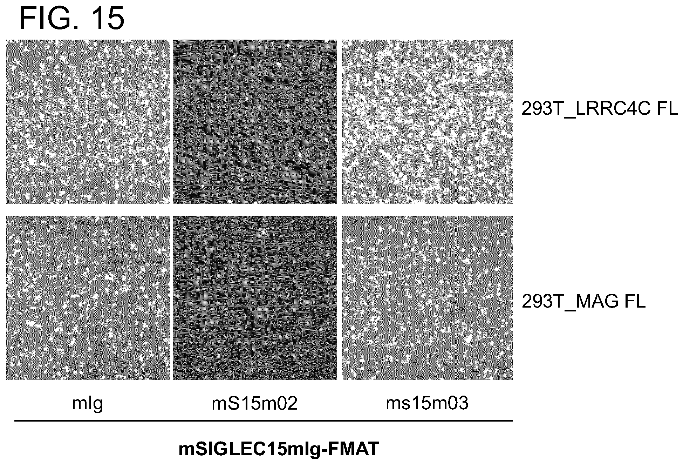

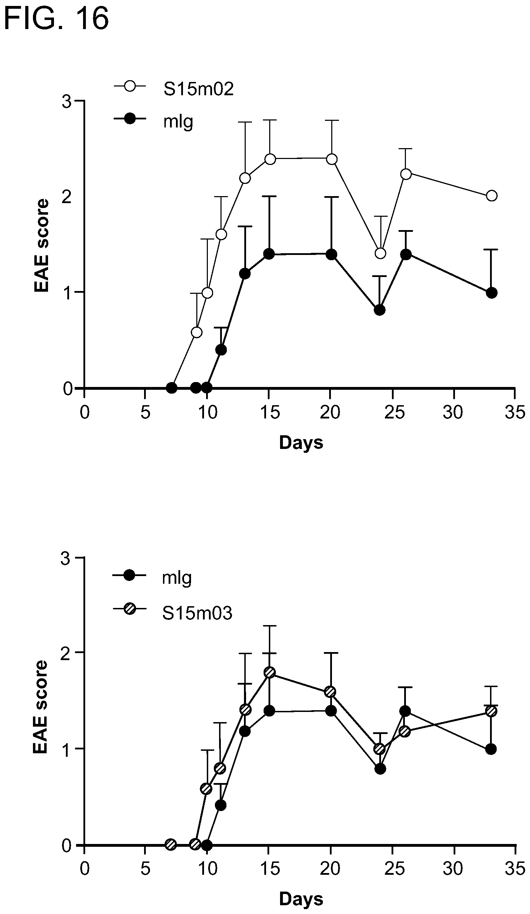

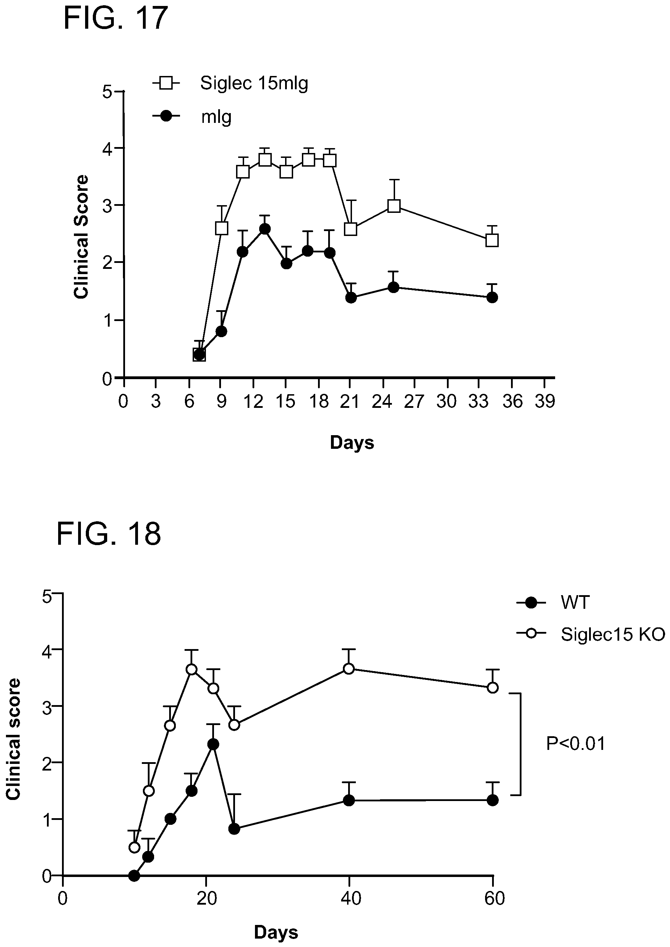

Disease severity was scored on the following scale: 0, no disease; 1, tail paralysis; 2, paraparesis; 3, paraplegia; 4, paraplegia with forelimb weakness or paralysis; 5, moribund or dead, as described previously (Stromnes IM, et al., Nature protocols. 2006; 1(4): 1810-9). Figure 15 illustrated that Siglec 15 antibody clone S15m02 acted as a blocking antibody that disrupted Siglec 15 from binding MAG or LRRC4C. As a result, mice receiving the S15m02 antibody developed more severe disease symptoms than their counterparts injected with control mAb or receiving S15m03 antibody (Figure 16), suggesting that interaction between Siglec 15 and MAG or LRRC4C is critical in regulating inflammatory responses in brain. The results were further confirmed by treating EAE mice with the Siglec 15-Fc fusion protein Siglec 15-mIg. EAE was accelerated after mice received the Siglec 15-Fc fusion protein (Figure 17).

### Example 13. Role of Siglec 15 in Suppressing Brain Inflammatory Responses

To confirm that Siglec 15 plays a role regulating brain inflammation, a Siglec 15 knockout (KO) mouse model were purchased from MMRRC (UC Davis) (Tao et al. J Immunol. 2008; 180(10): 6649-55). Both the wild-type (WT) and Siglec15 knockout mice were immunized with MOG (33-35) peptide to induce experimental autoimmune encephalomyelitis (EAE), followed by the measurement of clinical scores in EAE disease. As demonstrated in Figure 18, Siglec 15 KO mice exhibited more severe EAE disease symptoms than WT mice, indicating an inhibitory role of Siglec 15 in the regulation of brain inflammatory responses.

### Example 14. Deficiency of Siglec 15 Enhances Autoimmunity

Wild-type (WT) and Siglec15 knockout (Siglec15 KO) mice were immunized with MOG peptide to induce EAE. All mice were boosted with pertussis toxin at day 0 and day 1. EAE clinical scores of WT and Siglec15 KO mice were determined, confirming that Siglec 15 KO mice display more severe EAE disease symptoms than WT mice.

A separate cohort of wild-type (WT) mice were immunized with MOG peptide and boosted with pertussis toxin at day 0 and day 1, followed by treatment with a Siglec 15-mIg fusion protein (Siglec15-mIg) or control mIg, or a Siglec 15-hIg fusion protein (Siglec15-hIg) or control hIg (100 µg) twice per week starting at day 6, for a total of 4 doses. EAE clinical scores were evaluated over time (Figure 19A). At day 12, the splenocytes from control mIg treated mice were re-stimulated with MOG peptide (60 µg/ml) for 3 days, in the presence of 5ug/ml Siglec15-mIg (S15-mIg) or control mIg (mIg). ³H-thymidine incorporation in both groups was measured after further incubation for 16 hrs (Figure 19B). This data indicates that soluble Siglec15-mIg can behave as an antagonist *in vivo* and stimulate antigen-specific T cell responses. This mechanistic study furthers our understanding of the functional role of Siglec15 fusion protein in the brain inflammation model (EAE).

### Example 15. Role of Siglec 15 in Suppressing Immune Reponses in Brain Cancer

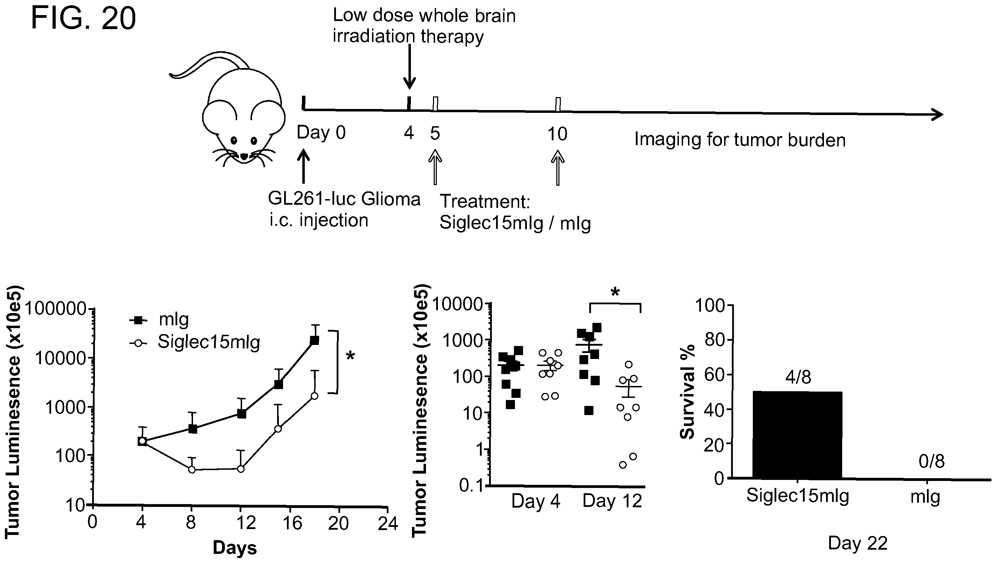

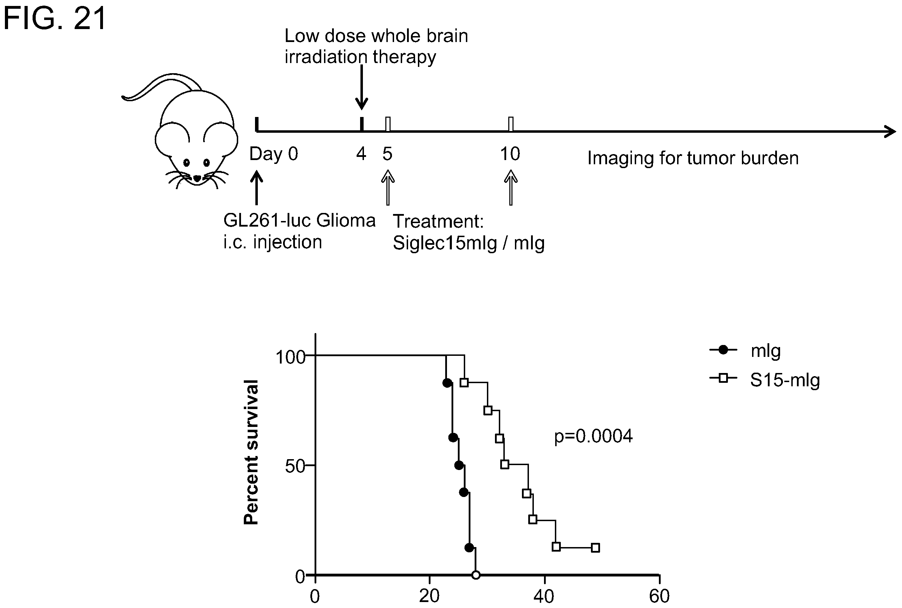

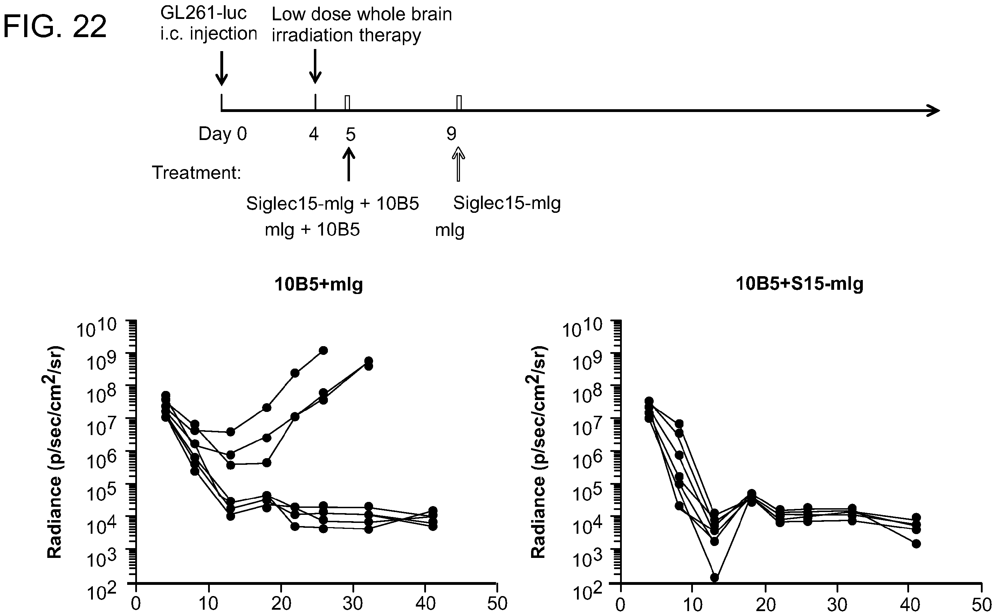

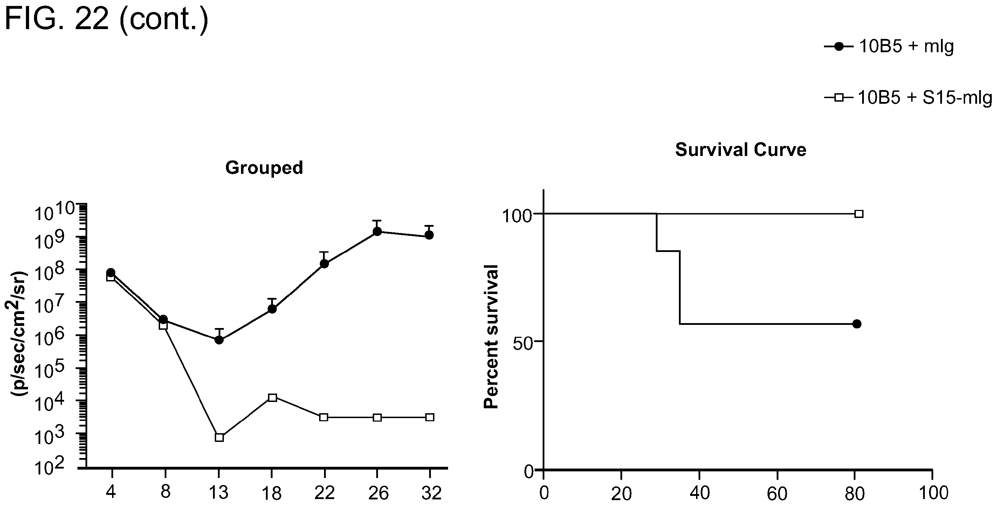

To test the effect of Siglec 15 in regulating immune responses in cancer, a brain tumor model was used. C57BL/6 mice were injected intarcranially with GL261 tumor cells containing a luciferase reporter which was used to quantitate tumor size. At day 4 after tumor injection, mice received a low dose whole brain irradiation therapy and were then treated with Siglec 15-Fc or control Ig at day 5 and day 10. Tumor size in both groups was intensively monitored. As demonstrated in Figures 20 and 21, blockade of Siglec 15 by Siglec 15-Fc treatment significantly reduced tumor size (Figure 20) and prolonged survival of the mice (Figure 21). Siglec15-Fc treated mice also showed a synergistic effect when treated with an anti-PD-L1 antibody, which markedly promoted the efficacy of anti-PD-L1 in tumor reduction and survival benefit (Figure 22). These results suggest that blocking or otherwise inhibiting the activity of Siglec 15 could enhance immune responses to cancer.

### Example 16. Significant Infiltration of Tumor-Associated CD8 T Cells in Tumor Bearing Mice

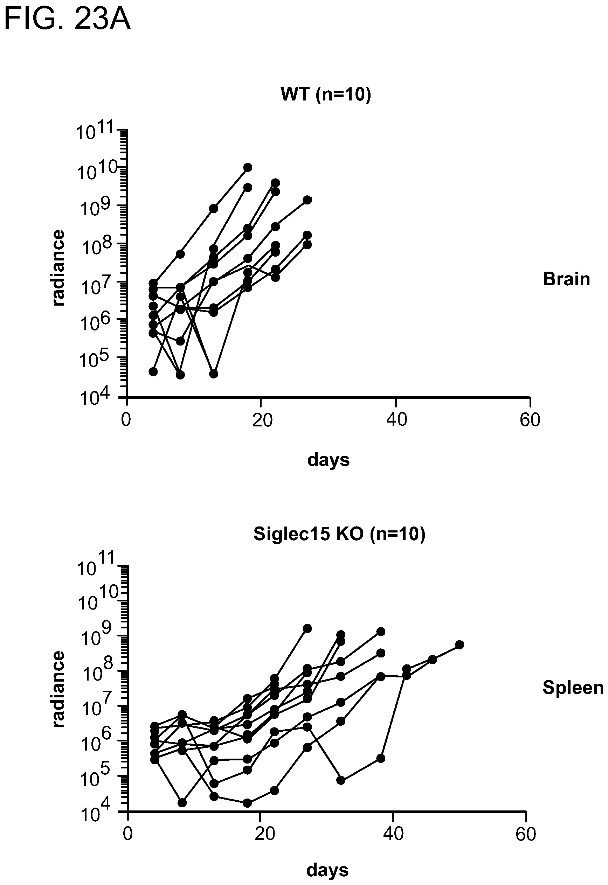

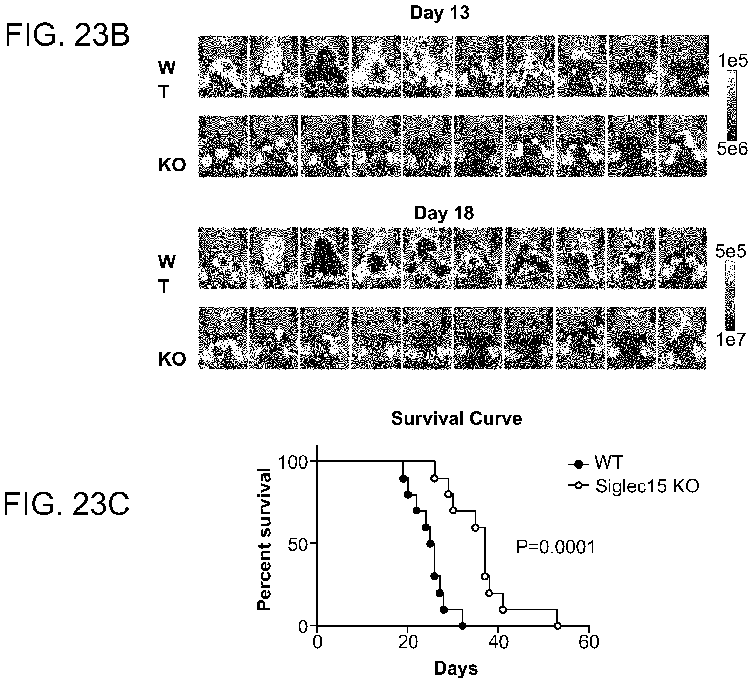

Siglec 15 is expressed in the brain, as demonstrated above. T cell numbers and activity in a wild-type (WT) and Siglec 15 knock-out (Siglec15 KO) brain tumor model were examined. WT or Siglec15 KO mice were inoculated with GL261 glioblastoma cells intra-cranially at day 0. Tumor burden in the brain was monitored at different time points by measuring luciferase activity (Figure 23A). Luciferase activity in the brain of WT and KO mice was imaged on days 13 and 18 after inoculation with GL261 cells (Figure 23B). A survival curve of WT and KO mice inoculated with GL261 demonstrates that Siglec 15 KO mice survive longer than WT (Figure 23C). At day 14, some mice from both groups were sacrificed. The number and percentage of CD8 T cells (Figure 24A), CD4 T cells (Figure 24B), or myeloid cell populations (Figure 24C) in the brain or spleen were monitored. In addition, brain lymphocytes from tumor bearing WT or KO mice were re-stimulated with GL-261 tumor cells overnight, and the percentage and total number of IFN-γ positive CD8 or CD4 T cells were determined (Figure 24D).

Significant infiltration of functional CD8 T cells was identified in the tumor site of Siglec15 KO mice, but not in the tumor site of WT mice. This effect is associated with the tumor, as there is no difference in the spleen. In addition, a significant difference in brain dendritic cell/macrophage populations was observed. This data indicates that Siglec15 could affect immune cell responses at the tumor site. In particular, Siglec 15 expression at the tumor/brain microenvironment can inhibit T cells and/or myeloid cells, and shut down the immune response against the tumor.

### Example 17. Growth of Tumor Cells Expressing Siglec 15

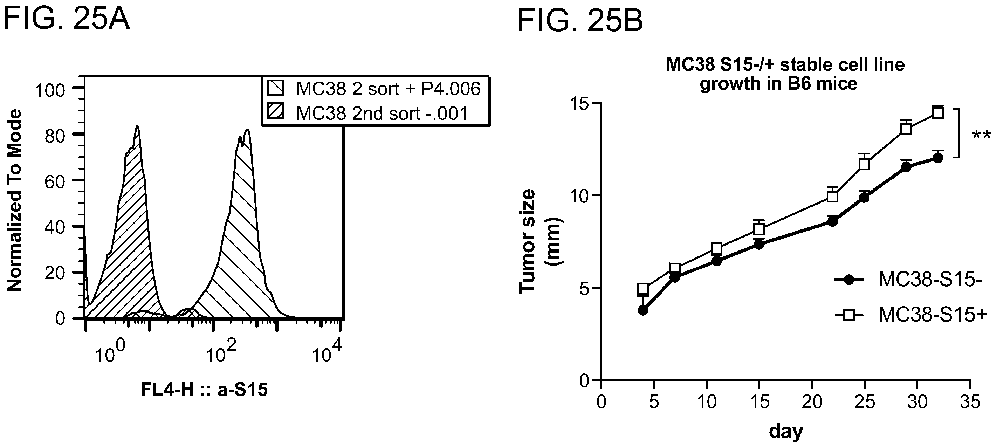

MC38 colon adenocarcinoma cells were infected with a lentiviral expression construct encoding Siglec 15 (S15+) or control (S15-). MC38-S15- and MC38-S15+ cell populations were sorted using FACS following lentiviral infection. Siglec 15 expression on MC38-S15- and MC38-S15+ cells was confirmed by FACS staining with a monoclonal antibody (Figure 25A). B6 mice were subcutaneously inoculated with MC38-S15- and MC38-S15+ cells (0.4M cells/mouse), and tumor growth was monitored (Figure 25B). Tumors derived from MC38 cells expressing Siglec 15 were larger than tumors derived from MC38 cells lacking Siglec 15 expression.

### Example 18. Siglec 15 Antagonists Inhibit Growth of Tumor Cells Expressing Siglec 15

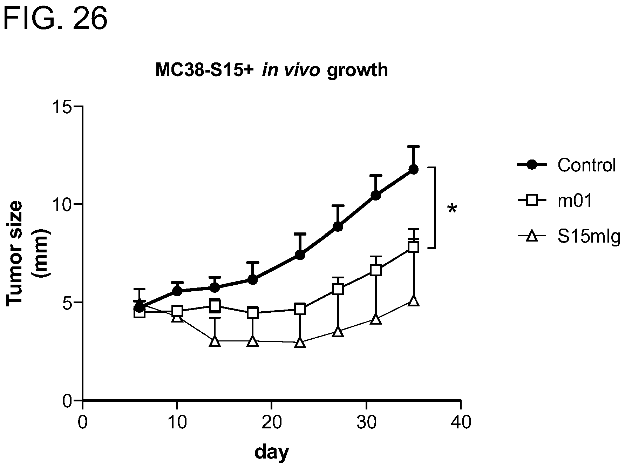

A MC38-S15+ stable cell line was subcutaneously inoculated into B6 mice (0.4M cells/mouse). Mice were treated with a control antibody, anti-Siglec 15 antibody m01, or S15-mlg (i.p., 200 µg/mouse) on day 6, and subsequently every four days, for a total of four doses. Average tumor size in each group is shown in Figure 26. Tumor size was significantly reduced in mice treated with Siglec 15 antagonists m01 or S15-mlg.

### Equivalents

Those skilled in the art will recognize, or be able to ascertain using no more that routine experimentation, many equivalents to the specific embodiments of the invention described herein. Such equivalents are intended to be encompassed by the following claims.

### ADDITIONAL STATEMENTS OF INVENTION

1. A method of treating a cancer in a subject in need thereof, comprising administering to the subject an effective amount of a modulator of Siglec 15, wherein the modulator decreases the expression and/or activity of Siglec 15 in the subject, thereby treating a cancer in the subject.
2. The method of paragraph 1, wherein the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15.
3. The method of paragraph 2, wherein the Siglec 15 fusion protein is a Siglec 15-Fc fusion protein.
4. The method of paragraph 1, wherein the modulator blocks the interaction between Siglec 15 and a binding ligand.
5. The method of paragraphs 4, wherein the binding ligand is selected from the group consisting of MAG, LRRC4C, and Sialyl-Tn.
6. The method of paragraph 4, wherein the modulator is a mutant Siglec 15 protein that has a single substitution mutation at residue 143.
7. The method of paragraph 4, wherein the modulator is a mutant Siglec 15 protein that has a deletion of the IgV domain.
8. The method of paragraph 1, wherein the modulator decreases the expression and/or activity levels of MAG and LRRC4C.
9. The method of paragraph 8, wherein the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.
10. The method of paragraph 1, wherein the cancer is selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma.
11. The method of paragraph 10, wherein the brain cancer is glioblastoma.
12. The method of paragraph 1, wherein the subject is human.
13. A method of reducing a tumor size in a subject in need thereof, comprising administering to the subject an effective amount of a modulator of Siglec 15, wherein the modulator decreases the expression and/or activity of Siglec 15, thereby reducing the tumor size in the subject.
14. The method of paragraph 13, wherein the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15.
15. The method of paragraph 14, wherein the Siglec 15 fusion protein is a Siglec 15-Fc fusion protein.
16. The method of paragraph 13, wherein the modulator blocks the interaction between Siglec 15 and a binding ligand.
17. The method of paragraphs 16, wherein the binding ligand is selected from the group consisting of MAG, LRRC4C, and Sialyl-Tn.
18. The method of paragraph 16, wherein the modulator is a mutant Siglec 15 protein that has a single substitution mutation at residue 143.
19. The method of paragraph 16, wherein the modulator is a mutant Siglec 15 protein that has a deletion of the IgV domain.
20. The method of paragraph 13, wherein the modulator decreases the expression and/or activity of MAG, LRRC4C, or Sialyl-Tn.
21. The method of paragraph 20, wherein the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.
22. The method of paragraph 13, wherein the tumor is associated with a cancer selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma.
23. The method of paragraph 22, wherein the brain cancer is glioblastoma.
24. The method of paragraph 13, wherein the subject is human.
25. A method of prolonging the survival of a subject having a cancer, comprising administering to the subject an effective amount of a modulator of Siglec 15, wherein the modulator decreases the expression and/or activity of Siglec 15, thereby prolonging the survival of the subject.
26. The method of paragraph 25, wherein the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15.
27. The method of paragraph 26, wherein the Siglec 15 fusion protein is a Siglec 15-Fc fusion protein.
28. The method of paragraph 25, wherein the modulator blocks the interaction between Siglec 15 and a binding ligand.
29. The method of paragraphs 28, wherein the binding ligand is selected from the group consisting of MAG, LRRC4C, and Sialyl-Tn.
30. The method of paragraph 28, wherein the modulator is a mutant Siglec 15 protein that has a single substitution mutation at residue 143.
31. The method of paragraph 28, wherein the modulator is a mutant Siglec 15 protein that has a deletion of the IgV domain.
32. The method of paragraph 25, wherein the modulator decreases the expression and/or activity of MAG and LRRC4C.
33. The method of paragraph 32, wherein the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.
34. The method of paragraph 25, wherein the cancer is selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma.
35. The method of paragraph 34, wherein the brain cancer is glioblastoma.
36. The method of paragraph 25, wherein the subject is human.
37. A method of increasing an immune response against a tumor in a subject in need thereof, comprising administering to the subject an effective amount of a modulator of Siglec15, wherein the modulator decreases the expression and/or activity of Siglec 15, thereby increasing an immune response against the tumor in the subject.
38. The method of paragraph 37, wherein the modulator is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15.
39. The method of paragraph 38, wherein the Siglec 15 fusion protein is a Siglec 15-Fc fusion protein.
40. The method of paragraph 37, wherein the modulator blocks the interaction between Siglec 15 and a binding ligand.
41. The method of paragraphs 40, wherein the binding ligand is selected from the group consisting of MAG, LRRC4C, and Sialyl-Tn.
42. The method of paragraph 40, wherein the modulator is a mutant Siglec 15 protein that has a single substitution mutation at residue 143.
43. The method of paragraph 40, wherein the modulator is a mutant Siglec 15 protein that has a deletion of the IgV domain.
44. The method of paragraph 37, wherein the modulator decreases the expression and/or activity of MAG and LRRC4C.
45. The method of paragraph 44, wherein the modulator is selected from the group consisting of a small molecule inhibitor of MAG, an antagonist antibody for MAG, or antigen-binding fragment thereof, a recombinant MAG fusion protein, an inhibitory peptide or nucleic acid targeting MAG, a small molecule inhibitor of LRRC4C, an antagonist antibody for LRRC4C, or antigen-binding fragment thereof, a recombinant LRRC4C fusion protein, or an inhibitory peptide or nucleic acid targeting LRRC4C.
46. The method of paragraph 37, wherein the tumor is associated with a cancer selected from the group consisting of brain cancer, lung cancer, pancreatic cancer, melanoma cancer, breast cancer, ovarian cancer, renal cell carcinoma, rectal adenocarcinoma, hepatocellular carcinoma, and Ewing sarcoma.
47. The method of paragraph 46, wherein the brain cancer is glioblastoma.
48. The method of paragraph 37, wherein the subject is human.
49. A method of treating an autoimmune disease in a subject in need thereof, comprising administering to the subject an effective amount of a modulator of Siglec 15, wherein the modulator increases the expression and/or activity of Siglec 15 in the subject, thereby treating an autoimmune disease in the subject.
50. The method of paragraph 49, wherein the modulator is selected from the group consisting of a small molecule activator of Siglec 15, an agonist antibody of Siglec 15, or antigen-binding fragment thereof, or a protein and a nucleic acid that activates the transcription and/or translation of Siglec 15.
51. The method of paragraph 49, wherein the modulator promotes the interaction between Siglec 15 and a binding ligand.
52. The method of paragraphs 51, wherein the binding ligand is selected from the group consisting of MAG, LRRC4C, and Sialyl-Tn.
53. The method of paragraph 49, wherein the modulator increases the expression and/or activity of MAG and LRRC4C.
54. The method of paragraph 53, wherein the modulator is selected from the group consisting of a small molecule activator of MAG, an agonist antibody for MAG, or antigen-binding fragment thereof, a protein and a nucleic acid that activates the transcription and/or translation of MAG, a small molecule activator of LRRC4C, an agonist antibody for LRRC4C, or antigen-binding fragment thereof, or a protein and a nucleic acid that activates the transcription and/or translation of LRRC4C.
55. The method of paragraph 53, wherein the modulator is selected from the group consisting of a MAG protein, a nucleic acid encoding the MAG protein, an LRRC4C protein or a nucleic acid encoding the LRRC4C protein.
56. The method of paragraph 49, wherein the autoimmune disease is an inflammatory brain disease.
57. The method of paragraph 56, wherein the inflammatory brain disease is multiple sclerosis.
58. The method of paragraph 49, wherein the subject is human.
59. A method of treating an autoimmune disease in a subject in need thereof, comprising administering to the subject an effective amount of Siglec 15 protein, a nucleic acid encoding the Siglec 15 protein, thereby treating an autoimmune disease in the subject.
60. The method of paragraph 59, wherein the Siglec 15 protein is selected from the group consisting of a full-length Siglec 15 protein, a functional fragment of Siglec 15, or an IgV domain of Siglec 15.
61. The method of paragraph 59, wherein the autoimmune disease is an inflammatory brain disease.
62. The method of paragraph 61, wherein the inflammatory brain disease is multiple sclerosis.
63. The method of paragraph 59, wherein the subject is human.
64. A method of decreasing a brain inflammatory response in a subject in need thereof, comprising administering to the subject an effective amount of a modulator of Siglec 15, wherein the modulator increases the expression and/or activity of Siglec 15, thereby decreasing a brain inflammatory response in the subject.
65. The method of paragraph 64, wherein the modulator is selected from the group consisting of a small molecule activator of Siglec 15, an agonist antibody of Siglec 15, or antigen-binding fragment thereof, or a protein and a nucleic acid that activates the transcription and/or translation of Siglec 15.
66. The method of paragraph 64, wherein the modulator promotes the interaction between Siglec 15 and a binding ligand.
67. The method of paragraphs 66, wherein the binding ligand is selected from the group consisting of MAG, LRRC4C, and Sialyl-Tn.
68. The method of paragraph 64, wherein the modulator increases the expression and/or activity of MAG and LRRC4C.
69. The method of paragraph 68, wherein the modulator is selected from the group consisting of a small molecule activator of MAG, an agonist antibody for MAG, or antigen-binding fragment thereof, a protein and a nucleic acid that activates the transcription and/or translation of MAG, a small molecule activator of LRRC4C, an agonist antibody for LRRC4C, or antigen-binding fragment thereof, or a protein and a nucleic acid that activates the transcription and/or translation of LRRC4C.
70. The method of paragraph 68, wherein the modulator is selected from the group consisting of
   a MAG protein, a nucleic acid encoding the MAG protein, an LRRC4C protein or a nucleic acid encoding the LRRC4C protein.
71. The method of paragraph 64, wherein the brain inflammatory response is associated with multiple sclerosis.
72. The method of paragraph 64, wherein the subject is human.
73. A method of decreasing a brain inflammatory response in a subject in need thereof, comprising administering to the subject an effective amount of Siglec 15 protein, or a nucleic acid encoding the Siglec 15 protein, thereby decreasing a brain inflammatory response in the subject.
74. The method of paragraph 73, wherein the Siglec 15 protein is selected from the group consisting of a full-length Siglec 15 protein, a functional fragment of Siglec 15, or an IgV domain of Siglec 15.
75. The method of paragraph 73, wherein the brain inflammatory response is associated with multiple sclerosis.
76. The method of paragraph 73, wherein the subject is human.
77. A method for identifying a compound useful for treating an autoimmune disease or a cancer in a subject, comprising
   providing a test compound;
   determining the effect of the test compound on the expression and/or activity of Siglec 15; and
   selecting a compound which modulates the expression and/or activity of Siglec 15, thereby identifying a compound useful for treating an autoimmune disease or a cancer in the subject.
78. The method of paragraph 77, wherein an increase in the expression and/or activity of Siglec 15 indicates that the compound is useful for treating an autoimmune disease.
79. The method of paragraph 77, wherein a decrease in the expression and/or activity of Siglec 15 indicates that the compound is useful for treating a cancer.
80. A method of identifying a compound useful for increasing an immune response against a tumor in a subject in need thereof, comprising
   providing a test compound;
   determining the effect of the test compound on the expression and/or activity of Siglec 15; and
   selecting a compound which decreases the expression and/or activity of Siglec 15, thereby identifying a compound useful for increasing an immune response against the tumor in the subject.
81. A method of identifying a compound useful for decreasing a brain inflammatory response in a subject in need thereof, comprising
   providing a test compound;
   determining the effect of the test compound on the expression and/or activity of Siglec 15; and
   selecting a compound which increases the expression and/or activity of Siglec 15, thereby identifying a compound useful for decreasing a brain inflammatory response in the subject.
82. A Siglec 15 modulator, wherein said modulator modulates the interaction between Siglec 15 and MAG, LRRC4C, and Sialyl-Tn.
83. The modulator of paragraph 82, wherein said modulator binds to the IgV domain of Siglec 15.
84. The modulator of paragraph 82, wherein said modulator binds to an epitope comprising residue 143 of Siglec 15.
85. The modulator of paragraph 82, wherein said modulator is an inhibitor of Siglec 15.
86. The modulator of paragraph 85, wherein said inhibitor is selected from the group consisting of a small molecule inhibitor of Siglec 15, an antagonist antibody for Siglec 15, or antigen-binding fragment thereof, a recombinant Siglec 15 fusion protein, or an inhibitory peptide or nucleic acid targeting Siglec 15.
87. The modulator of paragraph 82, wherein said modulator is an activator of Siglec 15.
88. The modulator of paragraph 87, wherein said activator is selected from the group consisting of a small molecule activator of Siglec 15, an agonist antibody of Siglec 15, or antigen-binding fragment thereof, or a protein and a nucleic acid that activates the transcription and/or translation of Siglec 15.
89. The method of paragraph 1, wherein said subject does not suffer from an ongoing autoimmune disease.
90. The method of paragraph 13, wherein said subject does not suffer from an ongoing autoimmune disease.
91. The method of paragraph 25, wherein said subject does not suffer from an ongoing autoimmune disease.
92. The method of paragraph 37, wherein said subject does not suffer from an ongoing autoimmune disease.

## Claims

1. A modulator of Siglec 15 for use in treating a cancer in a subject, wherein the modulator decreases the expression and/or activity of Siglec 15 in the subject.

2. The modulator for use in claim 1, wherein the modulator reduces tumor size in the subject.

3. The modulator for use in claim 1, wherein the modulator prolongs the survival of the subject.

4. The modulator for use in claim 1, wherein the modulator increases an immune response against a tumor in the subject.

5. The modulator for use in any one of claims 1-4, wherein the modulator comprises an antagonist antibody for Siglec 15, or antigen-binding fragment thereof; a recombinant Siglec 15 fusion protein, a mutant Siglec 15 protein, a small molecule inhibitor of Siglec 15, or an inhibitory peptide or nucleic acid targeting Siglec 15.

6. The modulator for use in claim 5, wherein the modulator comprises an antagonist antibody for Siglec 15, or antigen-binding fragment thereof.

7. The modulator for use in claim 5, wherein the modulator comprises a recombinant Siglec 15 fusion protein.

8. The modulator for use in claim 5, wherein the modulator comprises a mutant Siglec 15 protein.

9. The modulator for use in any one of claims 1-4, wherein the modulator blocks the interaction between Siglec 15 and a binding ligand.

10. The modulator for use in claim 9, wherein the binding ligand is selected from the group consisting of MAG, LRRC4C and Sialyl-Tn.

11. The modulator for use in any one of claims 1-4, wherein the subject is human.

12. The modulator for use in claim 1, wherein the cancer is a solid tumor cancer.

13. The modulator for use in claim 12, wherein the cancer is selected from the group consisting of colon cancer, endometriod cancer, kidney cancer, liver cancer, thyroid cancer, cervical cancer, prostate cancer, bladder cancer, glioblastoma, rectal cancer, bile duct cancer, brain cancer, lung cancer, pancreatic cancer, melanoma cancer, breast cancer, ovarian cancer, head and neck cancer, renal cell carcinoma, rectal adenocarcinoma, hepatocellular carcinoma, and Ewing sarcoma; optionally wherein the brain cancer is glioblastoma.
